# EUROPEAN PATENT SPECIFICATION

(11) **EP 3 931 191 B1**
(45) Date of publication and mention of the grant of the patent: **04.10.2023**
(21) Application number: 20706194.6
(22) Date of filing: 17.02.2020
(51) Int. Cl.: C07D 471/04, A61P 3/00, A61P 5/00, A61P 9/00, A61K 31/437

(54) **PYRAZOLOPYRIDINE DERIVATIVES AS INHIBITORS OF PASK**
PYRAZOLOPYRIDINDERIVATE ALS PASK-INHIBITOREN
DÉRIVÉS DE PYRAZOLOPYRIDINE UTILISÉS COMME INHIBITEURS DE PASK

(30) Priority: 25.02.2019 GB 201902490
(43) Date of publication of application: 05.01.2022
(73) Proprietor: Galapagos NV, 2800 Mechelen (BE)
(72) Inventor: BEAUMONT, Stéphane, Nicolas, Alain, 93230 Romainville (FR); BOCK, Xavier, Marie, 93230 Romainville (FR); COMAS MARTINEZ, Daniel, 93230 Romainville (FR); JONCOUR, Agnès, Marie, 93230 Romainville (FR); LABÉGUÈRE, Frédéric, Gilbert, 31270 Frouzins (FR); LÓPEZ RAMOS, Miriam, 93230 Romainville (FR); TEMAL-LAÏB, Taoues, 93230 Romainville (FR)
(74) Representative: Henry, Neil
(86) International application number: PCT/EP2020/054122
(87) International publication number: WO 2020/173739

(56) References cited:
- WO-A1-2014/066795
- WU, X. ET AL.: "PAS Kinase Drives Lipogenesis through SREBP-1 Maturation", CELL REPORTS, vol. 8, no. 1, 2014, pages 242-255, XP055483779, ISSN: 2211-1247, DOI: 10.1016/j.celrep.2014.06.006 cited in the application
- ZHANG, D.-D. ET AL.: "Per-Arnt-Sim Kinase (PASK): An Emerging Regulator of Mammalian Glucose and Lipid Metabolism", NUTRIENTS, vol. 7, no. 9, 2015, pages 7437-7450, XP055676997, DOI: 10.3390/nu7095347 cited in the application

## Description

### FIELD OF THE INVENTION

The present invention relates to compounds which may be useful in the prophylaxis and/or treatment of endocrine, nutritional, metabolic, and/or cardiovascular diseases. In particular, the compounds of the invention may inhibit PASK, a serine/threonine kinase involved in endocrine, nutritional, metabolic, and/or cardiovascular diseases. The present invention also provides methods for the production of the compounds of the invention, pharmaceutical compositions comprising the compound of the invention, and methods for the prophylaxis and/or treatment of endocrine, nutritional, metabolic, and/or cardiovascular diseases by administering the compound of the invention.

### BACKGROUND OF THE INVENTION

Type 2 diabetes mellitus (T2DM), is a chronic disease with significant morbidity and mortality. Recent projections indicate that approximately 629 million people will be affected by diabetes in 2045, making this a disease of considerable public health concern given the direct health costs and indirect costs of loss of work productivity. Most patients with diabetes have other features of the metabolic syndrome such as abdominal obesity, hypertriglyceridemia, low high-density lipoprotein cholesterol (HDL-C) levels and hypertension (Moller & Kaufman 2005). Despite the availability of twelve classes of anti-diabetic drugs, a limitation of currently available therapies is that no single agent is able to address more than one comorbid condition. Thus, multiple therapies are often prescribed in combination, leading to tolerability issues, poor patient compliance, and suboptimal outcomes. This provides an incentive to develop new therapeutic approaches that are able to address the multiple comorbidities associated with T2DM.

PASK is a Per-Arnt-Sim (PAS) domain-containing serine/threonine kinase that is described to be involved in glucose homeostasis and controlling lipid levels (Zhang et al. 2015). PASK is a nutrient-responsive protein kinase conserved from yeast to man. Biochemical and genetic data have implicated yeast PASK in the regulation of glucose utilization. Mammalian PASK is also involved in glucose and energy homeostasis through the regulation of insulin expression, lipid metabolism, and mitochondrial respiration. PASK may directly affect cellular glucose utilization through phosphorylation and inactivation of glycogen synthase (Hao & Rutter 2008).

Mice lacking PASK are viable and exhibit no obvious developmental or reproductive defect (Katschinski et al. 2003). Nevertheless, when PASK^{-/-} animals are fed with a high fat diet they show a nearly complete protection from obesity, hepatic triglyceride accumulation and insulin resistance (Hao et al. 2007; Pérez-García et al. 2018). This protection is likely due to increased metabolic rate and energy expenditure in PASK^{-/-} mice independent of the activity of AMP-activated protein kinase (AMPK), mammalian target of rapamycin (mTOR), and peroxisome proliferator-activated receptor γ coactivator 1 (PGC-1).

Increased oxidative metabolism and ATP generation are also observed in cultured cells upon acute PASK knockdown by RNA interference (RNAi) (Hao et al. 2007). Another important fact is the role of PASK in the control of lipogenesis through sterol regulatory element-binding protein 1 (SREBP-1) maturation. Indeed, elevated hepatic synthesis of fatty acids and triglycerides, driven by hyperactivation of the SREBP-1c transcription factor, has been implicated as a causal feature of metabolic syndrome. Using genetic and pharmacological approaches, it has been demonstrated that PASK is required for the proteolytic maturation of SREBP-1c in cultured cells and in the mouse and rat liver. Inhibition of PASK improves lipid and glucose metabolism in dietary animal models of obesity and dyslipidemia. Administration of a PASK inhibitor decreases hepatic expression of lipogenic SREBP-1c target genes, decreases serum triglycerides and partially reverses insulin resistance (Wu et al. 2014).

### SUMMARY OF THE INVENTION

The present invention relates to compounds which may be useful in the prophylaxis and/or treatment of endocrine, nutritional, metabolic, and/or cardiovascular diseases. In particular, the compounds of the invention may inhibit PASK, a serine/threonine kinase involved in endocrine, nutritional, metabolic, and/or cardiovascular diseases. The present invention also provides methods for the production of the compounds of the invention, pharmaceutical compositions comprising the compound of the invention, and methods for the prophylaxis and/or treatment of endocrine, nutritional, metabolic, and/or cardiovascular diseases by administering the compound of the invention.

Accordingly, in a first aspect of the invention, the compounds of the invention are provided having a Formula I: 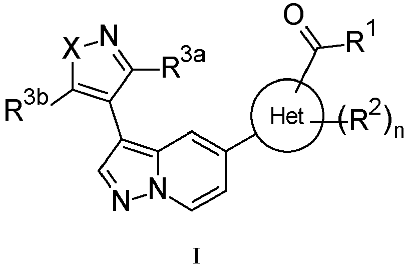 wherein,
Xis O or NR⁴;
n is 0, 1, or 2;
Het is 5 membered monocyclic heteroaryl comprising one, two or three heteroatoms independently selected from N, O, and S;
R¹ is -OR⁵ or -NR^{6a}R^{6b};
each R² is independently selected from
   - -O-R⁷,
   - C₁₋₆ alkyl optionally substituted with one or more independently selected halo,
   - C₃₋₆ cycloalkyl,
   - -C(=O)-NR^{8a}R^{8b},
   - 4-6 membered monocyclic heterocycloalkyl comprising one, two or three heteroatoms independently selected from N, O, and S, and
   - 4-6 membered monocyclic heterocycloalkenyl comprising one double bond and further comprising one, or two heteroatoms independently selected from N, O, and S;
R^{3a} and R^{3b} are independently H or C₁₋₃ alkyl optionally substituted with one or more independently selected halo;
R⁴ is C₁₋₃ alkyl optionally substituted with one or more F;
R⁵ is H or C₁₋₄ alkyl optionally substituted with one or more independently selected -C(=O)-NR^{9a}R^{9b} or -O-C(=O)-C₁₋₆ alkyl;
R^{6a} and R^{6b} are independently H, -S(=O)₂-C₁₋₄ alkyl, or -S(=O)₂-C₃₋₆ cycloalkyl;
each R⁷ is independently selected from:
   - C₁₋₆ alkyl optionally substituted with one or more independently selected halo or C₁₋₄ alkoxy, and
   - 4-6 membered monocyclic heterocycloalkyl comprising one, two or three heteroatoms independently selected from N, O, and S;
R^{8a} and R^{8b} are independently H, C₁₋₄ alkyl, or phenyl; and
R^{9a} and R^{9b} are independently H or C₁₋₄ alkyl.

In a particular aspect, the compounds of the invention are provided for use in the prophylaxis and/or treatment of endocrine, nutritional, metabolic, and/or cardiovascular diseases.

Furthermore, it has also been unexpectedly demonstrated that the compounds of the invention may improve both glucose and lipid profiles, in particular in treated animal models of metabolic disease.

In a further aspect, the present invention provides pharmaceutical compositions comprising a compound of the invention, and a pharmaceutical carrier, excipient or diluent. In a particular aspect, the pharmaceutical composition may additionally comprise further therapeutically active ingredients suitable for use in combination with the compounds of the invention. In a more particular aspect, the further therapeutically active ingredient is an agent for the treatment of endocrine, nutritional, metabolic, and/or cardiovascular diseases.

Moreover, the compounds of the invention, useful in the pharmaceutical compositions and treatment methods disclosed herein, are pharmaceutically acceptable as prepared and used.

In a further aspect of the invention, this invention provides a method of treating a mammal, in particular humans, afflicted with a condition selected from among those listed herein, and particularly endocrine, nutritional, metabolic, and/or cardiovascular diseases, which method comprises administering an effective amount of the pharmaceutical composition or compounds of the invention as described herein.

The present invention also provides pharmaceutical compositions comprising a compound of the invention, and a suitable pharmaceutical carrier, excipient or diluent for use in medicine. In a particular aspect, the pharmaceutical composition is for use in the prophylaxis and/or treatment of endocrine, nutritional, metabolic, and/or cardiovascular diseases.

In additional aspects, this invention provides methods for synthesizing the compounds of the invention, with representative synthetic protocols and pathways disclosed later on herein.

Other objects and advantages will become apparent to those skilled in the art from a consideration of the ensuing detailed description.

It will be appreciated that compounds of the invention may be metabolized to yield biologically active metabolites.

### DETAILED DESCRIPTION OF THE INVENTION

### Definitions

The following terms are intended to have the meanings presented therewith below and are useful in understanding the description and intended scope of the present invention.

When describing the invention, which may include compounds, pharmaceutical compositions containing such compounds and methods of using such compounds and compositions, the following terms, if present, have the following meanings unless otherwise indicated. It should also be understood that when described herein any of the moieties defined forth below may be substituted with a variety of substituents, and that the respective definitions are intended to include such substituted moieties within their scope as set out below. Unless otherwise stated, the term 'substituted' is to be defined as set out below. It should be further understood that the terms 'groups' and 'radicals' can be considered interchangeable when used herein.

The articles 'a' and 'an' may be used herein to refer to one or to more than one (*i.e.* at least one) of the grammatical objects of the article. By way of example 'an analogue' means one analogue or more than one analogue.

'Alkyl' means straight or branched aliphatic hydrocarbon having the specified number of carbon atoms. Particular alkyl groups have 1 to 6 carbon atoms or 1 to 4 carbon atoms. Branched means that one or more alkyl groups such as methyl, ethyl or propyl is attached to a linear alkyl chain. Particular alkyl groups are methyl (-CH₃), ethyl (-CH₂-CH₃), n-propyl (-CH₂-CH₂-CH₃), isopropyl (-CH(CH₃)₂), n-butyl (-CH₂-CH₂-CH₂-CH₃), tert-butyl (-CH₂-C(CH₃)₃), sec-butyl (-CH₂-CH(CH₃)₂), n-pentyl (-CH₂-CH₂-CH₂-CH₂-CH₃), n-hexyl (-CH₂-CH₂-CH₂-CH₂-CH₂-CH₃), and 1,2-dimethylbutyl (-CHCH₃)-C(CH₃)H₂-CH₂-CH₃). Particular alkyl groups have between 1 and 4 carbon atoms.

'Alkenyl' refers to monovalent olefinically (unsaturated) hydrocarbon groups with the number of carbon atoms specified. Particular alkenyl has 2 to 8 carbon atoms, and more particularly, from 2 to 6 carbon atoms, which can be straight-chained or branched and having at least 1 and particularly from 1 to 2 sites of olefinic unsaturation. Particular alkenyl groups include ethenyl (-CH=CH₂), n-propenyl (-CH₂CH=CH₂), isopropenyl (-C(CH₃)=CH₂) and the like.

'Alkoxy' refers to the group O-alkyl, where the alkyl group has the number of carbon atoms specified. In particular the term refers to the group -O-C₁₋₆ alkyl. Particular alkoxy groups are methoxy, ethoxy, n-propoxy, isopropoxy, n-butoxy, tert-butoxy, sec-butoxy, n-pentoxy, n-hexoxy, and 1,2-dimethylbutoxy. Particular alkoxy groups are lower alkoxy, i.e. with between 1 and 6 carbon atoms. Further particular alkoxy groups have between 1 and 4 carbon atoms.

'Amino' refers to the radical -NH₂.

'Aryl' refers to a monovalent aromatic hydrocarbon group derived by the removal of one hydrogen atom from a single carbon atom of a parent aromatic ring system. In particular aryl refers to an aromatic ring structure, monocyclic or fused polycyclic, with the number of ring atoms specified. Specifically, the term includes groups that include from 6 to 10 ring members. Particular aryl groups include phenyl, and naphthyl.

'Cycloalkyl'refers to a non-aromatic hydrocarbyl ring structure, monocyclic, fused polycyclic, bridged polycyclic, or spirocyclic, with the number of ring atoms specified. A cycloalkyl may have from 3 to 12 carbon atoms, in particular from 3 to 10, and more particularly from 3 to 7 carbon atoms. Such cycloalkyl groups include, by way of example, single ring structures such as cyclopropyl, cyclobutyl, cyclopentyl, cyclohexyl, and cycloheptyl.

'Cyano' refers to the radical -CN.

`Halo' or `halogen' refers to fluoro (F), chloro (Cl), bromo (Br) and iodo (I). Particular halo groups are either fluoro or chloro.

'Hetero' when used to describe a compound or a group present on a compound means that one or more carbon atoms in the compound or group have been replaced by a nitrogen, oxygen, or sulfur heteroatom. Hetero may be applied to any of the hydrocarbyl groups described above such as alkyl, *e.g.* heteroalkyl, cycloalkyl, *e.g.* heterocycloalkyl, aryl, *e.g.* heteroaryl, and the like having from 1 to 4, and particularly from 1 to 3 heteroatoms, more typically 1 or 2 heteroatoms, for example a single heteroatom.

`Heteroaryl' means an aromatic ring structure, monocyclic or fused polycyclic, that includes one or more heteroatoms independently selected from O, N and S and the number of ring atoms specified. In particular, the aromatic ring structure may have from 5 to 9 ring members. The heteroaryl group can be, for example, a five membered or six membered monocyclic ring or a fused bicyclic structure formed from fused five and six membered rings or two fused six membered rings or, by way of a further example, two fused five membered rings. Each ring may contain up to four heteroatoms typically selected from nitrogen, sulphur and oxygen. Typically the heteroaryl ring will contain up to 4 heteroatoms, more typically up to 3 heteroatoms, more usually up to 2, for example a single heteroatom. In one embodiment, the heteroaryl ring contains at least one ring nitrogen atom. The nitrogen atoms in the heteroaryl rings can be basic, as in the case of an imidazole or pyridine, or essentially non-basic as in the case of an indole or pyrrole nitrogen. In general the number of basic nitrogen atoms present in the heteroaryl group, including any amino group substituents of the ring, will be less than five.

Examples of five membered monocyclic heteroaryl groups include but are not limited to pyrrolyl, furanyl, thiophenyl, imidazolyl, furazanyl, oxazolyl, oxadiazolyl, oxatriazolyl, isoxazolyl, thiazolyl, isothiazolyl, pyrazolyl, triazolyl and tetrazolyl groups.

Examples of six membered monocyclic heteroaryl groups include but are not limited to pyridinyl, pyrazinyl, pyridazinyl, pyrimidinyl and triazinyl.

Particular examples of bicyclic heteroaryl groups containing a five membered ring fused to another five-membered ring include but are not limited to imidazothiazolyl and imidazoimidazolyl.

Particular examples of bicyclic heteroaryl groups containing a six membered ring fused to a five membered ring include but are not limited to benzofuranyl, benzothiophenyl, benzoimidazolyl, benzoxazolyl, isobenzoxazolyl, benzisoxazolyl, benzothiazolyl, benzoisothiazolyl, isobenzofuranyl, indolyl, isoindolyl, indolizinyl, purinyl (e.g. adenine, guanine), indazolyl, pyrazolopyrimidinyl, triazolopyrimidinyl, and pyrazolopyridinyl groups.

Particular examples of bicyclic heteroaryl groups containing two fused six membered rings include but are not limited to quinolinyl, isoquinolinyl, pyridopyridinyl, quinoxalinyl, quinazolinyl, cinnolinyl, phthalazinyl, naphthyridinyl, and pteridinyl groups. Particular heteroaryl groups are those derived from thiophenyl, pyrrolyl, benzothiophenyl, benzofuranyl, indolyl, pyridinyl, quinolinyl, imidazolyl, oxazolyl and pyrazinyl.

Examples of representative heteroaryls include the following: 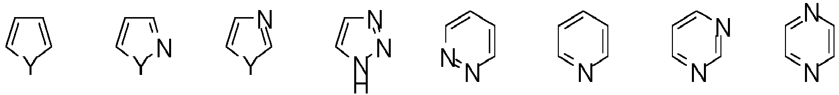 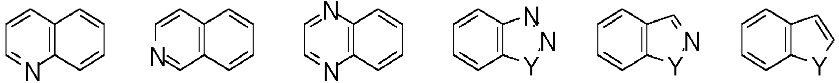 wherein each Y is selected from >C=O, NH, O and S.

'Heterocycloalkyl' means a non-aromatic fully saturated ring structure, monocyclic, fused polycyclic, spirocyclic, or bridged polycyclic, that includes one or more heteroatoms independently selected from O, N and S and the number of ring atoms specified. The heterocycloalkyl ring structure may have from 4 to 12 ring members, in particular from 4 to 10 ring members and more particularly from 4 to 7 ring members. Each ring may contain up to four heteroatoms typically selected from nitrogen, sulphur and oxygen. Typically the heterocycloalkyl ring will contain up to 4 heteroatoms, more typically up to 3 heteroatoms, more usually up to 2, for example a single heteroatom. Examples of heterocyclic rings include, but are not limited to azetidinyl, oxetanyl, thietanyl, pyrrolidinyl (*e.g*. 1-pyrrolidinyl, 2-pyrrolidinyl and 3-pyrrolidinyl), tetrahydrofuranyl (*e.g*. 1-tetrahydrofuranyl, 2-tetrahydrofuranyl and 3-tetrahydrofuranyl), tetrahydrothiophenyl (*e.g*. 1-tetrahydrothiophenyl, 2-tetrahydrothiophenyl and 3-tetrahydrothiophenyl), piperidinyl (*e.g.* 1-piperidinyl, 2-piperidinyl, 3-piperidinyl and 4-piperidinyl), tetrahydropyranyl (*e.g. 4-*tetrahydropyranyl), tetrahydrothiopyranyl (*e.g*. 4-tetrahydrothiopyranyl), morpholinyl, thiomorpholinyl, dioxanyl, or piperazinyl.

Particular examples of monocyclic rings are shown in the following illustrative examples: 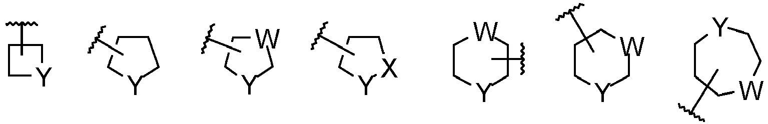 wherein each W and Y is independently selected from -CH₂-, -NH-, -O- and -S-.

Particular examples of fused bicyclic rings are shown in the following illustrative examples: 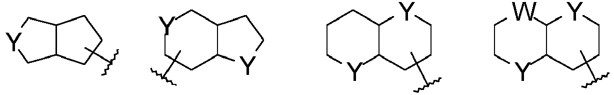 wherein each W and Y is independently selected from -CH₂-, -NH-, -O- and -S-.

Particular examples of bridged bicyclic rings are shown in the following illustrative examples: 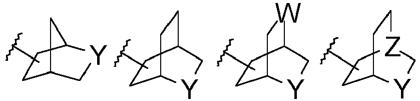 wherein each W and Y is independently selected from -CH₂-, -NH-, -O- and -S- and each Z is selected from N or CH.

Particular examples of spirocyclic rings are shown in the following illustrative examples: 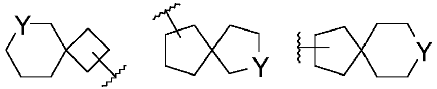 wherein each Y is selected from -CH₂-, -NH-, -O- and -S-.

As used herein, the term 'heterocycloalkenyl' means a 'heterocycloalkyl', which comprises at least one double bond. Particular examples of heterocycloalkenyl groups are shown in the following illustrative examples: 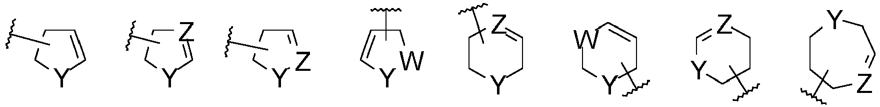 wherein each W is selected from CH₂, NH, O and S; each Y is selected from NH, O, C(=O), SOz, and S; and each Z is selected from N or CH.

`Hydroxyl' refers to the radical -OH.

'Oxo' refers to the radical =O.

'Substituted' refers to a group in which one or more hydrogen atoms are each independently replaced with the same or different substituent(s).

As used herein, term 'substituted with one or more' refers to one to four substituents. In one embodiment it refers to one to three substituents. In further embodiments it refers to one or two substituents. In a yet further embodiment it refers to one substituent.

One having ordinary skill in the art of organic synthesis will recognize that the maximum number of heteroatoms in a stable, chemically feasible heterocyclic ring, whether it is aromatic or non-aromatic, is determined by the size of the ring, the degree of unsaturation and the valence of the heteroatoms. In general, a heterocyclic ring may have one to four heteroatoms so long as the heteroaromatic ring is chemically feasible and stable.

'Pharmaceutically acceptable' means approved or approvable by a regulatory agency of the Federal or a state government or the corresponding agency in countries other than the United States, or that is listed in the U.S. Pharmacopoeia or other generally recognized pharmacopoeia for use in animals, and more particularly, in humans.

'Pharmaceutically acceptable salt' refers to a salt of a compound of the invention that is pharmaceutically acceptable and that possesses the desired pharmacological activity of the parent compound. In particular, such salts are non-toxic may be inorganic or organic acid addition salts and base addition salts. Specifically, such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, *e.g.* an alkali metal ion, an alkaline earth ion, or an aluminum ion; or coordinates with an organic base such as ethanolamine, diethanolamine, triethanolamine, N-methylglucamine and the like. Salts further include, by way of example only, sodium, potassium, calcium, magnesium, ammonium, tetraalkylammonium, and the like; and when the compound contains a basic functionality, salts of non toxic organic or inorganic acids, such as hydrochloride, hydrobromide, tartrate, mesylate, acetate, maleate, oxalate and the like. The term 'pharmaceutically acceptable cation' refers to an acceptable cationic counter-ion of an acidic functional group. Such cations are exemplified by sodium, potassium, calcium, magnesium, ammonium, tetraalkylammonium cations, and the like.

'Pharmaceutically acceptable vehicle' refers to a diluent, adjuvant, excipient or carrier with which a compound of the invention is administered.

'Prodrugs' refers to compounds, including derivatives of the compounds of the invention, which have cleavable groups and become by solvolysis or under physiological conditions the compounds of the invention which are pharmaceutically active *in vivo.* Such examples include, but are not limited to, choline ester derivatives and the like, N-alkylmorpholine esters and the like.

`Solvate' refers to forms of the compound that are associated with a solvent, usually by a solvolysis reaction. This physical association includes hydrogen bonding. Conventional solvents include water, EtOH, acetic acid and the like. The compounds of the invention may be prepared *e.g.* in crystalline form and may be solvated or hydrated. Suitable solvates include pharmaceutically acceptable solvates, such as hydrates, and further include both stoichiometric solvates and non-stoichiometric solvates. In certain instances the solvate will be capable of isolation, for example when one or more solvent molecules are incorporated in the crystal lattice of the crystalline solid. 'Solvate' encompasses both solution-phase and isolable solvates. Representative solvates include hydrates, ethanolates and methanolates.

'Subject' includes humans. The terms 'human', 'patient' and 'subject' are used interchangeably herein.

`Effective amount' means the amount of a compound of the invention that, when administered to a subject for treating a disease, is sufficient to effect such treatment for the disease. The `effective amount' can vary depending on the compound, the disease and its severity, and the age, weight, etc., of the subject to be treated.

'Preventing' or 'prevention' refers to a reduction in risk of acquiring or developing a disease or disorder (*i.e.* causing at least one of the clinical symptoms of the disease not to develop in a subject that may be exposed to a disease-causing agent, or predisposed to the disease in advance of disease onset.

The term 'prophylaxis' is related to 'prevention', and refers to a measure or procedure the purpose of which is to prevent, rather than to treat or cure a disease. Non-limiting examples of prophylactic measures may include the administration of vaccines; the administration of low molecular weight heparin to hospital patients at risk for thrombosis due, for example, to immobilization; and the administration of an anti-malarial agent such as chloroquine, in advance of a visit to a geographical region where malaria is endemic or the risk of contracting malaria is high.

'Treating' or 'treatment' of any disease or disorder refers, in one embodiment, to ameliorating the disease or disorder (*i.e.* arresting the disease or reducing the manifestation, extent or severity of at least one of the clinical symptoms thereof). In another embodiment 'treating' or 'treatment' refers to ameliorating at least one physical parameter, which may not be discernible by the subject. In yet another embodiment, 'treating' or 'treatment' refers to modulating the disease or disorder, either physically, (*e.g.* stabilization of a discernible symptom), physiologically, (*e.g*. stabilization of a physical parameter), or both. In a further embodiment, 'treating' or 'treatment' relates to slowing the progression of the disease.

As used herein the term `endocrine diseases' refers to disorders of the endocrine system and hormonal secretion. In particular, the term refers to adrenal diseases, obesity, metabolic syndrome, impaired glucose tolerance, prediabetes, Cushing's syndrome, chronic pancreatitis, insulin resistance, hyperglycemia, hyperinsulinemia, gestational diabetes, diabetes mellitus, insulin-dependent (type 1) diabetes mellitus, non-insulin-dependent (type 2) diabetes mellitus, and acromegaly. More particularly, the term refers to type 2 diabetes mellitus, obesity, and insulin resistance.

As used herein the term `nutritional diseases' refers to nutrient-related diseases and conditions resulting from eating a diet in which one or more nutrients are either not enough or are too much. In particular, the term refers to malnutrition, hyperalimentation, hyperglycemia, dyslipidemia, hyperlipidemia, hypertriglyceridemia, hypercholesterolemia, obesity, drug-induced obesity, morbid obesity, localized adiposity, and malnutrition-related diabetes mellitus. More particularly, the term refers to obesity, hyperlipidemia, and hyperglycemia.

As used herein the term `metabolic diseases' refers to disorders that disrupt normal metabolism, the process of converting food to energy on a cellular level. Metabolic diseases affect the ability to perform critical biochemical reactions that involve the processing or transport of proteins (amino acids), carbohydrates (sugars and starches), or lipids (fatty acids). In particular, the term refers to obesity, diabetes mellitus, especially type 2 diabetes, hyperinsulinemia, glucose intolerance, metabolic syndrome X, dyslipidemia, hyperlipidemia, hypertriglyceridemia, hypercholesterolemia, hyperlipoproteinemia, combined hyperlipidemia, and hepatic steatosis (fatty liver disease), including non-alcoholic fatty liver disease (NAFLD) and non-alcoholic steatohepatitis (NASH). More particularly, the term refers to type 2 diabetes, hyperlipidemia, and NASH.

As used herein the term `cardiovascular diseases' refers to diseases affecting the heart or blood vessels, or both. In particular, cardiovascular disease includes arrhythmia (atrial or ventricular or both); atherosclerosis and its sequelae; angina; cardiac rhythm disturbances; myocardial ischemia; myocardial infarction; cardiac or vascular aneurysm; vasculitis, stroke; peripheral obstructive arteriopathy of a limb, an organ, or a tissue; reperfusion injury following ischemia of the brain, heart, kidney or other organ or tissue; endotoxic, surgical, or traumatic shock; hypertension, valvular heart disease, heart failure, abnormal blood pressure; shock; vasoconstriction (including that associated with migraines); vascular abnormality, inflammation, insufficiency limited to a single organ or tissue. More particularly, the term refers to vascular disease, atherosclerosis, coronary heart disease, cerebrovascular disease, heart failure and peripheral vessel disease, and hypertension.

`Compound(s) of the invention', and equivalent expressions, are meant to embrace compounds of the Formula(e) as herein described, which expression includes the pharmaceutically acceptable salts, and the solvates, *e.g.* hydrates, and the solvates of the pharmaceutically acceptable salts where the context so permits. Similarly, reference to intermediates, whether or not they themselves are claimed, is meant to embrace their salts, and solvates, where the context so permits.

When ranges are referred to herein, for example but without limitation, C₁₋₈ alkyl, the citation of a range should be considered a representation of each member of said range.

Other derivatives of the compounds of this invention have activity in both their acid and acid derivative forms, but in the acid sensitive form often offers advantages of solubility, tissue compatibility, or delayed release in the mammalian organism (Bundgaard 1985). Prodrugs include acid derivatives well know to practitioners of the art, such as, for example, esters prepared by reaction of the parent acid with a suitable alcohol, or amides prepared by reaction of the parent acid compound with a substituted or unsubstituted amine, or acid anhydrides, or mixed anhydrides. Simple aliphatic or aromatic esters, amides and anhydrides derived from acidic groups pendant on the compounds of this invention are particularly useful prodrugs. In some cases it is desirable to prepare double ester type prodrugs such as (acyloxy)alkyl esters or ((alkoxycarbonyl)oxy)alkylesters. Particular such prodrugs are the C₁₋₈ alkyl, C₂₋₈ alkenyl, C₆₋₁₀ optionally substituted aryl, and (C₆₋₁₀ aryl)-(C₁₋₄ alkyl) esters of the compounds of the invention.

The present disclosure includes all isotopic forms of the compounds of the invention provided herein, whether in a form (i) wherein all atoms of a given atomic number have a mass number (or mixture of mass numbers) which predominates in nature (referred to herein as the `natural isotopic form') or (ii) wherein one or more atoms are replaced by atoms having the same atomic number, but a mass number different from the mass number of atoms which predominates in nature (referred to herein as an `unnatural variant isotopic form'). It is understood that an atom may naturally exists as a mixture of mass numbers. The term `unnatural variant isotopic form' also includes embodiments in which the proportion of an atom of given atomic number having a mass number found less commonly in nature (referred to herein as an `uncommon isotope') has been increased relative to that which is naturally occurring e.g. to the level of >20%, >50%, >75%, >90%, >95% or> 99% by number of the atoms of that atomic number (the latter embodiment referred to as an `isotopically enriched variant form'). The term `unnatural variant isotopic form' also includes embodiments in which the proportion of an uncommon isotope has been reduced relative to that which is naturally occurring. Isotopic forms may include radioactive forms (i.e. they incorporate radioisotopes) and non-radioactive forms. Radioactive forms will typically be isotopically enriched variant forms.

An unnatural variant isotopic form of a compound may thus contain one or more artificial or uncommon isotopes such as deuterium (²H or D), carbon-11 (¹¹C), carbon-13 (¹³C), carbon-14 (¹⁴C), nitrogen-13 (¹³N), nitrogen-15 (¹⁵N), oxygen-15 (¹⁵O), oxygen-17 (¹⁷O), oxygen-18 (¹⁸O), phosphorus-32 (³²P), sulphur-35 (³⁵S), chlorine-36 (³⁶Cl), chlorine-37 (³⁷Cl), fluorine-18 (¹⁸F) iodine-123 (¹²³I), iodine-125 (¹²⁵I) in one or more atoms or may contain an increased proportion of said isotopes as compared with the proportion that predominates in nature in one or more atoms.

Unnatural variant isotopic forms comprising radioisotopes may, for example, be used for drug and/or substrate tissue distribution studies. The radioactive isotopes tritium, i.e. ³H, and carbon-14, i.e. ¹⁴C, are particularly useful for this purpose in view of their ease of incorporation and ready means of detection. Unnatural variant isotopic forms which incorporate deuterium i.e ²H or D may afford certain therapeutic advantages resulting from greater metabolic stability, for example, increased in vivo half-life or reduced dosage requirements, and hence may be preferred in some circumstances. Further, unnatural variant isotopic forms may be prepared which incorporate positron emitting isotopes, such as ¹¹C, ¹⁸F, ¹⁵O and ¹³N, and would be useful in Positron Emission Topography (PET) studies for examining substrate receptor occupancy.

It is also to be understood that compounds that have the same molecular formula but differ in the nature or sequence of bonding of their atoms or the arrangement of their atoms in space are termed `isomers'. Isomers that differ in the arrangement of their atoms in space are termed `stereoisomers'.

Stereoisomers that are not mirror images of one another are termed 'diastereomers' and those that are non-superimposable mirror images of each other are termed 'enantiomers'. When a compound has an asymmetric center, for example, it is bonded to four different groups, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (i.e. as (+) or (-)-isomers respectively). A chiral compound can exist as either individual enantiomer or as a mixture thereof. A mixture containing equal proportions of the enantiomers is called a `racemic mixture'.

'Tautomers' refer to compounds that are interchangeable forms of a particular compound structure, and that vary in the displacement of hydrogen atoms and electrons. Thus, two structures may be in equilibrium through the movement of π electrons and an atom (usually H). For example, enols and ketones are tautomers because they are rapidly interconverted by treatment with either acid or base. Another example of tautomerism is the aci- and nitro- forms of phenylnitromethane, that are likewise formed by treatment with acid or base.

Tautomeric forms may be relevant to the attainment of the optimal chemical reactivity and biological activity of a compound of interest.

The compounds of the invention may possess one or more asymmetric centers; such compounds can therefore be produced as individual (R)- or (S)- stereoisomers or as mixtures thereof.

Unless indicated otherwise, the description or naming of a particular compound in the specification and claims is intended to include both individual enantiomers and mixtures, racemic or otherwise, thereof. The methods for the determination of stereochemistry and the separation of stereoisomers are well-known in the art.

### THE INVENTION

The present invention relates to compounds which may be useful in the prophylaxis and/or treatment of endocrine, nutritional, metabolic, and/or cardiovascular diseases. In particular, the compounds of the invention may inhibit PASK, a serine/threonine kinase involved in endocrine, nutritional, metabolic, and/or cardiovascular diseases.

Provided are also methods for the production of the compounds of the invention, pharmaceutical compositions comprising the compound of the invention, and methods for the prophylaxis and/or treatment of endocrine, nutritional, metabolic, and/or cardiovascular diseases by administering the compound of the invention.

Accordingly, in a first aspect of the invention, the compounds of the invention are provided having a Formula I: 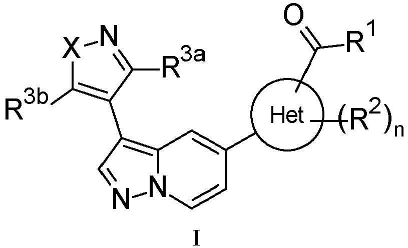 wherein,
Xis O or NR⁴;
n is 0, 1, or 2;
Het is 5 membered monocyclic heteroaryl comprising one, two or three heteroatoms independently selected from N, O, and S;
R¹ is -OR⁵ or -NR^{6a}R^{6b};
each R² is independently selected from
   - -O-R⁷,
   - C₁₋₆ alkyl optionally substituted with one or more independently selected halo,
   - C₃₋₆ cycloalkyl,
   - -C(=O)-NR^{8a}R^{8b},
   - 4-6 membered monocyclic heterocycloalkyl comprising one, two or three heteroatoms independently selected from N, O, and S, and
   - 4-6 membered monocyclic heterocycloalkenyl comprising one double bond and further comprising one, or two heteroatoms independently selected from N, O, and S;
R^{3a} and R^{3b} are independently H or C₁₋₃ alkyl optionally substituted with one or more independently selected halo;
R⁴ is C₁₋₃ alkyl optionally substituted with one or more F;
R⁵ is H or C₁₋₄ alkyl optionally substituted with one or more independently selected -C(=O)-NR^{9a}R^{9b} or -O-C(=O)-C₁₋₆ alkyl;
R^{6a} and R^{6b} are independently H, -S(=O)₂-C₁₋₄ alkyl, or -S(=O)₂-C₃₋₆ cycloalkyl;
each R⁷ is independently selected from
   - C₁₋₆ alkyl optionally substituted with one or more independently selected halo or C₁₋₄ alkoxy, and
   - 4-6 membered monocyclic heterocycloalkyl comprising one, two or three heteroatoms independently selected from N, O, and S;
R^{8a} and R^{8b} are independently H, C₁₋₄ alkyl, or phenyl; and
R^{9a} and R^{9b} are independently H or C₁₋₄ alkyl.

In one embodiment, a compound of the invention is according to Formula I, wherein Het is pyrrolyl, furanyl, thiophenyl, pyrazolyl, imidazolyl, oxazolyl, isoxazolyl, thiazolyl, isothiazolyl, triazolyl, furazanyl, oxadiazolyl, or thiadiazolyl. In a particular embodiment, Het is pyrrolyl, furanyl, thiophenyl, pyrazolyl, imidazolyl, oxazolyl, isoxazolyl, thiazolyl, isothiazolyl, triazolyl, 1,2,4-oxadiazolyl, 1,3,4-oxadiazolyl, 1,2,4-thiadiazolyl, or 1,3,4-thiadiazolyl. In a more particular embodiment, Het is furanyl, pyrazolyl, oxazolyl, or thiazolyl. In a further more particular embodiment, Het is furanyl or thiazolyl. In a most particular embodiment, Het is furanyl.

In one embodiment, a compound of the invention is according to Formula IIa, IIb, IIc, or IId: 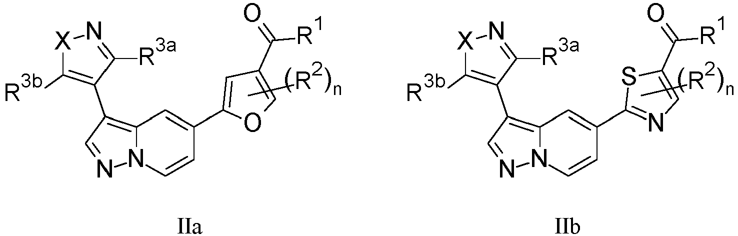 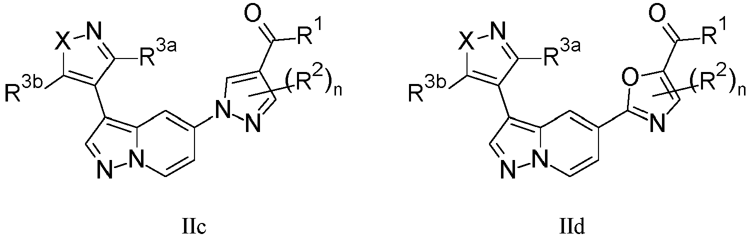 wherein R¹, R², R^{3a}, R^{3b}, X and the subscript n are as described above.

In one embodiment, a compound of the invention is according to any one of Formulae I-IId, wherein R^{3a} is H.

In one embodiment, a compound of the invention is according to any one of Formulae I-IId, wherein R^{3a} is C₁₋₃ alkyl. In a particular embodiment, R^{3a} is -CH₃, -CH₂CH₃, or -CH(CH₃)₂. In a more particular embodiment, R^{3a} is -CH₃.

In one embodiment, a compound of the invention is according to any one of Formulae I-IId, wherein R^{3a} is C₁₋₃ alkyl substituted with one or more independently selected halo. In a particular embodiment, R^{3a} is -CH₃, -CH₂CH₃, or -CH(CH₃)₂, each of which is substituted with one or more independently selected halo. In another particular embodiment, R^{3a} is C₁₋₃ alkyl substituted with one, two, or three independently selected halo. In yet another particular embodiment, R^{3a} is C₁₋₃ alkyl substituted with one or more independently selected F or Cl. In a more particular embodiment, R^{3a} is -CH₃ substituted with one or more independently selected halo. In another more particular embodiment, R^{3a} is -CH₃, -CH₂CH₃, or -CH(CH₃)₂, each of which is substituted with one, two or three independently selected halo. In yet another more particular embodiment, R^{3a} is -CH₃, -CH₂CH₃, or -CH(CH₃)₂, each of which is substituted with one or more independently selected F or Cl. In yet another more particular embodiment, R^{3a} is C₁₋₃ alkyl substituted with one, two, or three independently selected F or Cl. In yet another more particular embodiment, R^{3a} is C₁₋₃ alkyl substituted with one or more F. In a further more particular embodiment, R^{3a} is -CH₃ substituted with one, two or three independently selected halo. In another further more particular embodiment, R^{3a} is -CH₃, -CH₂CH₃, or -CH(CH₃)₂, each of which is substituted with one, two or three independently selected F or Cl. In yet another further more particular embodiment, R^{3a} is C₁₋₃ alkyl substituted with one, two, or three F. In yet another further more particular embodiment, R^{3a} is -CH₃ substituted with one or more independently selected F or Cl. In yet another further more particular embodiment, R^{3a} is -CH₃, -CH₂CH₃, or -CH(CH₃)₂, each of which is substituted with one or more F. In an even further more particular embodiment, R^{3a} is -CH₃ substituted with one, two, or three F. In a most particular embodiment, R^{3a} is -CF₃.

In one embodiment, a compound of the invention is according to any one of Formulae I-IId, wherein R^{3b} is H.

In one embodiment, a compound of the invention is according to any one of Formulae I-IId, wherein R^{3b} is C₁₋₃ alkyl. In a particular embodiment, R^{3b} is -CH₃, -CH₂CH₃, or -CH(CH₃)₂. In a more particular embodiment, R^{3b} is -CH₃.

In one embodiment, a compound of the invention is according to any one of Formulae I-IId, wherein R^{3b} is C₁₋₃ alkyl substituted with one or more independently selected halo. In a particular embodiment, R^{3b} is -CH₃, -CH₂CH₃, or -CH(CH₃)₂, each of which is substituted with one or more independently selected halo. In another particular embodiment, R^{3b} is C₁₋₃ alkyl substituted with one, two, or three independently selected halo. In yet another particular embodiment, R^{3b} is C₁₋₃ alkyl substituted with one or more independently selected F or Cl. In a more particular embodiment, R^{3b} is -CH₃ substituted with one or more independently selected halo. In another more particular embodiment, R^{3b} is -CH₃, -CH₂CH₃, or -CH(CH₃)₂, each of which is substituted with one, two or three independently selected halo. In yet another more particular embodiment, R^{3b} is -CH₃, -CH₂CH₃, or -CH(CH₃)₂, each of which is substituted with one or more independently selected F or Cl. In yet another more particular embodiment, R^{3b} is C₁₋₃ alkyl substituted with one, two, or three independently selected F or Cl. In yet another more particular embodiment, R^{3b} is C₁₋₃ alkyl substituted with one or more F. In a further more particular embodiment, R^{3b} is -CH₃ substituted with one, two or three independently selected halo. In another further more particular embodiment, R^{3b} is -CH₃, -CH₂CH₃, or -CH(CH₃)₂, each of which is substituted with one, two or three independently selected F or Cl. In yet another further more particular embodiment, R^{3b} is C₁₋₃ alkyl substituted with one, two, or three F. In yet another further more particular embodiment, R^{3b} is -CH₃ substituted with one or more independently selected F or Cl. In yet another further more particular embodiment, R^{3b} is -CH₃, -CH₂CH₃, or -CH(CH₃)₂, each of which is substituted with one or more F. In an even further more particular embodiment, R^{3b} is -CH₃ substituted with one, two, or three F. In a most particular embodiment, R^{3b} is -CF₃.

In one embodiment, a compound of the invention is according to any one of Formulae I-IId, wherein n is 0 or 1. In a particular embodiment, n is 0.

In one embodiment, a compound of the invention is according to Formula IIIa, IIIb, IIIc, or IIId: 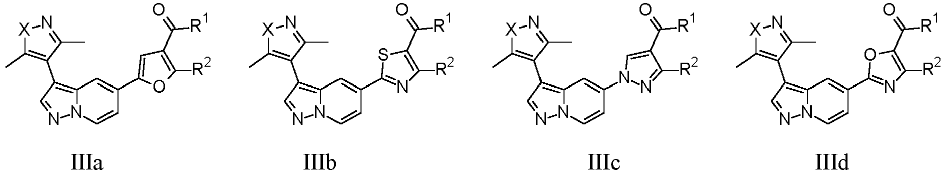 wherein R¹, R², and X are as described above.

In one embodiment, a compound of the invention is according to any one of Formulae I-IIId, wherein R² is -O-R⁷, and R⁷ is as previously described.

In one embodiment, a compound of the invention is according to any one of Formulae I-IIId, wherein R² is -O-R⁷, and R⁷ is C₁₋₆ alkyl. In a particular embodiment, R⁷ is -CH₃, -CH₂CH₃, -CH₂CH₂CH₃, -CH(CH₃)₂, -CH₂CH(CH₃)₂, -C(CH₃)₃, -CH(CH₃)CH₂CH₃, or -CH(CH₃)CH(CH₃)₂. In a more particular embodiment, R⁷ is -CH₃, -CH₂CH₃, or -CH(CH₃)₂. In a most particular embodiment, R⁷ is -CH₂CH₃.

In one embodiment, a compound of the invention is according to any one of Formulae I-IIId, wherein R² is -O-R⁷, and R⁷ is C₁₋₆ alkyl substituted with one or more independently selected halo or C₁₋₄ alkoxy. In a particular embodiment, R⁷ is -CH₃, -CH₂CH₃, -CH₂CH₂CH₃, -CH(CH₃)₂, -CH₂CH(CH₃)₂, -C(CH₃)₃, -CH(CH₃)CH₂CH₃, or -CH(CH₃)CH(CH₃)₂, each of which is substituted with one or more independently selected halo or C₁₋₄ alkoxy. In another particular embodiment, R⁷ is C₁₋₆ alkyl substituted with one, two, or three independently selected halo or C₁₋₄ alkoxy. In yet another particular embodiment, R⁷ is C₁₋₆ alkyl substituted with one or more independently selected F, Cl, -O-CH₃, -O-CH₂CH₃, or -O-CH(CH₃)₂. In a more particular embodiment, R⁷ is -CH₃ or -CH₂CH₃, each of which is substituted with one or more independently selected halo or C₁₋₄ alkoxy. In another more particular embodiment, R⁷ is -CH₃, -CH₂CH₃, -CH₂CH₂CH₃, -CH(CH₃)₂, -CH₂CH(CH₃)₂, -C(CH₃)₃, -CH(CH₃)CH₂CH₃, or -CH(CH₃)CH(CH₃)₂, each of which is substituted with one, two, or three independently selected halo or C₁₋₄ alkoxy. In yet another more particular embodiment, R⁷ is -CH₃, -CH₂CH₃, -CH₂CH₂CH₃, -CH(CH₃)₂, -CH₂CH(CH₃)₂, -C(CH₃)₃, -CH(CH₃)CH₂CH₃, or -CH(CH₃)CH(CH₃)₂, each of which is substituted with one or more independently selected F, Cl, -O-CH₃, -O-CH₂CH₃, or -O-CH(CH₃)₂. In yet another more particular embodiment, R⁷ is C₁₋₆ alkyl substituted with one, two, or three independently selected F, Cl, -O-CH₃, -O-CH₂CH₃, or -O-CH(CH₃)₂. In yet another more particular embodiment, R⁷ is C₁₋₆ alkyl substituted with one or more independently selected F, -O-CH₃, or -O-CH₂CH₃. In a further more particular embodiment, R⁷ is -CH₃ or -CH₂CH₃, each of which is substituted with one, two, or three independently selected halo or C₁₋₄ alkoxy. In another further more particular embodiment, R⁷ is -CH₃, -CH₂CH₃, -CH₂CH₂CH₃, -CH(CH₃)₂, -CH₂CH(CH₃)₂, -C(CH₃)₃, -CH(CH₃)CH₂CH₃, or -CH(CH₃)CH(CH₃)₂, each of which is substituted with one, two, or three independently selected F, Cl, -O-CH₃, -O-CH₂CH₃, or -O-CH(CH₃)₂. In yet another further more particular embodiment, R⁷ is C₁₋₆ alkyl substituted with one, two, or three independently selected F, -O-CH₃, or -O-CH₂CH₃. In yet another further more particular embodiment, R⁷ is -CH₃ or -CH₂CH₃, each of which is substituted with one or more independently selected F, Cl, -O-CH₃, -O-CH₂CH₃, or -O-CH(CH₃)₂. In yet another further more particular embodiment, R⁷ is -CH₃, -CH₂CH₃, -CH₂CH₂CH₃, -CH(CH₃)₂, -CH₂CH(CH₃)₂, -C(CH₃)₃, -CH(CH₃)CH₂CH₃, or -CH(CH₃)CH(CH₃)₂, each of which is substituted with one or more independently selected F, -O-CH₃, or -O-CH₂CH₃. In an even more particular embodiment, R⁷ is -CH₃ or -CH₂CH₃, each of which is substituted with one, two, or three independently selected F, -O-CH₃, or -O-CH₂CH₃. In a most particular embodiment, R⁷ is -CHF₂, -CH₂CF₃, -CH₂CH₂-O-CH₃, or -CH₂CH₂-O-CH₂CH₃.

In one embodiment, a compound of the invention is according to any one of Formulae I-IIId, wherein R² is -O-R⁷, and R⁷ is 4-6 membered monocyclic heterocycloalkyl comprising one, two or three heteroatoms independently selected from N, O, and S. In a particular embodiment, R⁷ is azetidinyl, oxetanyl, pyrrolidinyl, tetrahydrofuranyl, piperidinyl, tetrahydropyranyl, tetrahydrothiopyranyl, morpholinyl, thiomorpholinyl, dioxanyl, or piperazinyl. In a more particular embodiment, R⁷ is oxetanyl.

In one embodiment, a compound of the invention is according to any one of Formulae I-IIId, wherein R² is C₁₋₆ alkyl. In a particular embodiment, R² is -CH₃, -CH₂CH₃, -CH₂CH₂CH₃, -CH(CH₃)₂, -CH₂CH(CH₃)₂, -C(CH₃)₃, -CH(CH₃)CH₂CH₃, or -CH(CH₃)CH(CH₃)₂. In a more particular embodiment, R² is -CH₃, -CH₂CH₃, or -CH₂CH(CH₃)₂.

In one embodiment, a compound of the invention is according to any one of Formulae I-IIId, wherein R² is C₁₋₆ alkyl substituted with one or more independently selected halo. In a particular embodiment, R² is -CH₃, -CH₂CH₃, -CH₂CH₂CH₃, -CH(CH₃)₂, -CH₂CH(CH₃)₂, -C(CH₃)₃, -CH(CH₃)CH₂CH₃, or -CH(CH₃)CH(CH₃)₂, each of which is substituted with one or more independently selected halo. In another particular embodiment, R² is C₁₋₆ alkyl substituted with one, two, or three independently selected halo. In yet another particular embodiment, R² is C₁₋₆ alkyl substituted with one or more independently selected F, Cl, or Br. In a more particular embodiment, R² is -CH₃ substituted with one or more independently selected halo. In another more particular embodiment, R² is -CH₃, -CH₂CH₃, -CH₂CH₂CH₃, -CH(CH₃)₂, -CH₂CH(CH₃)₂, -C(CH₃)₃, -CH(CH₃)CH₂CH₃, or -CH(CH₃)CH(CH₃)₂, each of which is substituted with one, two, or three independently selected halo. In yet another more particular embodiment, R² is -CH₃, -CH₂CH₃, -CH₂CH₂CH₃, -CH(CH₃)₂, -CH₂CH(CH₃)₂, -C(CH₃)₃, -CH(CH₃)CH₂CH₃, or -CH(CH₃)CH(CH₃)₂, each of which is substituted with one or more independently selected F, Cl, or Br. In a further more particular embodiment, R² is -CH₃ substituted with one, two, or three independently selected halo. In another further more particular embodiment, R² is -CH₃, -CH₂CH₃, -CH₂CH₂CH₃, -CH(CH₃)₂, -CH₂CH(CH₃)₂, -C(CH₃)₃, -CH(CH₃)CH₂CH₃, or -CH(CH₃)CH(CH₃)₂, each of which is substituted with one, two, or three independently selected F, Cl, or Br. In yet another further more particular embodiment, R² is -CH₃, -CH₂CH₃, -CH₂CH₂CH₃, -CH(CH₃)₂, -CH₂CH(CH₃)₂, -C(CH₃)₃, -CH(CH₃)CH₂CH₃, or -CH(CH₃)CH(CH₃)₂, each of which is substituted with one or more F. In a most particular embodiment, R² is -CHF₂ or -CF₃.

In one embodiment, a compound of the invention is according to any one of Formulae I-IIId, wherein R² is C₃₋₆ cycloalkyl. In a particular embodiment, R² is cyclopropyl, cyclobutyl, or cyclopentyl. In a more particular embodiment, R² is cyclopropyl.

In one embodiment, a compound of the invention is according to any one of Formulae I-IIId, wherein R² is -C(=O)-NR^{8a}R^{8b}, and each R^{8a} and R^{8b} is as previously described. In a particular embodiment, R^{8a} and R^{8b} are both H. In another particular embodiment, one of R^{8a} and R^{8b} is H, and the other is C₁₋₄ alkyl, or phenyl. In yet another particular embodiment, R^{8a} and R^{8b} are both C₁₋₄ alkyl. In a more particular embodiment, one of R^{8a} and R^{8b} is H, and the other is -CH₃, -CH₂CH₃, or -CH(CH₃)₂, or phenyl. In another more particular embodiment, R^{8a} and R^{8b} are independently -CH₃, -CH₂CH₃, or -CH(CH₃)₂. In a most particular embodiment, one of R^{8a} and R^{8b} is H, and the other is phenyl.

In one embodiment, a compound of the invention is according to any one of Formulae I-IIId, wherein R² is 4-6 membered monocyclic heterocycloalkyl comprising one, two or three heteroatoms independently selected from N, O, and S. In a particular embodiment, R² is azetidinyl, oxetanyl, pyrrolidinyl, tetrahydrofuranyl, piperidinyl, tetrahydropyranyl, tetrahydrothiopyranyl, morpholinyl, thiomorpholinyl, dioxanyl, or piperazinyl. In a more particular embodiment, R² is tetrahydropyranyl.

In one embodiment, a compound of the invention is according to any one of Formulae I-IIId, wherein R² is 4-6 membered monocyclic heterocycloalkenyl comprising one double bond and further comprising one, or two heteroatoms independently selected from N, O, and S. In a particular embodiment, R² is pyrrolinyl, pyrazolinyl, imidazolinyl, tetrahydropyridinyl, or dihydropyranyl. In a more particular embodiment, R² is 3,6-dihydro-2H-pyranyl.

In one embodiment, a compound of the invention is according to Formula IVa, IVb, IVc, or IVd: 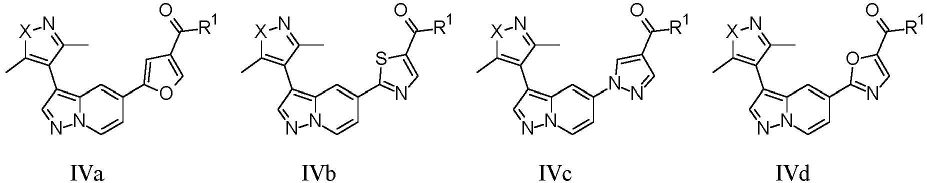 wherein R¹ and X are as described above.

In one embodiment, a compound of the invention is according to any one of Formulae I-IVd, wherein X is O.

In one embodiment, a compound of the invention is according to any one of Formulae I-IVd, wherein X is NR⁴, and R⁴ is as previously described. In a particular embodiment, R⁴ is C₁₋₃ alkyl. In a more particular embodiment, R⁴ is -CH₃, -CH₂CH₃, or -CH(CH₃)₂. In a most particular embodiment, R⁴ is -CH(CH₃)₂.

In one embodiment, a compound of the invention is according to any one of Formulae I-IVd, wherein X is NR⁴, and R⁴ is as previously described. In a particular embodiment, R⁴ is C₁₋₃ alkyl substituted with one or more F. In a more particular embodiment, R⁴ is -CH₃, -CH₂CH₃, or -CH(CH₃)₂, each of which is substituted with one or more F. In another more particular embodiment, R⁴ is C₁₋₃ alkyl substituted with one, two, or three F. In a most particular embodiment, R⁴ is -CH₃, -CH₂CH₃, or -CH(CH₃)₂, each of which is substituted with one, two or three F. In a further most particular embodiment, R⁴ is - CHF₂, -CF₃, -CH₂-CF₃, or -CH(CH₃)-CF₃. In a further most particular embodiment, R⁴ is -CHF₂ or -CH₂-CF₃.

In one embodiment, a compound of the invention is according to Formula Va, Vb, or Vc: 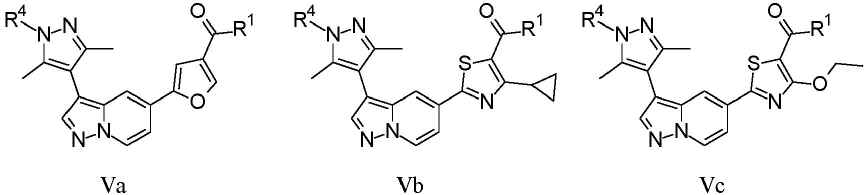 wherein R¹ and R⁴ are as described above.

In one embodiment, a compound of the invention is according to any one of Formulae Va-Vc, wherein R⁴ is C₁₋₃ alkyl. In a particular embodiment, R⁴ is -CH₃, -CH₂CH₃, or -CH(CH₃)₂. In a more particular embodiment, R⁴ is -CH(CH₃)₂.

In one embodiment, a compound of the invention is according to any one of Formulae Va-Vc, wherein R⁴ is C₁₋₃ alkyl substituted with one or more F. In a particular embodiment, R⁴ is -CH₃, -CH₂CH₃, or -CH(CH₃)₂, each of which is substituted with one or more F. In another particular embodiment, R⁴ is C₁₋₃ alkyl substituted with one, two, or three F. In a more particular embodiment, R⁴ is -CH₃, -CH₂CH₃, or -CH(CH₃)₂, each of which is substituted with one, two or three F. In a further more particular embodiment, R⁴ is -CHF₂, -CF₃, -CH₂-CF₃, or -CH(CH₃)-CF₃. In a most particular embodiment, R⁴ is -CHF₂ or -CH₂-CF₃.

In one embodiment, a compound of the invention is according to any one of Formulae I-Vc, wherein R¹ is -OR⁵, and R⁵ is as previously described.

In one embodiment, a compound of the invention is according to any one of Formulae I-Vc, wherein R¹ is -OR⁵, and R⁵ is H.

In one embodiment, a compound of the invention is according to any one of Formulae I-Vc, wherein R¹ is -OR⁵, and R⁵ is C₁₋₄ alkyl. In a particular embodiment, R⁵ is -CH₃, -CH₂CH₃, or -CH(CH₃)₂. In a more particular embodiment, R⁵ is -CH₃ or -CH₂CH₃.

In one embodiment, a compound of the invention is according to any one of Formulae I-Vc, wherein R¹ is -OR⁵, and R⁵ is C₁₋₄ alkyl substituted with one or more independently selected -C(=O)-NR^{9a}R^{9b} or -O-C(=O)-C₁₋₆ alkyl. In a particular embodiment, R⁵ is -CH₃, -CH₂CH₃, or -CH(CH₃)₂, each of which is substituted with one or more independently selected -C(=O)-NR^{9a}R^{9b} or -O-C(=O)-C₁₋₆ alkyl. In another particular embodiment, R⁵ is C₁₋₄ alkyl substituted with one, two, or three independently selected -C(=O)-NR^{9a}R^{9b} or -O-C(=O)-C₁₋₆ alkyl. In yet another particular embodiment, R⁵ is C₁₋₄ alkyl substituted with one or more independently selected -C(=O)-NR^{9a}R^{9b}, -O-C(=O)-CH₃, -O-C(=O)-CH₂CH₃, -O-C(=O)-CH₂CH₂CH₃, -O-C(=O)-CH(CH₃)₂, -O-C(=O)-CH₂CH(CH₃)₂, -O-C(=O)-C(CH₃)₃, -O-C(=O)-CH(CH₃)CH₂CH₃, -O-C(=O)-CH(CH₃)CH(CH₃)₂, -O-C(=O)-CH₂CH(CH₃)CH₂CH₃, or -O-C(=O)-CH₂CH₂CH(CH₃)₂. In a more particular embodiment, R⁵ is -CH₃ substituted with one or more independently selected -C(=O)-NR^{9a}R^{9b} or -O-C(=O)-C₁₋₆ alkyl. In another more particular embodiment, R⁵ is -CH₃, -CH₂CH₃, or -CH(CH₃)₂, each of which is substituted with one, two, or three independently selected -C(=O)-NR^{9a}R^{9b} or -O-C(=O)-C₁₋₆ alkyl. In yet another more particular embodiment, R⁵ is -CH₃, -CH₂CH₃, or -CH(CH₃)₂, each of which is substituted with one or more independently selected -C(=O)-NR^{9a}R^{9b}, -O-C(=O)-CH₃, -O-C(=O)-CH₂CH₃, -O-C(=O)-CH₂CH₂CH₃, -O-C(=O)-CH(CH₃)₂, -O-C(=O)-CH₂CH(CH₃)₂, -O-C(=O)-C(CH₃)₃, -O-C(=O)-CH(CH₃)CH₂CH₃, -O-C(=O)-CH(CH₃)CH(CH₃)₂, -O-C(=O)-CH₂CH(CH₃)CH₂CH₃, or -O-C(=O)-CH₂CH₂CH(CH₃)₂. In yet another more particular embodiment, R⁵ is C₁₋₄ alkyl substituted with one, two, or three independently selected -C(=O)-NR^{9a}R^{9b}, -O-C(=O)-CH₃, -O-C(=O)-CH₂CH₃, -O-C(=O)-CH₂CH₂CH₃, -O-C(=O)-CH(CH₃)₂, -O-C(=O)-CH₂CH(CH₃)₂, -O-C(=O)-C(CH₃)₃, -O-C(=O)-CH(CH₃)CH₂CH₃, -O-C(=O)-CH(CH₃)CH(CH₃)₂, -O-C(=O)-CH₂CH(CH₃)CH₂CH₃, or -O-C(=O)-CH₂CH₂CH(CH₃)₂. In yet another more particular embodiment, R⁵ is C₁₋₄ alkyl substituted with one or more independently selected -C(=O)-NR^{9a}R^{9b} or -O-C(=O)-C(CH₃)₃. In a further more particular embodiment, R⁵ is -CH₃ substituted with one, two, or three independently selected -C(=O)-NR^{9a}R^{9b} or -O-C(=O)-C₁₋₆ alkyl. In another further more particular embodiment, R⁵ is -CH₃, -CH₂CH₃, or -CH(CH₃)₂, each of which is substituted with one, two, or three independently selected -C(=O)-NR^{9a}R^{9b}, -O-C(=O)-CH₃, -O-C(=O)-CH₂CH₃, -O-C(=O)-CH₂CH₂CH₃, -O-C(=O)-CH(CH₃)₂, -O-C(=O)-CH₂CH(CH₃)₂, -O-C(=O)-C(CH₃)₃, -O-C(=O)-CH(CH₃)CH₂CH₃, -O-C(=O)-CH(CH₃)CH(CH₃)₂, -O-C(=O)-CH₂CH(CH₃)CH₂CH₃, or -O-C(=O)-CH₂CH₂CH(CH₃)₂. In yet another further more particular embodiment, R⁵ is C₁₋₄ alkyl substituted with one, two, or three independently selected -C(=O)-NR^{9a}R^{9b} or -O-C(=O)-C(CH₃)₃. In yet another further more particular embodiment, R⁵ is -CH₃ substituted with one or more independently selected -C(=O)-NR^{9a}R^{9b}, -O-C(=O)-CH₃, -O-C(=O)-CH₂CH₃, -O-C(=O)-CH₂CH₂CH₃, -O-C(=O)-CH(CH₃)₂, -O-C(=O)-CH₂CH(CH₃)₂, -O-C(=O)-C(CH₃)₃, -O-C(=O)-CH(CH₃)CH₂CH₃, -O-C(=O)-CH(CH₃)CH(CH₃)₂, -O-C(=O)-CH₂CH(CH₃)CH₂CH₃, or -O-C(=O)-CH₂CH₂CH(CH₃)₂. In yet another further more particular embodiment, R⁵ is -CH₃, -CH₂CH₃, or -CH(CH₃)₂, each of which is substituted with one or more independently selected -C(=O)-NR^{9a}R^{9b} or -O-C(=O)-C(CH₃)₃. In a most particular embodiment, R⁵ is -CH₃ substituted with one -C(=O)-NR^{9a}R^{9b} or -O-C(=O)-C(CH₃)₃.

In one embodiment, a compound of the invention is according to any one of Formulae I-Vc, wherein R¹ is -OR⁵, R⁵ is C₁₋₄ alkyl substituted with one or more independently selected -C(=O)-NR^{9a}R^{9b}, and each R^{9a} and R^{9b} is as previously described. In a particular embodiment, R^{9a} and R^{9b} are both H. In another particular embodiment, one of R^{9a} and R^{9b} is H, and the other is C₁₋₄ alkyl. In yet another particular embodiment, R^{9a} and R^{9b} are both C₁₋₄ alkyl. In a more particular embodiment, one of R^{9a} and R^{9b} is H, and the other is -CH₃, -CH₂CH₃, or -CH(CH₃)₂. In another more particular embodiment, R^{9a} and R^{9b} are independently -CH₃, -CH₂CH₃, or -CH(CH₃)₂. In a most particular embodiment, R^{9a} and R^{9b} are both -CH₃.

In one embodiment, a compound of the invention is according to any one of Formulae I-Vc, wherein R¹ is -NR^{6a}R^{6b}, and each R^{6a} and R^{6b} is as previously described. In a particular embodiment, R^{6a} and R^{6b} are both H. In another particular embodiment, one of R^{6a} and R^{6b} is H, and the other is -S(=O)₂-C₁₋₄ alkyl, or -S(=O)₂-C₃₋₆ cycloalkyl. In a more particular embodiment, one of R^{6a} and R^{6b} is H, and the other is -S(=O)₂-CH₃, -S(=O)₂-CH₂CH₃, -S(=O)₂-CH(CH₃)₂, -S(=O)₂-cyclopropyl, -S(=O)₂-cyclobutyl, or -S(=O)₂-cyclopentyl. In a most particular embodiment, one of R^{6a} and R^{6b} is H, and the other is -S(=O)₂-CH₃ or -S(=O)₂-cyclopropyl.

In one embodiment, a compound of the invention is according to Formula I, wherein the compound is selected from:
5-[3-(1-methylpyrazol-4-yl)pyrazolo[1,5-a]pyridin-5-yl]-2-(phenylcarbamoyl)furan-3-carboxylic acid,
5-[3-(1,3,5-trimethylpyrazol-4-yl)pyrazolo[1,5-a]pyridin-5-yl]furan-3-carboxylic acid,
5-[3-(1-methylpyrazol-4-yl)pyrazolo[1,5-a]pyridin-5-yl]furan-3-carboxylic acid,
5-[3-(3,5-dimethylisoxazol-4-yl)pyrazolo[1,5-a]pyridin-5-yl]furan-3-carboxylic acid,
methyl 5-[3-(1,3,5-trimethylpyrazol-4-yl)pyrazolo[1,5-a]pyridin-5-yl]furan-3-carboxylate,
ethyl 5-[3-(1,3,5-trimethylpyrazol-4-yl)pyrazolo[1,5-a]pyridin-5-yl]furan-3-carboxylate,
[2-(dimethylamino)-2-oxo-ethyl]5-[3-(1,3,5-trimethylpyrazol-4-yl)pyrazolo[1,5-a]pyridin-5-yl]furan-3-carboxylate,
4-methoxy-2-[3-(1,3,5-trimethylpyrazol-4-yl)pyrazolo[1,5-a]pyridin-5-yl]thiazole-5-carboxylic acid,
4-ethoxy-2-[3-(1,3,5-trimethylpyrazol-4-yl)pyrazolo[1,5-a]pyridin-5-yl]thiazole-5-carboxylic acid,
5-[3-[1-methyl-3-(trifluoromethyl)pyrazol-4-yl]pyrazolo[1,5-a]pyridin-5-yl]furan-3-carboxylic acid,
4-methoxy-2-[3-(1,3,5-trimethylpyrazol-4-yl)pyrazolo[1,5-a]pyridin-5-yl]thiazole-5-carboxamide,
5-[3-(1,5-dimethylpyrazol-4-yl)pyrazolo[1,5-a]pyridin-5-yl]furan-3-carboxylic acid,
2,2-dimethylpropanoyloxymethyl5-[3-(1,3,5-trimethylpyrazol-4-yl)pyrazolo[1,5-a]pyridin-5-yl]furan-3-carboxylate,
5-[3-(1,3-dimethylpyrazol-4-yl)pyrazolo[1,5-a]pyridin-5-yl]furan-3-carboxylic acid,
2-cyclopropyl-5-[3-(1,3,5-trimethylpyrazol-4-yl)pyrazolo[1,5-a]pyridin-5-yl]furan-3-carboxylic acid,
2-methyl-5-[3-(1,3,5-trimethylpyrazol-4-yl)pyrazolo[1,5-a]pyridin-5-yl]furan-3-carboxylic acid,
2-[3-(3,5-dimethylisoxazol-4-yl)pyrazolo[1,5-a]pyridin-5-yl]-4-ethoxy-thiazole-5-carboxylic acid,
5-[3-(1-isopropyl-3,5-dimethyl-pyrazol-4-yl)pyrazolo[1,5-a]pyridin-5-yl]furan-3-carboxylic acid,
5-[3-(3,5-dimethylisoxazol-4-yl)pyrazolo[1,5-a]pyridin-5-yl]-2-methyl-furan-3-carboxylic acid,
2-[3-(3,5-dimethylisoxazol-4-yl)pyrazolo[1,5-a]pyridin-5-yl]oxazole-4-carboxylic acid,
N-methylsulfonyl-5-[3-(1,3,5-trimethylpyrazol-4-yl)pyrazolo[1,5-a]pyridin-5-yl]furan-3-carboxamide,
N-cyclopropylsulfonyl-5-[3-(1,3,5-trimethylpyrazol-4-yl)pyrazolo[1,5-a]pyridin-5-yl]furan-3-carboxamide,
5-[3-[1,5-dimethyl-3-(trifluoromethyl)pyrazol-4-yl]pyrazolo[1,5-a]pyridin-5-yl]furan-3-carboxylic acid,
2-[3 -(3,5 -dimethylisoxazol-4-yl)pyrazolo [1,5 -a]pyridin-5 -yl]oxazole-5 -carboxylic acid,
2-[3 -(3,5 -dimethylisoxazol-4-yl)pyrazolo [1,5 -a]pyridin-5 -yl]thiazole-5 -carboxylic acid,
2-ethyl-5 -[3 -(1,3,5 -trimethylpyrazol-4-yl)pyrazolo [1,5 -a]pyridin-5 -yl]furan-3 -carboxylic acid,
2-isobutyl-5-[3-(1,3,5-trimethylpyrazol-4-yl)pyrazolo[1,5-a]pyridin-5-yl]furan-3-carboxylic acid,
5-[3-(1-ethyl-3,5-dimethyl-pyrazol-4-yl)pyrazolo[1,5-a]pyridin-5-yl]furan-3-carboxylic acid,
2-[3-(1-ethyl-3,5-dimethyl-pyrazol-4-yl)pyrazolo[1,5-a]pyridin-5-yl]thiazole-5-carboxylic acid,
2-[3-(1-isopropyl-3,5-dimethyl-pyrazol-4-yl)pyrazolo[1,5-a]pyridin-5-yl]thiazole-5-carboxylic acid,
5-[3-(3,5-dimethylisoxazol-4-yl)pyrazolo[1,5-a]pyridin-5-yl]furan-2-carboxylic acid,
2-cyclopropyl-5 - [3 -(3,5 -dimethylisoxazol-4-yl)pyrazolo [1,5 -a]pyridin-5 -yl]furan-3 -carboxylic acid,
2-[3-(1,5-dimethylpyrazol-4-yl)pyrazolo[1,5-a]pyridin-5-yl]oxazole-4-carboxylic acid,
2-[3 -(3,5 -dimethylisoxazol-4-yl)pyrazolo [1,5 -a]pyridin-5 -yl] -5 -methyl-oxazole-4-carboxylic acid,
5 -[3 -[1-(difluoromethyl)-3,5 -dimethyl-pyrazol-4-yl]pyrazolo [1,5 -a]pyridin-5 -yl]furan-3 -carboxylic acid,
2-[3-(3,5-dimethylisoxazol-4-yl)pyrazolo[1,5-a]pyridin-5-yl]-4-(trifluoromethyl)oxazole-5-carboxylic acid,
4-cyclopropyl-2- [3 -(3,5 -dimethylisoxazol-4-yl)pyrazolo [1,5 -a]pyridin-5 -yl]thiazole-5 -carboxylic acid,
4-cyclopropyl-2-[3-(1-ethyl-3,5-dimethyl-pyrazol-4-yl)pyrazolo[1,5-a]pyridin-5-yl]thiazole-5-carboxylic acid,
4-cyclopropyl-2-[3-(1-isopropyl-3,5-dimethyl-pyrazol-4-yl)pyrazolo[1,5-a]pyridin-5-yl]thiazole-5-carboxylic acid,
5-[3-[3,5-dimethyl-1-(2,2,2-trifluoroethyl)pyrazol-4-yl]pyrazolo[1,5-a]pyridin-5-yl]furan-3-carboxylic acid,
2-(3,6-dihydro-2H-pyran-4-yl)-5-[3-(3,5-dimethylisoxazol-4-yl)pyrazolo[1,5-a]pyridin-5-yl]furan-3-carboxylic acid,
4-cyclopropyl-2-[3-[3,5-dimethyl-1-(2,2,2-trifluoroethyl)pyrazol-4-yl]pyrazolo[1,5-a]pyridin-5-yl]thiazole-5-carboxylic acid,
5-[3-(1,3,5-trimethylpyrazol-4-yl)pyrazolo[1,5-a]pyridin-5-yl]furan-3-carboxamide,
5 -[3 -(3,5 -dimethylisoxazol-4-yl)pyrazolo[1,5-a]pyridin-5 -yl] -2-tetrahydropyran-4-yl-furan-3 -carboxylic acid,
4-(difluoromethyl)-2-[3 -(3,5 -dimethylisoxazol-4-yl)pyrazolo [1,5 -a]pyridin-5 -yl]thiazole-5 -carboxylic acid,
4-(difluoromethyl)-2-[3-(1-isopropyl-3,5-dimethyl-pyrazol-4-yl)pyrazolo[1,5-a]pyridin-5-yl]thiazole-5-carboxylic acid,
1-[3-(3,5-dimethylisoxazol-4-yl)pyrazolo[1,5-a]pyridin-5-yl]pyrazole-4-carboxylic acid,
5-[3-(1-isopropyl-3,5-dimethyl-pyrazol-4-yl)pyrazolo[1,5-a]pyridin-5-yl]furan-2-carboxylic acid,
2-[3-[1-(difluoromethyl)-3,5-dimethyl-pyrazol-4-yl]pyrazolo[1,5-a]pyridin-5-yl]oxazole-5-carboxylic acid,
2-[3-[1-(difluoromethyl)-3,5-dimethyl-pyrazol-4-yl]pyrazolo[1,5-a]pyridin-5-yl]thiazole-5-carboxylic acid,
5-[3-[1-(difluoromethyl)-3,5-dimethyl-pyrazol-4-yl]pyrazolo[1,5-a]pyridin-5-yl]furan-2-carboxylic acid,
1-[3-(1-isopropyl-3,5-dimethyl-pyrazol-4-yl)pyrazolo[1,5-a]pyridin-5-yl]pyrazole-4-carboxylic acid,
2-(3,6-dihydro-2H-pyran-4-yl)-5-[3-[3,5-dimethyl-1-(2,2,2-trifluoroethyl)pyrazol-4-yl]pyrazolo[1,5-a]pyridin-5-yl]furan-3-carboxylic acid,
5-[3-[1-(difluoromethyl)-3,5-dimethyl-pyrazol-4-yl]pyrazolo[1,5-a]pyridin-5-yl]-2-(3,6-dihydro-2H-pyran-4-yl)furan-3-carboxylic acid,
5-[3-[3,5-dimethyl-1-(2,2,2-trifluoroethyl)pyrazol-4-yl]pyrazolo[1,5-a]pyridin-5-yl]-2-tetrahydropyran-4-yl-furan-3-carboxylic acid,
2-[3-(3,5-dimethylisoxazol-4-yl)pyrazolo[1,5-a]pyridin-5-yl]-4-isopropoxy-thiazole-5-carboxylic acid,
2-[3-[1,5-dimethyl-3-(trifluoromethyl)pyrazol-4-yl]pyrazolo[1,5-a]pyridin-5-yl]oxazole-4-carboxylic acid,
2-[3-(3,5-dimethylisoxazol-4-yl)pyrazolo[1,5-a]pyridin-5-yl]-4-(oxetan-3-yloxy)thiazole-5-carboxylic acid,
2-[3-(3,5-dimethylisoxazol-4-yl)pyrazolo[1,5-a]pyridin-5-yl]-4-(2-methoxyethoxy)thiazole-5-carboxylic acid,
2-[3-(3,5-dimethylisoxazol-4-yl)pyrazolo[1,5-a]pyridin-5-yl]-4-(2-ethoxyethoxy)thiazole-5-carboxylic acid,
4-(difluoromethoxy)-2-[3-(3,5-dimethylisoxazol-4-yl)pyrazolo[1,5-a]pyridin-5-yl]thiazole-5-carboxylic acid,
1-[3-[3,5-dimethyl-1-(2,2,2-trifluoroethyl)pyrazol-4-yl]pyrazolo[1,5-a]pyridin-5-yl]pyrazole-4-carboxylic acid,
1-[3-(3,5-dimethylisoxazol-4-yl)pyrazolo[1,5-a]pyridin-5-yl]-3-methoxy-pyrazole-4-carboxylic acid,
4-ethoxy-2-[3-(1-ethyl-3,5-dimethyl-pyrazol-4-yl)pyrazolo[1,5-a]pyridin-5-yl]thiazole-5-carboxylicacid,
4-ethoxy-2-[3-(1-isopropyl-3,5-dimethyl-pyrazol-4-yl)pyrazolo[1,5-a]pyridin-5-yl]thiazole-5-carboxylic acid,
1-[3-(1-isopropyl-3,5-dimethyl-pyrazol-4-yl)pyrazolo[1,5-a]pyridin-5-yl]-3-methoxy-pyrazole-4-carboxylic acid,
1-[3-[1,5-dimethyl-3-(trifluoromethyl)pyrazol-4-yl]pyrazolo[1,5-a]pyridin-5-yl]pyrazole-4-carboxylic acid,
1-[3-[3,5-dimethyl-1-(2,2,2-trifluoroethyl)pyrazol-4-yl]pyrazolo[1,5-a]pyridin-5-yl]-3-methoxy-pyrazole-4-carboxylic acid,
1-[3-[1-(difluoromethyl)-3,5-dimethyl-pyrazol-4-yl]pyrazolo[1,5-a]pyridin-5-yl]pyrazole-4-carboxylic acid,
2-[3-[3,5-dimethyl-1-(2,2,2-trifluoroethyl)pyrazol-4-yl]pyrazolo[1,5-a]pyridin-5-yl]-4-ethoxy-thiazole-5-carboxylic acid,
1-[3-[1-(difluoromethyl)-3,5-dimethyl-pyrazol-4-yl]pyrazolo[1,5-a]pyridin-5-yl]-3-methoxy-pyrazole-4-carboxylic acid,
2-[3-[1-(difluoromethyl)-3,5-dimethyl-pyrazol-4-yl]pyrazolo[1,5-a]pyridin-5-yl]-4-ethoxy-thiazole-5-carboxylic acid,
1-[3-(1,3,5-trimethylpyrazol-4-yl)pyrazolo[1,5-a]pyridin-5-yl]pyrazole-4-carboxylic acid,
2-[3-(1-isopropyl-3,5-dimethyl-pyrazol-4-yl)pyrazolo[1,5-a]pyridin-5-yl]-4-methoxy-thiazole-5-carboxylic acid,
1-[3-(1-ethyl-3,5-dimethyl-pyrazol-4-yl)pyrazolo[1,5-a]pyridin-5-yl]-3-methoxy-pyrazole-4-carboxylic acid,
1-[3-[1-isopropyl-5-methyl-3-(trifluoromethyl)pyrazol-4-yl]pyrazolo[1,5-a]pyridin-5-yl]pyrazole-4-carboxylic acid,
2-[3-[3,5-dimethyl-1-(2,2,2-trifluoroethyl)pyrazol-4-yl]pyrazolo[1,5-a]pyridin-5-yl]-4-methoxy-thiazole-5-carboxylic acid,
1-[3-[1,5-dimethyl-3-(trifluoromethyl)pyrazol-4-yl]pyrazolo[1,5-a]pyridin-5-yl]-3-methoxy-pyrazole-4-carboxylic acid,
2-[3-[1-(difluoromethyl)-3,5-dimethyl-pyrazol-4-yl]pyrazolo[1,5-a]pyridin-5-yl]-4-methoxy-thiazole-5-carboxylic acid,
1-[3-(1-isopropyl-3,5-dimethyl-pyrazol-4-yl)pyrazolo[1,5-a]pyridin-5-yl]-3-(trifluoromethyl)pyrazole-4-carboxylic acid,
1-[3-[3,5-dimethyl-1-(2,2,2-trifluoroethyl)pyrazol-4-yl]pyrazolo[1,5-a]pyridin-5-yl]-3-(trifluoromethyl)pyrazole-4-carboxylic acid,
1-[3-(1-ethyl-3,5-dimethyl-pyrazol-4-yl)pyrazolo[1,5-a]pyridin-5-yl]-3-methoxy-pyrazole-4-carboxamide,
1-[3-[1-(difluoromethyl)-3,5-dimethyl-pyrazol-4-yl]pyrazolo[1,5-a]pyridin-5-yl]-3-methoxy-pyrazole-4-carboxamide,
2-[3-(1-ethyl-3,5-dimethyl-pyrazol-4-yl)pyrazolo[1,5-a]pyridin-5-yl]-4-methoxy-thiazole-5-carboxylic acid,
2-[3-[3,5-dimethyl-1-(2,2,2-trifluoro-1-methyl-ethyl)pyrazol-4-yl]pyrazolo[1,5-a]pyridin-5-yl]-4-methoxy-thiazole-5-carboxylic acid,
4-cyclopropyl-2-[3-(1,3,5-trimethylpyrazol-4-yl)pyrazolo[1,5-a]pyridin-5-yl]thiazole-5-carboxylic acid,
ethyl 4-methoxy-2-[3-(1,3,5-trimethylpyrazol-4-yl)pyrazolo[1,5-a]pyridin-5-yl]thiazole-5-carboxylate,
ethyl 4-ethoxy-2-[3-(1,3,5-trimethylpyrazol-4-yl)pyrazolo[1,5-a]pyridin-5-yl]thiazole-5-carboxylate,
2-[3-[1,5-dimethyl-3-(trifluoromethyl)pyrazol-4-yl]pyrazolo[1,5-a]pyridin-5-yl]-4-methoxy-thiazole-5-carboxylic acid,
4-(2,2,2-trifluoroethoxy)-2-[3-(1,3,5-trimethylpyrazol-4-yl)pyrazolo[1,5-a]pyridin-5-yl]thiazole-5-carboxylic acid,
methyl 5-[3-(1-methylpyrazol-4-yl)pyrazolo[1,5-a]pyridin-5-yl]furan-3-carboxylate,
methyl 5-[3-(3,5-dimethylisoxazol-4-yl)pyrazolo[1,5-a]pyridin-5-yl]furan-3-carboxylate,
ethyl 5-[3-(3,5-dimethylisoxazol-4-yl)pyrazolo[1,5-a]pyridin-5-yl]furan-3-carboxylate,
ethyl 4-methoxy-2-[3-(1,3,5-trimethylpyrazol-4-yl)pyrazolo[1,5-a]pyridin-5-yl]thiazole-5-carboxylate,
ethyl 4-ethoxy-2-[3-(1,3,5-trimethylpyrazol-4-yl)pyrazolo[1,5-a]pyridin-5-yl]thiazole-5-carboxylate,
methyl 5-[3-[1-methyl-3-(trifluoromethyl)pyrazol-4-yl]pyrazolo[1,5-a]pyridin-5-yl]furan-3-carboxylate,
methyl 5-[3-(1,5-dimethylpyrazol-4-yl)pyrazolo[1,5-a]pyridin-5-yl]furan-3-carboxylate,
ethyl 5-[3-(1,3-dimethylpyrazol-4-yl)pyrazolo[1,5-a]pyridin-5-yl]furan-3-carboxylate,
methyl 2-cyclopropyl-5-[3-(1,3,5-trimethylpyrazol-4-yl)pyrazolo[1,5-a]pyridin-5-yl]furan-3-carboxylate,
methyl 2-methyl-5-[3-(1,3,5-trimethylpyrazol-4-yl)pyrazolo[1,5-a]pyridin-5-yl]furan-3-carboxylate,
ethyl 2-[3-(3,5-dimethylisoxazol-4-yl)pyrazolo[1,5-a]pyridin-5-yl]-4-ethoxy-thiazole-5-carboxylate,
ethyl 5-[3-(1-isopropyl-3,5-dimethyl-pyrazol-4-yl)pyrazolo[1,5-a]pyridin-5-yl]furan-3-carboxylate,
methyl 5-[3-(3,5-dimethylisoxazol-4-yl)pyrazolo[1,5-a]pyridin-5-yl]-2-methyl-furan-3 -carboxylate,
ethyl 2-[3-(3,5-dimethylisoxazol-4-yl)pyrazolo[1,5-a]pyridin-5-yl]oxazole-4-carboxylate,
ethyl 5-[3-[1,5-dimethyl-3-(trifluoromethyl)pyrazol-4-yl]pyrazolo[1,5-a]pyridin-5-yl]furan-3-carboxylate,
ethyl 2-[3-(3,5-dimethylisoxazol-4-yl)pyrazolo[1,5-a]pyridin-5-yl]oxazole-5-carboxylate,
methyl 2-[3-(3,5-dimethylisoxazol-4-yl)pyrazolo[1,5-a]pyridin-5-yl]thiazole-5-carboxylate,
methyl 2-ethyl-5-[3-(1,3,5-trimethylpyrazol-4-yl)pyrazolo[1,5-a]pyridin-5-yl]furan-3-carboxylate,
methyl 2-isobutyl-5-[3-(1,3,5-trimethylpyrazol-4-yl)pyrazolo[1,5-a]pyridin-5-yl]furan-3-carboxylate,
ethyl 5-[3-(1-ethyl-3,5-dimethyl-pyrazol-4-yl)pyrazolo[1,5-a]pyridin-5-yl]furan-3-carboxylate,
methyl 2-[3-(1-ethyl-3,5-dimethyl-pyrazol-4-yl)pyrazolo[1,5-a]pyridin-5-yl]thiazole-5-carboxylate,
methyl 2-[3 -(1-isopropyl-3,5 -dimethyl-pyrazol-4-yl)pyrazolo [1,5 -a]pyridin-5 -yl]thiazole-5 -carboxylate,
methyl 5 -[3 -(3,5 -dimethylisoxazol-4-yl)pyrazolo [1,5 -a]pyridin-5 -yl]furan-2-carboxylate,
ethyl 2-cyclopropyl-5-[3-(3,5-dimethylisoxazol-4-yl)pyrazolo[1,5-a]pyridin-5-yl]furan-3-carboxylate,
ethyl 2-[3 -(1,2-dimethylpyrrol-3 -yl)pyrazolo [1,5 -a]pyridin-5 -yl]oxazole-4-carboxylate,
ethyl 2-[3 -(3,5 -dimethylisoxazol-4-yl)pyrazolo[1,5-a]pyridin-5 -yl] -5 -methyl-oxazole-4-carboxylate,
ethyl 5-[3-[1-(difluoromethyl)-3,5-dimethyl-pyrazol-4-yl]pyrazolo[1,5-a]pyridin-5-yl]furan-3-carboxylate,
ethyl 4-cyclopropyl-2-[3-(3,5-dimethylisoxazol-4-yl)pyrazolo[1,5-a]pyridin-5-yl]thiazole-5-carboxylate,
ethyl 4-cyclopropyl-2-[3-(1-ethyl-3,5-dimethyl-pyrazol-4-yl)pyrazolo[1,5-a]pyridin-5-yl]thiazole-5-carboxylate,
ethyl 4-cyclopropyl-2-[3 -(1-isopropyl-3,5 -dimethyl-pyrazol-4-yl)pyrazolo [1,5 -a]pyridin-5 -yl]thiazole-5 - carboxylate,
ethyl 5-[3-[3,5-dimethyl-1-(2,2,2-trifluoroethyl)pyrazol-4-yl]pyrazolo[1,5-a]pyridin-5-yl]furan-3-carboxylate,
ethyl 2-(3,6-dihydro-2H-pyran-4-yl)-5-[3-(3,5-dimethylisoxazol-4-yl)pyrazolo[1,5-a]pyridin-5-yl]furan-3-carboxylate,
ethyl 4-cyclopropyl-2-[3-[3,5-dimethyl-1-(2,2,2-trifluoroethyl)pyrazol-4-yl]pyrazolo[1,5-a]pyridin-5-yl]thiazole-5-carboxylate,
ethyl 5 -[3 -(3,5 -dimethylisoxazol-4-yl)pyrazolo[1,5-a]pyridin-5 -yl] -2-tetrahydropyran-4-yl-furan-3 - carboxylate,
ethyl 4-(difluoromethyl)-2-[3-(3,5-dimethylisoxazol-4-yl)pyrazolo[1,5-a]pyridin-5-yl]thiazole-5-carboxylate,
ethyl 4-(difluoromethyl)-2-[3-(1-isopropyl-3,5-dimethyl-pyrazol-4-yl)pyrazolo[1,5-a]pyridin-5-yl]thiazole-5-carboxylate,
ethyl 1-[3-(3,5-dimethylisoxazol-4-yl)pyrazolo[1,5-a]pyridin-5-yl]pyrazole-4-carboxylate,
methyl 5-[3-(1-isopropyl-3,5-dimethyl-pyrazol-4-yl)pyrazolo[1,5-a]pyridin-5-yl]furan-2-carboxylate,
ethyl 2-[3-[1-(difluoromethyl)-3,5-dimethyl-pyrazol-4-yl]pyrazolo[1,5-a]pyridin-5-yl]oxazole-5-carboxylate,
methyl 2-[3-[1-(difluoromethyl)-3,5-dimethyl-pyrazol-4-yl]pyrazolo[1,5-a]pyridin-5-yl]thiazole-5-carboxylate,
methyl 5-[3-[1-(difluoromethyl)-3,5-dimethyl-pyrazol-4-yl]pyrazolo[1,5-a]pyridin-5-yl]furan-2-carboxylate,
ethyl 5-[3-[1-(difluoromethyl)-3,5-dimethyl-pyrazol-4-yl]pyrazolo[1,5-a]pyridin-5-yl]furan-2-carboxylate,
ethyl 1-[3-(1-isopropyl-3,5-dimethyl-pyrazol-4-yl)pyrazolo[1,5-a]pyridin-5-yl]pyrazole-4-carboxylate,
ethyl 2-(3,6-dihydro-2H-pyran-4-yl)-5-[3-[3,5-dimethyl-1-(2,2,2-trifluoroethyl)pyrazol-4-yl]pyrazolo[1,5-a]pyridin-5-yl]furan-3-carboxylate,
ethyl 5-[3-[1-(difluoromethyl)-3,5-dimethyl-pyrazol-4-yl]pyrazolo[1,5-a]pyridin-5-yl]-2-(3,6-dihydro-2H-pyran-4-yl)furan-3-carboxylate,
ethyl 5-[3-[1-(difluoromethyl)-3,5-dimethyl-pyrazol-4-yl]pyrazolo[1,5-a]pyridin-5-yl]-2-tetrahydropyran-4-yl-furan-3 -carboxylate,
ethyl 2-[3 -(3,5 -dimethylisoxazol-4-yl)pyrazolo[1,5-a]pyridin-5 -yl] -4-isopropoxy-thiazole-5 -carboxylate,
ethyl 2-[3-[1,5-dimethyl-3-(trifluoromethyl)pyrazol-4-yl]pyrazolo[1,5-a]pyridin-5-yl]oxazole-4-carboxylate,
ethyl 2-[3 -(3,5 -dimethylisoxazol-4-yl)pyrazolo[1,5-a]pyridin-5 -yl] -4-(oxetan-3 -yloxy)thiazole-5 - carboxylate,
ethyl 2-[3-(3,5-dimethylisoxazol-4-yl)pyrazolo[1,5-a]pyridin-5-yl]-4-(2-methoxyethoxy)thiazole-5-carboxylate,
ethyl 2-[3 -(3,5 -dimethylisoxazol-4-yl)pyrazolo[1,5-a]pyridin-5 -yl] -4-(2-ethoxyethoxy)thiazole-5 - carboxylate,
ethyl 4-(difluoromethoxy)-2-[3-(3,5-dimethylisoxazol-4-yl)pyrazolo[1,5-a]pyridin-5-yl]thiazole-5-carboxylate,
ethyl 1-[3-[3,5-dimethyl-1-(2,2,2-trifluoroethyl)pyrazol-4-yl]pyrazolo[1,5-a]pyridin-5-yl]pyrazole-4-carboxylate,
ethyl 1-[3-(3,5-dimethylisoxazol-4-yl)pyrazolo[1,5-a]pyridin-5-yl]-3-methoxy-pyrazole-4-carboxylate,
ethyl 4-ethoxy-2-[3-(1-ethyl-3,5-dimethyl-pyrazol-4-yl)pyrazolo[1,5-a]pyridin-5-yl]thiazole-5-carboxylate,
ethyl 4-ethoxy-2-[3-(1-isopropyl-3,5-dimethyl-pyrazol-4-yl)pyrazolo[1,5-a]pyridin-5-yl]thiazole-5 - carboxylate,
ethyl 1-[3-(1-isopropyl-3,5-dimethyl-pyrazol-4-yl)pyrazolo[1,5-a]pyridin-5-yl]-3-methoxy-pyrazole-4-carboxylate,
ethyl 1-[3-[1,5-dimethyl-3-(trifluoromethyl)pyrazol-4-yl]pyrazolo[1,5-a]pyridin-5-yl]pyrazole-4-carboxylate,
ethyl 1-[3-[3,5-dimethyl-1-(2,2,2-trifluoroethyl)pyrazol-4-yl]pyrazolo[1,5-a]pyridin-5-yl]-3-methoxy-pyrazole-4-carboxylate,
ethyl 1-[3-[1-(difluoromethyl)-3,5-dimethyl-pyrazol-4-yl]pyrazolo[1,5-a]pyridin-5-yl]pyrazole-4-carboxylate,
ethyl 2-[3-[3,5-dimethyl-1-(2,2,2-trifluoroethyl)pyrazol-4-yl]pyrazolo[1,5-a]pyridin-5-yl]-4-ethoxy-thiazole-5 -carboxylate,
ethyl 1-[3-[1-(difluoromethyl)-3,5-dimethyl-pyrazol-4-yl]pyrazolo[1,5-a]pyridin-5-yl]-3-methoxy-pyrazole-4-carboxylate,
ethyl 2-[3-[1-(difluoromethyl)-3,5-dimethyl-pyrazol-4-yl]pyrazolo[1,5-a]pyridin-5-yl]-4-ethoxy-thiazole-5-carboxylate,
ethyl 1-[3 -(1,3,5 -trimethylpyrazol-4-yl)pyrazolo [1,5 -a]pyridin-5 -yl]pyrazole-4-carboxylate,
ethyl 2-[3-(1-isopropyl-3,5-dimethyl-pyrazol-4-yl)pyrazolo[1,5-a]pyridin-5-yl]-4-methoxy-thiazole-5-carboxylate,
ethyl 2-[3 -(3,5 -dimethylisoxazol-4-yl)pyrazolo[1,5-a]pyridin-5 -yl] -4-methoxy-thiazole-5 -carboxylate,
ethyl 1-[3 -(1 -ethyl-3,5 -dimethyl-pyrazol-4-yl)pyrazolo[1,5-a]pyridin-5 -yl] -3 -methoxy-pyrazole-4-carboxylate,
ethyl 1-[3-[1-isopropyl-5-methyl-3-(trifluoromethyl)pyrazol-4-yl]pyrazolo[1,5-a]pyridin-5-yl]pyrazole-4-carboxylate,
ethyl 2-[3-[3,5-dimethyl-1-(2,2,2-trifluoroethyl)pyrazol-4-yl]pyrazolo[1,5-a]pyridin-5-yl]-4-methoxy-thiazole-5 -carboxylate,
ethyl 1-[3-[1,5-dimethyl-3-(trifluoromethyl)pyrazol-4-yl]pyrazolo[1,5-a]pyridin-5-yl]-3-methoxy-pyrazole-4-carboxylate,
ethyl 2-[3-[1-(difluoromethyl)-3,5-dimethyl-pyrazol-4-yl]pyrazolo[1,5-a]pyridin-5-yl]-4-methoxy-thiazole-5 -carboxylate,
ethyl 1-[3-('1-isopropyl-3,5-dimethyl-pyrazol-4-yl)pyrazolo[1,5-a]pyridin-5-yl]-3-(trifluoromethyl)pyrazole-4-carboxylate,
ethyl 1-[3 -(3,5 -dimethylisoxazol-4-yl)pyrazolo[1,5-a]pyridin-5 -yl] -3 -(trifluoromethyl)pyrazole-4-carboxylate,
ethyl 1-[3-[3,5-dimethyl-1-(2,2,2-trifluoroethyl)pyrazol-4-yl]pyrazolo[1,5-a]pyridin-5-yl]-3-(trifluoromethyl)pyrazole-4-carboxylate,
ethyl 2-[3-(1-ethyl-3,5-dimethyl-pyrazol-4-yl)pyrazolo[1,5-a]pyridin-5-yl]-4-methoxy-thiazole-5-carboxylate,
ethyl 2-[3-[3,5-dimethyl-1-(2,2,2-trifluoro-1-methyl-ethyl)pyrazol-4-yl]pyrazolo[1,5-a]pyridin-5-yl] -4-methoxy-thiazole-5 -carboxylate,
ethyl 4-cyclopropyl-2-[3-(1,3,5-trimethylpyrazol-4-yl)pyrazolo[1,5-a]pyridin-5-yl]thiazole-5-carboxylate,
ethyl 2-[3-[1,5-dimethyl-3-(trifluoromethyl)pyrazol-4-yl]pyrazolo[1,5-a]pyridin-5-yl]-4-methoxy-thiazole-5-carboxylate, and
ethyl 4-(2,2,2-trifluoroethoxy)-2-[3-(1,3,5-trimethylpyrazol-4-yl)pyrazolo[1,5-a]pyridin-5-yl]thiazole-5-carboxylate.

In one embodiment, a compound of the invention is according to Formula I, wherein the compound is 5 -[3 -(1 -isopropyl-3,5 -dimethyl-pyrazol-4-yl)pyrazolo [1,5 -a]pyridin-5 -yl]furan-3 -carboxylic acid.

In one embodiment, a compound of the invention is according to Formula I, wherein the compound is not 5-[3-(1-isopropyl-3,5-dimethyl-pyrazol-4-yl)pyrazolo[1,5-a]pyridin-5-yl]furan-3-carboxylic acid.

In one embodiment, a compound of the invention is according to Formula I, wherein the compound is 5-[3-[3,5-dimethyl-1-(2,2,2-trifluoroethyl)pyrazol-4-yl]pyrazolo[1,5-a]pyridin-5-yl]furan-3-carboxylic acid.

In one embodiment, a compound of the invention is according to Formula I, wherein the compound is not 5-[3-[3,5-dimethyl-1-(2,2,2-trifluoroethyl)pyrazol-4-yl]pyrazolo[1,5-a]pyridin-5-yl]furan-3-carboxylic acid.

In one embodiment, the compounds of the invention are provided in a natural isotopic form.

In one embodiment, the compounds of the invention are provided in an unnatural variant isotopic form. In a specific embodiment, the unnatural variant isotopic form is a form in which deuterium (i.e. ²H or D) is incorporated where hydrogen is specified in the chemical structure in one or more atoms of a compound of the invention. In one embodiment, the atoms of the compounds of the invention are in an isotopic form which is not radioactive. In one embodiment, one or more atoms of the compounds of the invention are in an isotopic form which is radioactive. Suitably radioactive isotopes are stable isotopes. Suitably the unnatural variant isotopic form is a pharmaceutically acceptable form.

In one embodiment, a compound of the invention is provided whereby a single atom of the compound exists in an unnatural variant isotopic form. In another embodiment, a compound of the invention is provided whereby two or more atoms exist in an unnatural variant isotopic form.

Unnatural isotopic variant forms can generally be prepared by conventional techniques known to those skilled in the art or by processes described herein e.g. processes analogous to those described in the accompanying Examples for preparing natural isotopic forms. Thus, unnatural isotopic variant forms could be prepared by using appropriate isotopically variant (or labelled) reagents in place of the normal reagents employed in the illustrative example as examples.

In one aspect a compound of the invention according to any one of the embodiments herein described is present as the free base.

In one aspect a compound of the invention according to any one of the embodiments herein described is a pharmaceutically acceptable salt.

In one aspect a compound of the invention according to any one of the embodiments herein described is a solvate of the compound.

In one aspect a compound of the invention according to any one of the embodiments herein described is a solvate of a pharmaceutically acceptable salt of a compound.

While specified groups for each embodiment have generally been listed above separately, a compound of the invention includes one in which several or each embodiment in the above Formula, as well as other formulae presented herein, is selected from one or more of particular members or groups designated respectively, for each variable. Therefore, this invention is intended to include all combinations of such embodiments within its scope.

While specified groups for each embodiment have generally been listed above separately, a compound of the invention may be one for which one or more variables (for example, R groups) is selected from one or more embodiments according to any of the Formula(e) listed above. Therefore, the present invention is intended to include all combinations of variables from any of the disclosed embodiments within its scope.

Alternatively, the exclusion of one or more of the specified variables from a group or an embodiment, or combinations thereof is also contemplated by the present invention.

Other derivatives of the compounds of this invention have activity in both their acid and acid derivative forms, but the acid sensitive form often offers advantages of solubility, tissue compatibility, or delayed release in the mammalian organism (Bundgaard 1985). Prodrugs include acid derivatives well known to practitioners of the art, such as, for example, esters prepared by reaction of the parent acid with a suitable alcohol, or amides prepared by reaction of the parent acid compound with a substituted or unsubstituted amine, or acid anhydrides, or mixed anhydrides. Simple aliphatic or aromatic esters, amides and anhydrides derived from acidic groups pendant on the compounds of this invention are preferred prodrugs. In some cases it is desirable to prepare double ester type prodrugs such as (acyloxy)alkyl esters or ((alkoxycarbonyl)oxy)alkylesters. Particularly useful are the C1 to C8 alkyl, C2-C8 alkenyl, aryl, C7-C 12 substituted aryl, and C7-C12 arylalkyl esters of the compounds of the invention.

Embodiments according to the invention are provided as set out below.
Embodiment 1 provides a compound according to Formula I: 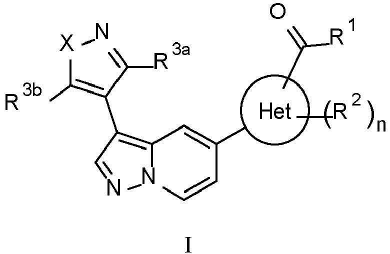 wherein,
   Xis O or NR⁴;
   n is 0, 1, or 2;
   Het is 5 membered monocyclic heteroaryl comprising one, two or three heteroatoms independently selected from N, O, and S;
   R¹ is -OR⁵ or -NR^{6a}R^{6b};
   each R² is independently selected from
      -O-R⁷,
      C₁₋₆ alkyl optionally substituted with one or more independently selected halo,
      C₃₋₆ cycloalkyl,
      -C(=O)-NR^{8a}R^{8b},
      4-6 membered monocyclic heterocycloalkyl comprising one, two or three heteroatoms independently selected from N, O, and S, and
      4-6 membered monocyclic heterocycloalkenyl comprising one double bond and further comprising one, or two heteroatoms independently selected from N, O, and S;
   R^{3a} and R^{3b} are independently H or C₁₋₃ alkyl optionally substituted with one or more independently selected halo;
   R⁴ is C₁₋₃ alkyl optionally substituted with one or more F;
   R⁵ is H or C₁₋₄ alkyl optionally substituted with one or more independently selected -C(=O)-NR^{9a}R^{9b} or -O-C(=O)-C₁₋₆ alkyl;
   R^{6a} and R^{6b} are independently H, -S(=O)₂-C₁₋₄ alkyl, or -S(=O)₂-C₃₋₆ cycloalkyl;
   each R⁷ is independently selected from
      C₁₋₆ alkyl optionally substituted with one or more independently selected halo or C₁₋₄ alkoxy, and
      4-6 membered monocyclic heterocycloalkyl comprising one, two or three heteroatoms independently selected from N, O, and S;
   R^{8a} and R^{8b} are independently H, C₁₋₄ alkyl, or phenyl; and
   R^{9a} and R^{9b} are independently H or C₁₋₄ alkyl;
   or a pharmaceutically acceptable salt, solvate, or salt of a solvate thereof.
Embodiment 2 provides a compound or pharmaceutically acceptable salt thereof, according to embodiment 1, wherein Het is pyrrolyl, furanyl, thiophenyl, pyrazolyl, imidazolyl, oxazolyl, isoxazolyl, thiazolyl, isothiazolyl, triazolyl, furazanyl, oxadiazolyl, or thiadiazolyl.
Embodiment 3 provides a compound or pharmaceutically acceptable salt thereof, according to embodiment 1, wherein Het is pyrrolyl, furanyl, thiophenyl, pyrazolyl, imidazolyl, oxazolyl, isoxazolyl, thiazolyl, isothiazolyl, triazolyl, 1,2,4-oxadiazolyl, 1,3,4-oxadiazolyl, 1,2,4-thiadiazolyl, or 1,3,4-thiadiazolyl.
Embodiment 4 provides a compound or pharmaceutically acceptable salt thereof, according to embodiment 1, wherein Het is furanyl, pyrazolyl, oxazolyl, or thiazolyl.
Embodiment 5 provides a compound or pharmaceutically acceptable salt thereof, according to embodiment 1, wherein Het is furanyl or thiazolyl.
Embodiment 6 provides a compound or pharmaceutically acceptable salt thereof, according to embodiment 1, wherein Het is furanyl.
Embodiment 7 provides a compound or pharmaceutically acceptable salt thereof, according to embodiment 1, wherein the compound is according to Formula IIa, IIb, IIc, or IId: 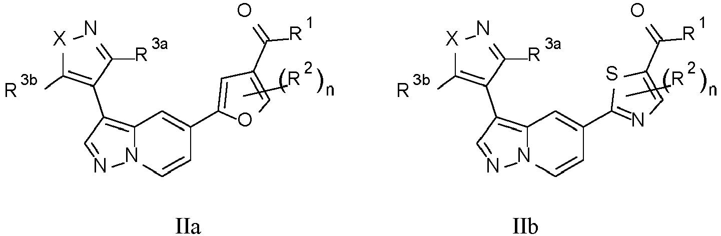 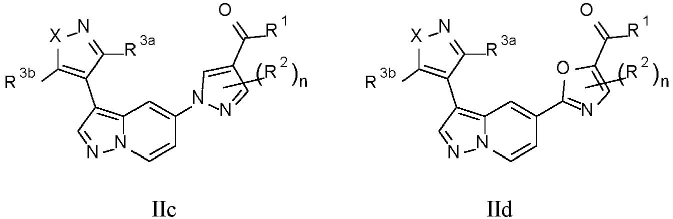
Embodiment 8 provides a compound or pharmaceutically acceptable salt thereof, according to any one of embodiments 1-7, wherein R^{3a} is H.
Embodiment 9 provides a compound or pharmaceutically acceptable salt thereof, according to any one of embodiments 1-7, wherein R^{3a} is -CH₃, -CH₂CH₃, or -CH(CH₃)₂.
Embodiment 10 provides a compound or pharmaceutically acceptable salt thereof, according to any one of embodiments 1-7, wherein R^{3a} is -CH₃.
Embodiment 11 provides a compound or pharmaceutically acceptable salt thereof, according to any one of embodiments 1-7, wherein R^{3a} is -CH₃, -CH₂CH₃, or -CH(CH₃)₂, each of which is substituted with one, two or three independently selected F or Cl.
Embodiment 12 provides a compound or pharmaceutically acceptable salt thereof, according to any one of embodiments 1-7, wherein R^{3a} is -CH₃ substituted with one, two, or three F.
Embodiment 13 provides a compound or pharmaceutically acceptable salt thereof, according to any one of embodiments 1-7, wherein R^{3a} is -CF₃.
Embodiment 14 provides a compound or pharmaceutically acceptable salt thereof, according to any one of embodiments 1-13, wherein R^{3b} is H.
Embodiment 15 provides a compound or pharmaceutically acceptable salt thereof, according to any one of embodiments 1-13, wherein R^{3b} is -CH₃, -CH₂CH₃, or -CH(CH₃)₂.
Embodiment 16 provides a compound or pharmaceutically acceptable salt thereof, according to any one of embodiments 1-13, wherein R^{3b} is -CH₃.
Embodiment 17 provides a compound or pharmaceutically acceptable salt thereof, according to any one of embodiments 1-13, wherein R^{3b} is -CH₃, -CH₂CH₃, or -CH(CH₃)₂, each of which is substituted with one, two or three independently selected F or Cl.
Embodiment 18 provides a compound or pharmaceutically acceptable salt thereof, according to any one of embodiments 1-13, wherein R^{3b} is -CH₃ substituted with one, two, or three F.
Embodiment 19 provides a compound or pharmaceutically acceptable salt thereof, according to any one of embodiments 1-13, wherein R^{3b} is -CF₃.
Embodiment 20 provides a compound or pharmaceutically acceptable salt thereof, according to any one of embodiments 1-7, wherein R^{3a} and R^{3b} are both -CH₃.
Embodiment 21 provides a compound or pharmaceutically acceptable salt thereof, according to any one of embodiments 1-20, wherein n is 0 or 1.
Embodiment 22 provides a compound or pharmaceutically acceptable salt thereof, according to any one of embodiments 1-20, wherein n is 0.
Embodiment 23 provides a compound or pharmaceutically acceptable salt thereof, according to embodiment 1, wherein the compound is according to Formula IIIa, IIIb, IIIc, or IIId: 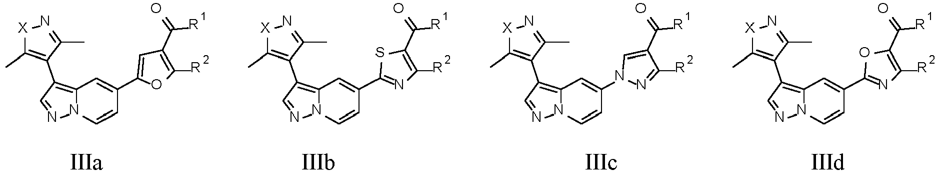
Embodiment 24 provides a compound or pharmaceutically acceptable salt thereof, according to any one of embodiments 1-23, wherein R² is -O-R⁷.
Embodiment 25 provides a compound or pharmaceutically acceptable salt thereof, according to any one of embodiments 1-24, wherein R⁷ is C₁₋₆ alkyl.
Embodiment 26 provides a compound or pharmaceutically acceptable salt thereof, according to any one of embodiments 1-24, wherein R⁷ is -CH₃, -CH₂CH₃, -CH₂CH₂CH₃, -CH(CH₃)₂, -CH₂CH(CH₃)₂, -C(CH₃)₃, -CH(CH₃)CH₂CH₃, or -CH(CH₃)CH(CH₃)₂.
Embodiment 27 provides a compound or pharmaceutically acceptable salt thereof, according to any one of embodiments 1-24, wherein R⁷ is -CH₃, -CH₂CH₃, or -CH(CH₃)₂.
Embodiment 28 provides a compound or pharmaceutically acceptable salt thereof, according to any one of embodiments 1-24, wherein R⁷ is -CH₂CH₃.
Embodiment 29 provides a compound or pharmaceutically acceptable salt thereof, according to any one of embodiments 1-24, wherein R⁷ is C₁₋₆ alkyl substituted with one or more independently selected halo or C₁₋₄ alkoxy.
Embodiment 30 provides a compound or pharmaceutically acceptable salt thereof, according to any one of embodiments 1-24, wherein R⁷ is -CH₃, -CH₂CH₃, -CH₂CH₂CH₃, -CH(CH₃)₂, -CH₂CH(CH₃)₂, -C(CH₃)₃, -CH(CH₃)CH₂CH₃, or -CH(CH₃)CH(CH₃)₂, each of which is substituted with one, two, or three independently selected F, Cl, -O-CH₃, -O-CH₂CH₃, or -O-CH(CH₃)₂.
Embodiment 31 provides a compound or pharmaceutically acceptable salt thereof, according to any one of embodiments 1-24, wherein R⁷ is -CH₃ or -CH₂CH₃, each of which is substituted with one, two, or three independently selected F, -O-CH₃, or -O-CH₂CH₃.
Embodiment 32 provides a compound or pharmaceutically acceptable salt thereof, according to any one of embodiments 1-24, wherein R⁷ is -CHF₂, -CH₂CF₃, -CH₂CH₂-O-CH₃, or -CH₂CH₂-O-CH₂CH₃.
Embodiment 33 provides a compound or pharmaceutically acceptable salt thereof, according to any one of embodiments 1-24, wherein R⁷ is 4-6 membered monocyclic heterocycloalkyl comprising one, two or three heteroatoms independently selected from N, O, and S.
Embodiment 34 provides a compound or pharmaceutically acceptable salt thereof, according to any one of embodiments 1-24, wherein R⁷ is azetidinyl, oxetanyl, pyrrolidinyl, tetrahydrofuranyl, piperidinyl, tetrahydropyranyl, tetrahydrothiopyranyl, morpholinyl, thiomorpholinyl, dioxanyl, or piperazinyl.
Embodiment 35 provides a compound or pharmaceutically acceptable salt thereof, according to any one of embodiments 1-24, wherein R⁷ is oxetanyl.
Embodiment 36 provides a compound or pharmaceutically acceptable salt thereof, according to any one of embodiments 1-23, wherein R² is C₁₋₆ alkyl.
Embodiment 37 provides a compound or pharmaceutically acceptable salt thereof, according to any one of embodiments 1-23, wherein R² is -CH₃, -CH₂CH₃, -CH₂CH₂CH₃, -CH(CH₃)₂, -CH₂CH(CH₃)₂, -C(CH₃)₃, -CH(CH₃)CH₂CH₃, or -CH(CH₃)CH(CH₃)₂.
Embodiment 38 provides a compound or pharmaceutically acceptable salt thereof, according to any one of embodiments 1-23, wherein R² is -CH₃, -CH₂CH₃, or -CH₂CH(CH₃)₂.
Embodiment 39 provides a compound or pharmaceutically acceptable salt thereof, according to any one of embodiments 1-23, wherein R² is C₁₋₆ alkyl substituted with one or more independently selected halo.
Embodiment 40 provides a compound or pharmaceutically acceptable salt thereof, according to any one of embodiments 1-23, wherein R² is -CH₃, -CH₂CH₃, -CH₂CH₂CH₃, -CH(CH₃)₂, -CH₂CH(CH₃)₂, -C(CH₃)₃, -CH(CH₃)CH₂CH₃, or -CH(CH₃)CH(CH₃)₂, each of which is substituted with one or more independently selected halo.
Embodiment 41 provides a compound or pharmaceutically acceptable salt thereof, according to any one of embodiments 1-23, wherein R² is C₁₋₆ alkyl substituted with one, two, or three independently selected halo. Embodiment 42 provides a compound or pharmaceutically acceptable salt thereof, according to any one of embodiments 1-23, wherein R² is C₁₋₆ alkyl substituted with one or more independently selected F, Cl, or Br.
Embodiment 43 provides a compound or pharmaceutically acceptable salt thereof, according to any one of embodiments 1-23, wherein R² is -CH₃ substituted with one or more independently selected halo.
Embodiment 44 provides a compound or pharmaceutically acceptable salt thereof, according to any one of embodiments 1-23, wherein R² is -CH₃, -CH₂CH₃, -CH₂CH₂CH₃, -CH(CH₃)₂, -CH₂CH(CH₃)₂, -C(CH₃)₃, -CH(CH₃)CH₂CH₃, or -CH(CH₃)CH(CH₃)₂, each of which is substituted with one, two, or three independently selected halo.
Embodiment 45 provides a compound or pharmaceutically acceptable salt thereof, according to any one of embodiments 1-23, wherein R² is -CH₃, -CH₂CH₃, -CH₂CH₂CH₃, -CH(CH₃)₂, -CH₂CH(CH₃)₂, -C(CH₃)₃, -CH(CH₃)CH₂CH₃, or -CH(CH₃)CH(CH₃)₂, each of which is substituted with one or more independently selected F, Cl, or Br.
Embodiment 46 provides a compound or pharmaceutically acceptable salt thereof, according to any one of embodiments 1-23, wherein R² is -CH₃ substituted with one, two, or three independently selected halo.
Embodiment 47 provides a compound or pharmaceutically acceptable salt thereof, according to any one of embodiments 1-23, wherein R² is -CH₃, -CH₂CH₃, -CH₂CH₂CH₃, -CH(CH₃)₂, -CH₂CH(CH₃)₂, -C(CH₃)₃, -CH(CH₃)CH₂CH₃, or -CH(CH₃)CH(CH₃)₂, each of which is substituted with one, two, or three independently selected F, Cl, or Br.
Embodiment 48 provides a compound or pharmaceutically acceptable salt thereof, according to any one of embodiments 1-23, wherein R² is -CH₃, -CH₂CH₃, -CH₂CH₂CH₃, -CH(CH₃)₂, -CH₂CH(CH₃)₂, -C(CH₃)₃, -CH(CH₃)CH₂CH₃, or -CH(CH₃)CH(CH₃)₂, each of which is substituted with one or more F.
Embodiment 49 provides a compound or pharmaceutically acceptable salt thereof, according to any one of embodiments 1-23, wherein R² is -CHF₂ or -CF₃.
Embodiment 50 provides a compound or pharmaceutically acceptable salt thereof, according to any one of embodiments 1-23, wherein R² is C₃₋₆ cycloalkyl.
Embodiment 51 provides a compound or pharmaceutically acceptable salt thereof, according to any one of embodiments 1-23, wherein R² is cyclopropyl, cyclobutyl, or cyclopentyl.
Embodiment 52 provides a compound or pharmaceutically acceptable salt thereof, according to any one of embodiments 1-23, wherein R² is cyclopropyl.
Embodiment 53 provides a compound or pharmaceutically acceptable salt thereof, according to any one of embodiments 1-23, wherein R² is -C(=O)-NR^{8a}R^{8b}.
Embodiment 54 provides a compound or pharmaceutically acceptable salt thereof, according to any one of embodiments 1-23 and 53, wherein R^{8a} and R^{8b} are both H.
Embodiment 55 provides a compound or pharmaceutically acceptable salt thereof, according to any one of embodiments 1-23 and 53, wherein one of R^{8a} and R^{8b} is H, and the other is C₁₋₄ alkyl, or phenyl.
Embodiment 56 provides a compound or pharmaceutically acceptable salt thereof, according to any one of embodiments 1-23 and 53, wherein R^{8a} and R^{8b} are both C₁₋₄ alkyl.
Embodiment 57 provides a compound or pharmaceutically acceptable salt thereof, according to any one of embodiments 1-23 and 53, wherein R^{8a} and R^{8b} is H, and the other is -CH₃, -CH₂CH₃, or -CH(CH₃)₂, or phenyl.
Embodiment 58 provides a compound or pharmaceutically acceptable salt thereof, according to any one of embodiments 1-23 and 53, wherein R^{8a} and R^{8b} are independently -CH₃, -CH₂CH₃, or -CH(CH₃)₂.
Embodiment 59 provides a compound or pharmaceutically acceptable salt thereof, according to any one of embodiments 1-23 and 53, wherein one of R^{8a} and R^{8b} is H, and the other is phenyl.
Embodiment 60 provides a compound or pharmaceutically acceptable salt thereof, according to any one of embodiments 1-23, wherein R² is 4-6 membered monocyclic heterocycloalkyl comprising one, two or three heteroatoms independently selected from N, O, and S.
Embodiment 61 provides a compound or pharmaceutically acceptable salt thereof, according to any one of embodiments 1-23, wherein R² is azetidinyl, oxetanyl, pyrrolidinyl, tetrahydrofuranyl, piperidinyl, tetrahydropyranyl, tetrahydrothiopyranyl, morpholinyl, thiomorpholinyl, dioxanyl, or piperazinyl.
Embodiment 62 provides a compound or pharmaceutically acceptable salt thereof, according to any one of embodiments 1-23, wherein R² is tetrahydropyranyl.
Embodiment 63 provides a compound or pharmaceutically acceptable salt thereof, according to any one of embodiments 1-23, wherein R² is 4-6 membered monocyclic heterocycloalkenyl comprising one double bond and further comprising one, or two heteroatoms independently selected from N, O, and S.
Embodiment 64 provides a compound or pharmaceutically acceptable salt thereof, according to any one of embodiments 1-23, wherein R² is pyrrolinyl, pyrazolinyl, imidazolinyl, tetrahydropyridinyl, or dihydropyranyl.
Embodiment 65 provides a compound or pharmaceutically acceptable salt thereof, according to any one of embodiments 1-23, wherein R² is 3,6-dihydro-2H-pyranyl.
Embodiment 66 provides a compound or pharmaceutically acceptable salt thereof, according to embodiment 1, wherein the compound is according to Formula IVa, IVb, IVc, or IVd: 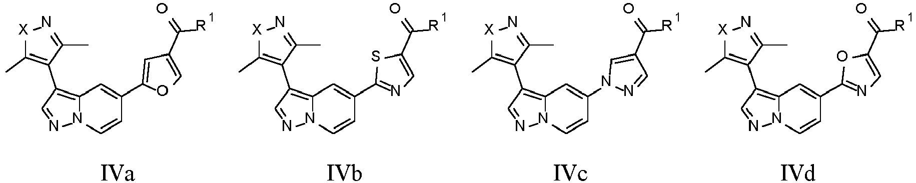
Embodiment 67 provides a compound or pharmaceutically acceptable salt thereof, according to any one of embodiments 1-66, wherein X is O.
Embodiment 68 provides a compound or pharmaceutically acceptable salt thereof, according to any one of embodiments 1-66, wherein X is NR4.
Embodiment 69 provides a compound or pharmaceutically acceptable salt thereof, according to embodiment 1, wherein the compound is according to Formula Va, Vb, or Vc: 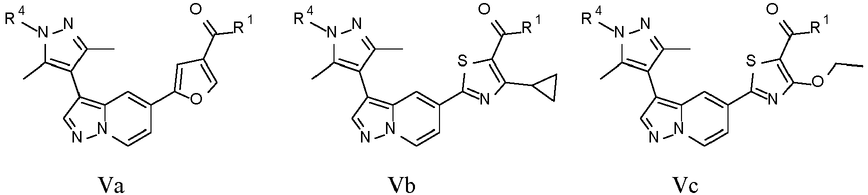
Embodiment 70 provides a compound or pharmaceutically acceptable salt thereof, according to any one of embodiments 1-66, 68, and 69, wherein R⁴ is C₁₋₃ alkyl.
Embodiment 71 provides a compound or pharmaceutically acceptable salt thereof, according to any one of embodiments 1-66, 68, and 69, wherein R⁴ is -CH₃, -CH₂CH₃, or -CH(CH₃)₂.
Embodiment 72 provides a compound or pharmaceutically acceptable salt thereof, according to any one of embodiments 1-66, 68, and 69, wherein R⁴ is -CH(CH₃)₂.
Embodiment 73 provides a compound or pharmaceutically acceptable salt thereof, according to any one of embodiments 1-66, 68, and 69, wherein R⁴ is C₁₋₃ alkyl substituted with one or more F.
Embodiment 74 provides a compound or pharmaceutically acceptable salt thereof, according to any one of embodiments 1-66, 68, and 69, wherein R⁴ is -CH₃, -CH₂CH₃, or -CH(CH₃)₂, each of which is substituted with one or more F.
Embodiment 75 provides a compound or pharmaceutically acceptable salt thereof, according to any one of embodiments 1-66, 68, and 69, wherein R⁴ is C₁₋₃ alkyl substituted with one, two, or three F.
Embodiment 76 provides a compound or pharmaceutically acceptable salt thereof, according to any one of embodiments 1-66, 68, and 69, wherein R⁴ is -CH₃, -CH₂CH₃, or -CH(CH₃)₂, each of which is substituted with one, two or three F.
Embodiment 77 provides a compound or pharmaceutically acceptable salt thereof, according to any one of embodiments 1-66, 68, and 69, wherein R⁴ is -CHF₂, -CF₃, -CH₂-CF₃, or -CH(CH₃)-CF₃.
Embodiment 78 provides a compound or pharmaceutically acceptable salt thereof, according to any one of embodiments 1-66, 68, and 69, wherein R⁴ is -CHF₂ or -CH₂-CF₃.
Embodiment 79 provides a compound or pharmaceutically acceptable salt thereof, according to any one of embodiments 1-78, wherein R¹ is -OR⁵.
Embodiment 80 provides a compound or pharmaceutically acceptable salt thereof, according to any one of embodiments 1-79, wherein R⁵ is H.
Embodiment 81 provides a compound or pharmaceutically acceptable salt thereof, according to any one of embodiments 1-79, wherein R⁵ is C₁₋₄ alkyl.
Embodiment 82 provides a compound or pharmaceutically acceptable salt thereof, according to any one of embodiments 1-79, wherein R⁵ is -CH₃, -CH₂CH₃, or -CH(CH₃)₂.
Embodiment 83 provides a compound or pharmaceutically acceptable salt thereof, according to any one of embodiments 1-79, wherein R⁵ is -CH₃ or-CH₂CH₃.
Embodiment 84 provides a compound or pharmaceutically acceptable salt thereof, according to any one of embodiments 1-79, wherein R⁵ is C₁₋₄ alkyl substituted with one or more independently selected -C(=O)-NR^{9a}R^{9b} or -O-C(=O)-C₁₋₆ alkyl.
Embodiment 85 provides a compound or pharmaceutically acceptable salt thereof, according to any one of embodiments 1-79, wherein R⁵ is -CH₃, -CH₂CH₃, or -CH(CH₃)₂, each of which is substituted with one or more independently selected -C(=O)-NR^{9a}R^{9b} or -O-C(=O)-C₁₋₆ alkyl.
Embodiment 86 provides a compound or pharmaceutically acceptable salt thereof, according to any one of embodiments 1-79, wherein R⁵ is C₁₋₄ alkyl substituted with one, two, or three independently selected -C(=O)-NR^{9a}R^{9b} or -O-C(=O)-C₁₋₆ alkyl.
Embodiment 87 provides a compound or pharmaceutically acceptable salt thereof, according to any one of embodiments 1-79, wherein R⁵ is C₁₋₄ alkyl substituted with one or more independently selected -C(=O)-NR^{9a}R^{9b}, -O-C(=O)-CH₃, -O-C(=O)-CH₂CH₃, -O-C(=O)-CH₂CH₂CH₃, -O-C(=O)-CH(CH₃)₂, -O-C(=O)-CH₂CH(CH₃)₂, -O-C(=O)-C(CH₃)₃, -O-C(=O)-CH(CH₃)CH₂CH₃, -O-C(=O)-CH(CH₃)CH(CH₃)₂, -O-C(=O)-CH₂CH(CH₃)CH₂CH₃, or -O-C(=O)-CH₂CH₂CH(CH₃)₂.
Embodiment 88 provides a compound or pharmaceutically acceptable salt thereof, according to any one of embodiments 1-79, wherein R⁵ is -CH₃ substituted with one or more independently selected -C(=O)-NR^{9a}R^{9b} or -O-C(=O)-C₁₋₆ alkyl.
Embodiment 89 provides a compound or pharmaceutically acceptable salt thereof, according to any one of embodiments 1-79, wherein R⁵ is -CH₃, -CH₂CH₃, or -CH(CH₃)₂, each of which is substituted with one, two, or three independently selected -C(=O)-NR^{9a}R^{9b} or -O-C(=O)-C₁₋₆ alkyl.
Embodiment 90 provides a compound or pharmaceutically acceptable salt thereof, according to any one of embodiments 1-79, wherein R⁵ is -CH₃, -CH₂CH₃, or -CH(CH₃)₂, each of which is substituted with one or more independently selected -C(=O)-NR^{9a}R^{9b}, -O-C(=O)-CH₃, -O-C(=O)-CH₂CH₃, -O-C(=O)-CH₂CH₂CH₃, -O-C(=O)-CH(CH₃)₂, -O-C(=O)-CH₂CH(CH₃)₂, -O-C(=O)-C(CH₃)₃, -O-C(=O)-CH(CH₃)CH₂CH₃, -O-C(=O)-CH(CH₃)CH(CH₃)₂, -O-C(=O)-CH₂CH(CH₃)CH₂CH₃, or -O-C(=O)-CH₂CH₂CH(CH₃)₂.
Embodiment 91 provides a compound or pharmaceutically acceptable salt thereof, according to any one of embodiments 1-79, wherein R⁵ is C₁₋₄ alkyl substituted with one, two, or three independently selected -C(=O)-NR^{9a}R^{9b}, -O-C(=O)-CH₃, -O-C(=O)-CH₂CH₃, -O-C(=O)-CH₂CH₂CH₃, -O-C(=O)-CH(CH₃)₂, -O-C(=O)-CH₂CH(CH₃)₂, -O-C(=O)-C(CH₃)₃, -O-C(=O)-CH(CH₃)CH₂CH₃, -O-C(=O)-CH(CH₃)CH(CH₃)₂, -O-C(=O)-CH₂CH(CH₃)CH₂CH₃, or -O-C(=O)-CH₂CH₂CH(CH₃)₂.
Embodiment 92 provides a compound or pharmaceutically acceptable salt thereof, according to any one of embodiments 1-79, wherein R⁵ is C₁₋₄ alkyl substituted with one or more independently selected -C(=O)-NR^{9a}R^{9b} or -O-C(=O)-C(CH₃)₃.
Embodiment 93 provides a compound or pharmaceutically acceptable salt thereof, according to any one of embodiments 1-79, wherein R⁵ is -CH₃ substituted with one, two, or three independently selected -C(=O)-NR^{9a}R^{9b} or -O-C(=O)-C₁₋₆ alkyl.
Embodiment 94 provides a compound or pharmaceutically acceptable salt thereof, according to any one of embodiments 1-79, wherein R⁵ is -CH₃, -CH₂CH₃, or -CH(CH₃)₂, each of which is substituted with one, two, or three independently selected -C(=O)-NR^{9a}R^{9b}, -O-C(=O)-CH₃, -O-C(=O)-CH₂CH₃, -O-C(=O)-CH₂CH₂CH₃, -O-C(=O)-CH(CH₃)₂, -O-C(=O)-CH₂CH(CH₃)₂, -O-C(=O)-C(CH₃)₃, -O-C(=O)-CH(CH₃)CH₂CH₃, -O-C(=O)-CH(CH₃)CH(CH₃)₂, -O-C(=O)-CH₂CH(CH₃)CH₂CH₃, or -O-C(=O)-CH₂CH₂CH(CH₃)₂.
Embodiment 95 provides a compound or pharmaceutically acceptable salt thereof, according to any one of embodiments 1-79, wherein R⁵ is C₁₋₄ alkyl substituted with one, two, or three independently selected -C(=O)-NR^{9a}R^{9b} or -O-C(=O)-C(CH₃)₃.
Embodiment 96 provides a compound or pharmaceutically acceptable salt thereof, according to any one of embodiments 1-79, wherein R⁵ is -CH₃ substituted with one or more independently selected -C(=O)-NR^{9a}R^{9b}, -O-C(=O)-CH₃, -O-C(=O)-CH₂CH₃, -O-C(=O)-CH₂CH₂CH₃, -O-C(=O)-CH(CH₃)₂, -O-C(=O)-CH₂CH(CH₃)₂, -O-C(=O)-C(CH₃)₃, -O-C(=O)-CH(CH₃)CH₂CH₃, -O-C(=O)-CH(CH₃)CH(CH₃)₂, -O-C(=O)-CH₂CH(CH₃)CH₂CH₃, or -O-C(=O)-CH₂CH₂CH(CH₃)₂.
Embodiment 97 provides a compound or pharmaceutically acceptable salt thereof, according to any one of embodiments 1-79, wherein R⁵ is -CH₃, -CH₂CH₃, or -CH(CH₃)₂, each of which is substituted with one or more independently selected -C(=O)-NR^{9a}R^{9b} or -O-C(=O)-C(CH₃)₃.
Embodiment 98 provides a compound or pharmaceutically acceptable salt thereof, according to any one of embodiments 1-79, wherein R⁵ is -CH₃ substituted with one -C(=O)-NR^{9a}R^{9b} or -O-C(=O)-C(CH₃)₃. Embodiment 99 provides a compound or pharmaceutically acceptable salt thereof, according to any one of embodiments 1-79 and 84-98, wherein R^{9a} and R^{9b} are both H.
Embodiment 100 provides a compound or pharmaceutically acceptable salt thereof, according to any one of embodiments 1-79 and 84-98, wherein one of R^{9a} and R^{9b} is H, and the other is C₁₋₄ alkyl.
Embodiment 101 provides a compound or pharmaceutically acceptable salt thereof, according to any one of embodiments 1-79 and 84-98, wherein R^{9a} and R^{9b} are both C₁₋₄ alkyl.
Embodiment 102 provides a compound or pharmaceutically acceptable salt thereof, according to any one of embodiments 1-79 and 84-98, wherein one of R^{9a} and R^{9b} is H, and the other is -CH₃, -CH₂CH₃, or - CH(CH₃)₂.
Embodiment 103 provides a compound or pharmaceutically acceptable salt thereof, according to any one of embodiments 1-79 and 84-98, wherein R^{9a} and R^{9b} are independently -CH₃, -CH₂CH₃, or -CH(CH₃)₂.
Embodiment 104 provides a compound or pharmaceutically acceptable salt thereof, according to any one of embodiments 1-79 and 84-98, wherein R^{9a} and R^{9b} are both -CH₃.
Embodiment 105 provides a compound or pharmaceutically acceptable salt thereof, according to any one of embodiments 1-78, wherein R¹ is -NR^{6a}R^{6b}.
Embodiment 106 provides a compound or pharmaceutically acceptable salt thereof, according to any one of embodiments 1-78 and 105, wherein R^{6a} and R^{6b} are both H.
Embodiment 107 provides a compound or pharmaceutically acceptable salt thereof, according to any one of embodiments 1-78 and 105, wherein one of R^{6a} and R^{6b} is H, and the other is -S(=O)₂-C₁₋₄ alkyl, or - S(=O)₂-C₃₋₆ cycloalkyl.
Embodiment 108 provides a compound or pharmaceutically acceptable salt thereof, according to any one of embodiments 1-78 and 105, wherein one of R^{6a} and R^{6b} is H, and the other is -S(=O)₂-CH₃, -S(=O)₂-CH₂CH₃, -S(=O)₂-CH(CH₃)₂, -S(=O)z-cyclopropyl, -S(=O)z-cyclobutyl, or -S(=O)₂-cyclopentyl.
Embodiment 109 provides a compound or pharmaceutically acceptable salt thereof, according to any one of embodiments 1-78 and 105, wherein one of R^{6a} and R^{6b} is H, and the other is -S(=O)₂-CH₃ or -S(=O)z-cyclopropyl.
Embodiment 110 provides a compound or pharmaceutically acceptable salt thereof, according to embodiment 1, wherein the compound is selected from Table III.
Embodiment 111 provides a pharmaceutical composition comprising a pharmaceutically acceptable carrier and a pharmaceutically effective amount of a compound or pharmaceutically acceptable salt thereof according to any one of embodiments 1-110.
Embodiment 112 provides a pharmaceutical composition according to embodiment 111 comprising a further therapeutic agent.
Embodiment 113 provides a compound or pharmaceutically acceptable salt thereof, according to any one of embodiments 1-110, or a pharmaceutical composition according to embodiment 111 or 112 for use in medicine.
Embodiment 114 provides a compound or pharmaceutically acceptable salt thereof, according to any one of embodiments 1-110, or a pharmaceutical composition according to embodiment 111 or 112 for use in the prophylaxis and/or treatment of endocrine, nutritional, metabolic, and/or cardiovascular diseases.
Embodiment 115 provides a compound or pharmaceutically acceptable salt thereof, according to any one of embodiments 1-110, or a pharmaceutical composition according to embodiment 111 or 112, wherein said compound or pharmaceutical composition is administered in combination with a further therapeutic agent.
Embodiment 116 provides the pharmaceutical composition according to embodiment 112, or the compound according to embodiment 115, wherein the further therapeutic agent is an agent for the prophylaxis and/or treatment of endocrine, nutritional, metabolic, and/or cardiovascular diseases.

### PHARMACEUTICAL COMPOSITIONS

When employed as a pharmaceutical, a compound of the invention is typically administered in the form of a pharmaceutical composition. Such compositions can be prepared in a manner well known in the pharmaceutical art and comprise at least one active compound of the invention according to Formula I. Generally, a compound of the invention is administered in a pharmaceutically effective amount. The amount of compound of the invention actually administered will typically be determined by a physician, in the light of the relevant circumstances, including the condition to be treated, the chosen route of administration, the actual compound of the invention administered, the age, weight, and response of the individual patient, the severity of the patient's symptoms, and the like.

The pharmaceutical compositions of this invention can be administered by a variety of routes including oral, rectal, transdermal, subcutaneous, intra-articular, intravenous, intramuscular, and intranasal. Depending on the intended route of delivery, a compound of the invention is preferably formulated as either injectable or oral compositions or as salves, as lotions or as patches all for transdermal administration.

The compositions for oral administration can take the form of bulk liquid solutions or suspensions, or bulk powders. More commonly, however, the compositions are presented in unit dosage forms to facilitate accurate dosing. The term 'unit dosage forms' refers to physically discrete units suitable as unitary dosages for human subjects and other mammals, each unit containing a predetermined quantity of active material calculated to produce the desired therapeutic effect, in association with a suitable pharmaceutical excipient, vehicle or carrier. Typical unit dosage forms include prefilled, premeasured ampules or syringes of the liquid compositions or pills, tablets, capsules or the like in the case of solid compositions. In such compositions, the compound of the invention according to Formula I is usually a minor component (from about 0.1 to about 50% by weight or preferably from about 1 to about 40% by weight) with the remainder being various vehicles or carriers and processing aids helpful for forming the desired dosing form.

Liquid forms suitable for oral administration may include a suitable aqueous or non-aqueous vehicle with buffers, suspending and dispensing agents, colorants, flavors and the like. Solid forms may include, for example, any of the following ingredients, or compound of the inventions of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint or orange flavoring.

Injectable compositions are typically based upon injectable sterile saline or phosphate-buffered saline or other injectable carriers known in the art. As before, the active compound of the invention according to Formula I in such compositions is typically a minor component, often being from about 0.05 to 10% by weight with the remainder being the injectable carrier and the like.

Transdermal compositions are typically formulated as a topical ointment or cream containing the active ingredient(s), generally in an amount ranging from about 0.01 to about 20% by weight, preferably from about 0.1 to about 20% by weight, preferably from about 0.1 to about 10% by weight, and more preferably from about 0.5 to about 15% by weight. When formulated as an ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration or stability of the active ingredients or the formulation. All such known transdermal formulations and ingredients are included within the scope of this invention.

A compound of the invention can also be administered by a transdermal device. Accordingly, transdermal administration can be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania.

A compound of the invention can also be administered in sustained release forms or from sustained release drug delivery systems. A description of representative sustained release materials can be found in Remington's Pharmaceutical Sciences.

The following formulation examples illustrate representative pharmaceutical compositions that may be prepared in accordance with this invention. The present invention, however, is not limited to the following pharmaceutical compositions.

### Formulation 1 - Tablets

A compound of the invention according to Formula I may be admixed as a dry powder with a dry gelatin binder in an approximate 1:2 weight ratio. A minor amount of magnesium stearate may be added as a lubricant. The mixture may be formed into 240-270 mg tablets (80-90 mg of active compound of the invention according to Formula I per tablet) in a tablet press.

### Formulation 2 - Capsules

A compound of the invention according to Formula I may be admixed as a dry powder with a starch diluent in an approximate 1: 1 weight ratio. The mixture may be filled into 250 mg capsules (125 mg of active compound of the invention according to Formula I per capsule).

### Formulation 3 - Liquid

A compound of the invention according to Formula I (125 mg), may be admixed with sucrose (1.75 g) and xanthan gum (4 mg) and the resultant mixture may be blended, passed through a No. 10 mesh U.S. sieve, and then mixed with a previously made solution of microcrystalline cellulose and sodium carboxymethyl cellulose (11:89, 50 mg) in water. Sodium benzoate (10 mg), flavor, and color may be diluted with water and added with stirring. Sufficient water may then be added with stirring. Further sufficient water may be then added to produce a total volume of 5 mL.

### Formulation 4 - Tablets

A compound of the invention according to Formula I may be admixed as a dry powder with a dry gelatin binder in an approximate 1:2 weight ratio. A minor amount of magnesium stearate may be added as a lubricant. The mixture may be formed into 450-900 mg tablets (150-300 mg of active compound of the invention according to Formula I) in a tablet press.

### Formulation 5 - Injection

A compound of the invention according to Formula I may be dissolved or suspended in a buffered sterile saline injectable aqueous medium to a concentration of approximately 5 mg/mL.

### Formulation 6 - Topical

Stearyl alcohol (250 g) and a white petrolatum (250 g) may be melted at about 75°C and then a mixture of A compound of the invention according to Formula I (50 g) methylparaben (0.25 g), propylparaben (0.15 g), sodium lauryl sulfate (10 g), and propylene glycol (120 g) dissolved in water (about 370 g) may be added and the resulting mixture may be stirred until it congeals.

### METHODS OF TREATMENT

In one embodiment, the present invention provides compounds of the invention, or pharmaceutical compositions comprising a compound of the invention, for use in medicine.

In one embodiment, the present invention provides compounds of the invention or pharmaceutical compositions comprising a compound of the invention, for use in the prophylaxis and/or treatment of endocrine diseases. In particular, the term endocrine diseases refers to adrenal diseases, obesity, metabolic syndrome, impaired glucose tolerance, prediabetes, Cushing's syndrome, chronic pancreatitis, insulin resistance, hyperglycemia, hyperinsulinemia, gestational diabetes, diabetes mellitus, insulin-dependent (type 1) diabetes mellitus, non-insulin-dependent (type 2) diabetes mellitus, and acromegaly. More particularly, the term refers to type 2 diabetes mellitus, and insulin resistance.

In another embodiment, the present invention provides the use of compounds of the invention or pharmaceutical compositions comprising compounds of the invention in the manufacture of a medicament for the prophylaxis and/or treatment of endocrine diseases. In particular, the term endocrine diseases refers to adrenal diseases, obesity, metabolic syndrome, impaired glucose tolerance, prediabetes, Cushing's syndrome, chronic pancreatitis, insulin resistance, hyperglycemia, hyperinsulinemia, gestational diabetes, diabetes mellitus, insulin-dependent (type 1) diabetes mellitus, non-insulin-dependent (type 2) diabetes mellitus, and acromegaly. More particularly, the term refers to type 2 diabetes mellitus, and insulin resistance.

In additional method of treatment aspects, this invention provides methods of prophylaxis and/or treatment of a mammal afflicted with an endocrine disease, which methods comprise the administration of an effective amount of a compound of the invention or one or more of the pharmaceutical compositions herein described for the treatment or prophylaxis of said condition. In particular, the term endocrine diseases refers to adrenal diseases, obesity, metabolic syndrome, impaired glucose tolerance, prediabetes, Cushing's syndrome, chronic pancreatitis, insulin resistance, hyperglycemia, hyperinsulinemia, gestational diabetes, diabetes mellitus, insulin-dependent (type 1) diabetes mellitus, non-insulin-dependent (type 2) diabetes mellitus, and acromegaly. More particularly, the term refers to type 2 diabetes mellitus, and insulin resistance.

In one embodiment, the present invention provides pharmaceutical compositions comprising a compound of the invention, and another therapeutic agent. In a particular embodiment, the other therapeutic agent is an endocrine diseases treatment agent. In particular, the term endocrine diseases refers to adrenal diseases, obesity, metabolic syndrome, impaired glucose tolerance, prediabetes, Cushing's syndrome, chronic pancreatitis, insulin resistance, hyperglycemia, hyperinsulinemia, gestational diabetes, diabetes mellitus, insulin-dependent (type 1) diabetes mellitus, non-insulin-dependent (type 2) diabetes mellitus, and acromegaly. More particularly, the term refers to type 2 diabetes mellitus, and insulin resistance.

In one embodiment, the present invention provides compounds of the invention or pharmaceutical compositions comprising a compound of the invention, for use in the prophylaxis and/or treatment of nutritional diseases. In particular, the term nutritional diseases refers to malnutrition, hyperalimentation, hyperglycemia, dyslipidemia, hyperlipidemia, hypertriglyceridemia, hypercholesterolemia, obesity, drug-induced obesity, morbid obesity, localized adiposity, and malnutrition-related diabetes mellitus. More particularly, the term refers to obesity, hyperlipidemia, and hyperglycemia.

In another embodiment, the present invention provides the use of compounds of the invention or pharmaceutical compositions comprising compounds of the invention in the manufacture of a medicament for the prophylaxis and/or treatment of nutritional diseases. In particular, the term nutritional diseases refers to malnutrition, hyperalimentation, hyperglycemia, dyslipidemia, hyperlipidemia, hypertriglyceridemia, hypercholesterolemia, obesity, drug-induced obesity, morbid obesity, localized adiposity, and malnutrition-related diabetes mellitus. More particularly, the term refers to obesity, hyperlipidemia, and hyperglycemia.

In additional method of treatment aspects, this invention provides methods of prophylaxis and/or treatment of a mammal afflicted with a nutritional disease, which methods comprise the administration of an effective amount of a compound of the invention or one or more of the pharmaceutical compositions herein described for the treatment or prophylaxis of said condition. In particular, the term nutritional diseases refers to malnutrition, hyperalimentation, hyperglycemia, dyslipidemia, hyperlipidemia, hypertriglyceridemia, hypercholesterolemia, obesity, drug-induced obesity, morbid obesity, localized adiposity, and malnutrition-related diabetes mellitus. More particularly, the term refers to obesity, hyperlipidemia, and hyperglycemia.

In one embodiment, the present invention provides pharmaceutical compositions comprising a compound of the invention, and another therapeutic agent. In a particular embodiment, the other therapeutic agent is a nutritional diseases treatment agent. In particular, the term nutritional diseases refers to malnutrition, hyperalimentation, hyperglycemia, dyslipidemia, hyperlipidemia, hypertriglyceridemia, hypercholesterolemia, obesity, drug-induced obesity, morbid obesity, localized adiposity, and malnutrition-related diabetes mellitus. More particularly, the term refers to obesity, hyperlipidemia, and hyperglycemia.

In one embodiment, the present invention provides compounds of the invention or pharmaceutical compositions comprising a compound of the invention, for use in the prophylaxis and/or treatment of metabolic diseases. In particular, the term metabolic diseases refers to obesity, diabetes mellitus, especially type 2 diabetes, hyperinsulinemia, glucose intolerance, metabolic syndrome X, dyslipidemia, hyperlipidemia, hypertriglyceridemia, hypercholesterolemia, hyperlipoproteinemia, combined hyperlipidemia, and hepatic steatosis (fatty liver disease), including non-alcoholic fatty liver disease (NAFLD) and non-alcoholic steatohepatitis (NASH). More particularly, the term refers to type 2 diabetes, hyperlipidemia, and NASH.

In another embodiment, the present invention provides the use of compounds of the invention or pharmaceutical compositions comprising compounds of the invention in the manufacture of a medicament for the prophylaxis and/or treatment of metabolic diseases. In particular, the term metabolic diseases refers to obesity, diabetes mellitus, especially type 2 diabetes, hyperinsulinemia, glucose intolerance, metabolic syndrome X, dyslipidemia, hyperlipidemia, hypertriglyceridemia, hypercholesterolemia, hyperlipoproteinemia, combined hyperlipidemia, and hepatic steatosis (fatty liver disease), including non-alcoholic fatty liver disease (NAFLD) and non-alcoholic steatohepatitis (NASH). More particularly, the term refers to type 2 diabetes, hyperlipidemia, and NASH.

In additional method of treatment aspects, this invention provides methods of prophylaxis and/or treatment of a mammal afflicted with a metabolic disease, which methods comprise the administration of an effective amount of a compound of the invention or one or more of the pharmaceutical compositions herein described for the treatment or prophylaxis of said condition. In particular, the term metabolic diseases refers to obesity, diabetes mellitus, especially type 2 diabetes, hyperinsulinemia, glucose intolerance, metabolic syndrome X, dyslipidemia, hyperlipidemia, hypertriglyceridemia, hypercholesterolemia, hyperlipoproteinemia, combined hyperlipidemia, and hepatic steatosis (fatty liver disease), including non-alcoholic fatty liver disease (NAFLD) and non-alcoholic steatohepatitis (NASH). More particularly, the term refers to type 2 diabetes, hyperlipidemia, and NASH.

In one embodiment, the present invention provides pharmaceutical compositions comprising a compound of the invention, and another therapeutic agent. In a particular embodiment, the other therapeutic agent is a metabolic diseases treatment agent. In particular, the term metabolic diseases refers to obesity, diabetes mellitus, especially type 2 diabetes, hyperinsulinemia, glucose intolerance, metabolic syndrome X, dyslipidemia, hyperlipidemia, hypertriglyceridemia, hypercholesterolemia, hyperlipoproteinemia, combined hyperlipidemia, and hepatic steatosis (fatty liver disease), including non-alcoholic fatty liver disease (NAFLD) and non-alcoholic steatohepatitis (NASH). More particularly, the term refers to type 2 diabetes, hyperlipidemia, and NASH.

In one embodiment, the present invention provides compounds of the invention or pharmaceutical compositions comprising a compound of the invention, for use in the prophylaxis and/or treatment of cardiovascular diseases. In particular, the term cardiovascular diseases refers to arrhythmia (atrial or ventricular or both); atherosclerosis and its sequelae; angina; cardiac rhythm disturbances; myocardial ischemia; myocardial infarction; cardiac or vascular aneurysm; vasculitis, stroke; peripheral obstructive arteriopathy of a limb, an organ, or a tissue; reperfusion injury following ischemia of the brain, heart, kidney or other organ or tissue; endotoxic, surgical, or traumatic shock; hypertension, valvular heart disease, heart failure, abnormal blood pressure; shock; vasoconstriction (including that associated with migraines); vascular abnormality, inflammation, insufficiency limited to a single organ or tissue. More particularly, the term refers to vascular disease, atherosclerosis, coronary heart disease, cerebrovascular disease, heart failure and peripheral vessel disease, and hypertension.

In another embodiment, the present invention provides the use of compounds of the invention or pharmaceutical compositions comprising compounds of the invention in the manufacture of a medicament for the prophylaxis and/or treatment of cardiovascular diseases. In particular, the term cardiovascular diseases refers to arrhythmia (atrial or ventricular or both); atherosclerosis and its sequelae; angina; cardiac rhythm disturbances; myocardial ischemia; myocardial infarction; cardiac or vascular aneurysm; vasculitis, stroke; peripheral obstructive arteriopathy of a limb, an organ, or a tissue; reperfusion injury following ischemia of the brain, heart, kidney or other organ or tissue; endotoxic, surgical, or traumatic shock; hypertension, valvular heart disease, heart failure, abnormal blood pressure; shock; vasoconstriction (including that associated with migraines); vascular abnormality, inflammation, insufficiency limited to a single organ or tissue. More particularly, the term refers to vascular disease, atherosclerosis, coronary heart disease, cerebrovascular disease, heart failure and peripheral vessel disease, and hypertension.

In additional method of treatment aspects, this invention provides methods of prophylaxis and/or treatment of a mammal afflicted with cardiovascular diseases, which methods comprise the administration of an effective amount of a compound of the invention or one or more of the pharmaceutical compositions herein described for the treatment or prophylaxis of said condition. In particular, the term cardiovascular diseases refers to arrhythmia (atrial or ventricular or both); atherosclerosis and its sequelae; angina; cardiac rhythm disturbances; myocardial ischemia; myocardial infarction; cardiac or vascular aneurysm; vasculitis, stroke; peripheral obstructive arteriopathy of a limb, an organ, or a tissue; reperfusion injury following ischemia of the brain, heart, kidney or other organ or tissue; endotoxic, surgical, or traumatic shock; hypertension, valvular heart disease, heart failure, abnormal blood pressure; shock; vasoconstriction (including that associated with migraines); vascular abnormality, inflammation, insufficiency limited to a single organ or tissue. More particularly, the term refers to vascular disease, atherosclerosis, coronary heart disease, cerebrovascular disease, heart failure and peripheral vessel disease, and hypertension.

In one embodiment, the present invention provides pharmaceutical compositions comprising a compound of the invention, and another therapeutic agent. In a particular embodiment, the other therapeutic agent is a cardiovascular diseases treatment agent. In particular, the term cardiovascular diseases refers to arrhythmia (atrial or ventricular or both); atherosclerosis and its sequelae; angina; cardiac rhythm disturbances; myocardial ischemia; myocardial infarction; cardiac or vascular aneurysm; vasculitis, stroke; peripheral obstructive arteriopathy of a limb, an organ, or a tissue; reperfusion injury following ischemia of the brain, heart, kidney or other organ or tissue; endotoxic, surgical, or traumatic shock; hypertension, valvular heart disease, heart failure, abnormal blood pressure; shock; vasoconstriction (including that associated with migraines); vascular abnormality, inflammation, insufficiency limited to a single organ or tissue. More particularly, the term refers to vascular disease, atherosclerosis, coronary heart disease, cerebrovascular disease, heart failure and peripheral vessel disease, and hypertension.

Injection dose levels range from about 0.1 mg/kg/h to at least 10 mg/kg/h, all for from about 1 to about 120 h and especially 24 to 96 h. A preloading bolus of from about 0.1 mg/kg to about 10 mg/kg or more may also be administered to achieve adequate steady state levels. The maximum total dose is not expected to exceed about 1 g/day for a 40 to 80 kg human patient.

For the prophylaxis and/or treatment of long-term conditions, such as degenerative conditions, the regimen for treatment usually stretches over many months or years so oral dosing is preferred for patient convenience and tolerance. With oral dosing, one to four (1-4) regular doses daily, especially one to three (1-3) regular doses daily, typically one to two (1-2) regular doses daily, and most typically one (1) regular dose daily are representative regimens. Alternatively for long lasting effect drugs, with oral dosing, once every other week, once weekly, and once a day are representative regimens. In particular, dosage regimen can be every 1-14 days, more particularly 1-10 days, even more particularly 1-7 days, and most particularly 1-3 days.

Using these dosing patterns, each dose provides from about 1 to about 1000 mg of a compound of the invention, with particular doses each providing from about 10 to about 500 mg and especially about 30 to about 250 mg.

Transdermal doses are generally selected to provide similar or lower blood levels than are achieved using injection doses.

When used to prevent the onset of a condition, a compound of the invention will be administered to a patient at risk for developing the condition, typically on the advice and under the supervision of a physician, at the dosage levels described above. Patients at risk for developing a particular condition generally include those that have a family history of the condition, or those who have been identified by genetic testing or screening to be particularly susceptible to developing the condition.

A compound of the invention can be administered as the sole active agent or it can be administered in combination with other therapeutic agents, including other compound of the inventions that demonstrate the same or a similar therapeutic activity and that are determined to be safe and efficacious for such combined administration. In a specific embodiment, co-administration of two (or more) agents allows for significantly lower doses of each to be used, thereby reducing the side effects seen.

In one embodiment, a compound of the invention or a pharmaceutical composition comprising a compound of the invention is administered as a medicament. In a specific embodiment, said pharmaceutical composition additionally comprises a further active ingredient.

In one embodiment, a compound of the invention is co-administered with another therapeutic agent for the treatment and/or prophylaxis of endocrine, nutritional and metabolic diseases. Particular agents include, but are not limited to, (i) anti-diabetic agents such as insulin, insulin derivatives and mimetics; insulin secretagogues such as sulfonylureas, e.g., glipizide, glibenclamide and glimepiride; insulinotropic sulfonylurea receptor ligands such as meglitinides, e.g., nateglinide and repaglinide; insulin sensitizers; GSK-3 (glycogen synthase kinase-3) inhibitors; RXR ligands; sodium-dependent glucose co-transporter inhibitors; glycogen phosphorylase A inhibitors such as BAY R3401; biguanides such as metformin; alpha-glucosidase inhibitors such as acarbose; GLP-1 (glucagon like peptide-1), GLP-1 analogs such as exendin-4, exenatide, and GLP-1 mimetics; dipeptidyl peptidase-4 (DPP4) inhibitors; an advanced glycation end product (AGE) breaker such as *N*-phenacylthiazolium bromide, alagebrium, TRC4149 and TRC4186; a thiazolidinedione derivative (glitazone) such as pioglitazone or rosiglitazone; and a non-glitazone type PPARδ agonist; (ii) hypolipidemic agents such as 3-hydroxy-3-methyl-glutaryl coenzyme A (HMG-CoA) reductase inhibitors, e.g., lovastatin, pitavastatin, simvastatin, pravastatin, cerivastatin, mevastatin, velostatin, fluvastatin, dalvastatin, atorvastatin, rosuvastatin and rivastatin; squalene synthase inhibitors; FXR (farnesoid X receptor) and LXR (liver X receptor) ligands; cholestyramine; fibrates; nicotinic acid and aspirin; (iii) anti-obesity agents or appetite regulating agents such as phentermine, leptin, bromocriptine, dexamphetamine, amphetamine, fenfluramine, dexfenfluramine, sibutramine, orlistat, mazindol, phendimetrazine, diethylpropion, fluoxetine, bupropion, topiramate, benzphetamine, phenylpropanolamine, ecopipam, ephedrine, pseudoephedrine or cannabinoid receptor antagonists; (iv) HDL-increasing and cholesterol absorption modulators such as niacin, ezetimibe, SCH-48461 and KT6-971; (v) agents interacting with the 5-HT₃ and/or 5-HT₄ receptors, such as tegaserod, mosapride, metoclopramide, renzapride, zacopride, cisapride, alosetron, cilansetron, ondansetron, tropisetron, granisetron, dolasetron, palonosetron and ramosetron; (vi) estrogen, testosterone, selective estrogen receptor modulators, and selective androgen receptor modulators.

In one embodiment, a compound of the invention is co-administered with another therapeutic agent for the treatment and/or prophylaxis of cardiovascular diseases. Particular agents include, but are not limited to, (i) anti-hypertensive agents, e.g., loop diuretics such as etacrynic acid, furosemide and torsemide; diuretics such as thiazide derivatives, chlorothiazide, hydrochlorothiazide, amiloride; angiotensin converting enzyme (ACE) inhibitors such as benazepril, captopril, enalapril, fosinopril, lisinopril, moexipril, perindopril, quinapril, ramipril and trandolapril; inhibitors of the Na⁺/K⁺-ATPase membrane pump such as digoxin; neutral endopeptidase (NEP) inhibitors, e.g., thiorphan, acetorphan, SQ 29,072; endothelin converting enzymes (ECE) inhibitors, e.g., SLV306; ACE/NEP inhibitors such as omapatrilat, sampatrilat and fasidotril; angiotensin II receptor antagonists such as candesartan, eprosartan, irbesartan, losartan, telmisartan and valsartan; renin inhibitors such as aliskiren, terlakiren, ditekiren, RO 66-1132, RO 66-1168; β-adrenergic receptor blockers such as acebutolol, atenolol, betaxolol, bisoprolol, metoprolol, nadolol, propranolol, sotalol and timolol; inotropic agents such as digoxin, dobutamine and milrinone; calcium channel blockers such as amlodipine, bepridil, diltiazem, felodipine, nicardipine, nimodipine, nifedipine, nisoldipine and verapamil; aldosterone receptor antagonists such as anastrazole, spironolactone, fadrazole, and eplerenone; and aldosterone synthase inhibitors; (ii) Apo-A1 analogues and mimetics; (iii) thrombin inhibitors such as hirudin, bivalirudin, lepirudin, desirudin, argatroban, inogatran, melagatran, ximelagatran, and dabigatran; (iv) inhibitors of platelet aggregation such as aspirin and clopidogrel.

By co-administration is included any means of delivering two or more therapeutic agents to the patient as part of the same treatment regime, as will be apparent to the skilled person. Whilst the two or more agents may be administered simultaneously in a single formulation, i.e. as a single pharmaceutical composition, this is not essential. The agents may be administered in different formulations and at different times.

### CHEMICAL SYNTHETIC PROCEDURES

### General

The compound of the invention can be prepared from readily available starting materials using the following general methods and procedures. It will be appreciated that where typical or preferred process conditions (*i.e*., reaction temperatures, times, mole ratios of reactants, solvents, pressures, etc.) are given, other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. The choice of a suitable protecting group for a particular functional group as well as suitable conditions for protection and deprotection are well known in the art (Wuts & Greene 2006).

The following methods are presented with details as to the preparation of a compound of the invention as defined hereinabove and the comparative examples. A compound of the invention may be prepared from known or commercially available starting materials and reagents by one skilled in the art of organic synthesis.

All reagents are of commercial grade and are used as received without further purification, unless otherwise stated. Commercially available anhydrous solvents are used for reactions conducted under inert atmosphere. Reagent grade solvents are used in all other cases, unless otherwise specified. Column chromatography is performed on silica gel 60 (35-70 µm) or with Biotage^{®} SNAP KP-NH, Biotage^{®} SNAP Ultra, or Interchim^{®} PuriFlash^{®} Si HC flash chromatography cartridges. Thin layer chromatography is carried out using pre-coated silica gel F-254 plates (thickness 0.25 mm). Biotage^{®} ISOLUTE^{®} phase separators (e.g., Cat# 120-1907-E) are used for aqueous phase separation. ¹H NMR spectra are recorded on a Bruker DPX 400 NMR spectrometer (400 MHz) or a Bruker Avance 300 NMR spectrometer (300 MHz). Chemical shifts (δ) for ¹H NMR spectra are reported in parts per million (ppm) relative to tetramethylsilane (δ 0.00) or the appropriate residual solvent peak, i.e. CHCl₃ (δ 7.27), as internal reference. Multiplicities are given as singlet (s), doublet (d), triplet (t), quartet (q), quintet (quin), multiplet (m) and broad (br). Electrospray MS spectra are obtained on a Waters Acquity H-Class or I-Class UPLC system coupled to a UV PDA detector and to a Waters SQD or SQD2 mass spectrometer. Columns used: Waters Acquity UPLC BEH C18 1.7 µm, 2.1 mm ID × 30/50 mm L. The methods are using MeCN/H₂O gradients with either 0.1% formic acid in both mobile phases or 0.05% NH₄OH in both mobile phases. Preparative HPLC is performed on a Waters AutoPurification system with UV and MS detections using Waters XBRIDGE BEH C18 OBD 30 mm ID × 100 mm L columns and MeCN/H₂O gradients with either 0.1% formic acid in both mobile phases or 0.1% diethylamine in both mobile phases. Microwave heating is performed with a Biotage^{®} Initiator.

**Table I. List of abbreviations used in the experimental section**

| **Abbreviation** | **Definition** | | **Abbreviation** | **Definition** |
|---|---|---|---|---|
| AcOH | acetic acid | | HATU | (1-[bis(dimethylamino) methylene]-1H-1,2,3-triazolo[4,5-b]pyridinium 3-oxid hexafluorophosphate (CAS# 148893-10-1) |
| aq. | aqueous | | | |
| Boc | tert-butyloxy-carbonyl | | | |
| B₂pin₂ | 4,4,4',4',5,5,5',5'-octamethyl-2,2'-bi-1,3,2-dioxaborolane (CAS# 73183-34-3) | | | |
| | | | HPLC | high performance liquid chromatography |
| BBBPY | 4,4'-di-tert-butyl-2,2'-dipyridyl (CAS# 72914-19-3) | | iPrzO | diisopropyl ether |
| | | | [Ir(OMe)(1,5-cod)]₂ | (1,5-cyclooctadiene) (methoxy)iridium(I) dimer (CAS# 12148-71-9) |
| br | broad | | | |
| CDI | 1,1'-carbonyldiimidazole (CAS# 530-62-1) | | LCMS | liquid chromatography-mass spectrometry |
| d | doublet | | LDA | lithium diisopropylamide |
| DCM | dichloromethane | | m | multiplet |
| dd | doublet of doublets | | MeCN | acetonitrile |
| DIPEA | N,N-diisopropylethylamine | | MeI | iodomethane |
| DMAP | 4-(dimethylamino)pyridine | | MeOH | methanol |
| DME | 1,2-dimethoxyethane | | min | minute |
| DMF | N,N-dimethylformamide | | MS | mass spectrometry |
| EDCI | 1-ethyl-3-[3-(dimethyl amino)propyl]carbodiimide (CAS# 1892-57-5) | | MW | molecular weight |
| | | | NA | not available |
| | | | NBS | N-bromosuccinimide |
| EtOAc | ethyl acetate | | Pd₂(dba)₃ | tris(dibenzylideneacetone) dipalladium(0) (CAS# 51364-51-3) |
| EtOH | Ethanol | | | |
| eq. | Equivalent | | | |
| h | Hour | | | |
| PdCl₂(dppf). DCM | [1,1'- | | *t*BuOH | *tert*-butanol |
| | bis(diphenylphosphino) | | td | triplet of doublets |
| | ferrocene]dichloropalladium | | TEA | triethylamine |
| | (II) complex with DCM | | TFA | trifluoroacetic acid |
| | (CAS# 95464-05-4) | | THF | tetrahydrofuran |
| Pd(OAc)₂ | palladium(II) acetate | | tt | triplet of triplets |
| Pd(PPh₃)₄ | tetrakis(triphenylphosphine) palladium(0) | | Xantphos | 4,5-bis(diphenylphosphino)-9,9-dimethylxanthene (CAS# 161265-03-8) |
| ppm | part-per-million | | | |
| q | quartet | | XPhos | 2-dicyclohexylphosphino-2',4',6'-triisopropylbiphenyl (CAS# 564483-18-7) |
| RBF | round-bottom flask | | | |
| RT | room temperature | | | |
| s | singlet | | XPhos Pd G3 | (2-dicyclohexylphosphino-2',4',6'-triisopropyl-1,1'-biphenyl)[2-(2'-amino-1,1'-biphenyl)]palladium(II) methanesulfonate (CAS# 1445085-55-1) |
| sat. | saturated | | | |
| SPhos | 2-dicyclohexylphosphino-2',6'-dimethoxybiphenyl (CAS# 657408-07-6) | | | |
| t | triplet | | | |

### SYNTHETIC PREPARATION OF THE COMPOUNDS OF THE INVENTION

### Example 1. General synthetic methods

### 1.1. Synthetic methods overview

**General method A: Bromination of a pyrazolopyridine with NBS**
**General method B: Difluoromethylation of pyrazoles**
**General method C: Pyrazoles synthesis by cyclization with hydrazines**
**General method D: Opening of oxazoles with Mo(CO)₆**
**General method E: Suzuki coupling**
**General method F: Saponification with NaOH**
**General method G: 4-hydroxy- and 4-alkyl-thiazoles synthesis from thioamides**
**General method H: Alkylation of hydroxythiazoles**
**General method I: Ullmann reaction**
**General method J: Alkylation of carboxylic acid with Mel**
**General method K: Suzuki coupling of halogenated heterocycles with Int 22**
**General method L: Suzuki coupling on a 2-bromofuran**
**General method M: Amide synthesis from aqueous ammonia**
**General method N: Synthesis of thioamides from nitriles**
**General method O: Metal-catalyzed borylation reaction**
**General method P: Chlorination of β-diketones and malonates with SO₂Cl₂**
**General method Q: Hydrogenation**

### 1.2. General methods

### 1.2.1. Method A: Bromination of a pyrazolopyridine with NBS

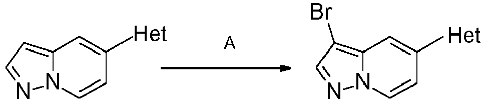

To a solution of the pyrazolopyridine (1 eq.) in DMF (or a mixture DMF/DCM) at 0 °C is added NBS (1 eq. to 1.2 eq.). The reaction mixture is stirred for 10 min to overnight at RT or heated at 50 °C to 80 °C.
*Alternative work-up 1*: the reaction mixture is filtered, the solid is washed with water and dried to afford the expected compound.
*Alternative work-up 2*: water is added. The precipitate is filtered, washed and dried to afford the expected compound.
*Alternative work-up 3*: water is added and the aq. layer is extracted with EtOAc or DCM. The combined organic layers are dried, filtered and the filtrate is concentrated to dryness to afford the expected compound. *Alternative work-up 4*: the reaction mixture is concentrated. Water is added and the solution is stirred 10 min. The precipitate is filtered, washed with water, EtOH and/or pentane to afford the expected product.

### 1.2.1.1. Illustrative synthesis of Int 20

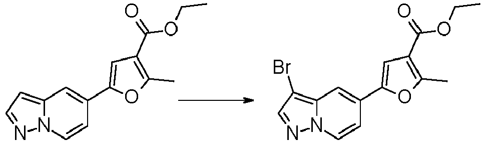

To a cooled suspension of **Int 21** (0.210 g, 0.820 mmol, 1 eq.) in DMF (1.5 mL) is added NBS (0.153 g, 0.861 mmol, 1.05 eq.) portionwise. The reaction mixture is warmed up to RT and stirred 1 h at RT. Water is added and the precipitate is filtered to afford **Int 20.**

### 1.2.2. Method B: Difluoromethylation of pyrazoles

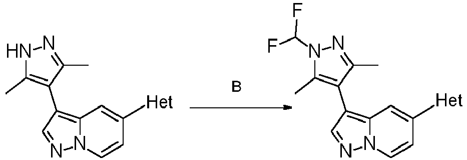

### 1.2.2.1. Method B1:

To a solution of the pyrazole intermediate (1 eq.) in DMF is added Cs₂CO₃ (5 eq.) and ethyl 2-chloro-2,2-difluoro-acetate (CAS# 383-62-0; 1.1 eq. to 1.2 eq.). The reaction mixture is stirred at 60 °C overnight. Water is added, the aq. layer is extracted with EtOAc and the combined organic layers are washed with brine, dried over Na₂SO₄ (or MgSO₄), filtered and concentrated. The crude is purified by flash chromatography on silica gel to afford the expected compound.

### 1.2.2.1.1 Illustrative synthesis of Cpd 117

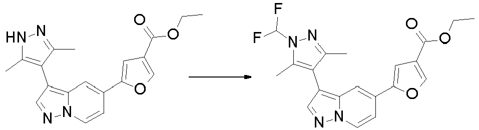

To a solution of **Int 36** (150 mg, 0.428 mmol, 1 eq.) in DMF (4 mL) is added Cs₂CO₃ (697 mg, 2.14 mmol, 5 eq.) and ethyl 2-chloro-2,2-difluoro-acetate (65 µL, 0.514 mmol, 1.2 eq.). The reaction mixture is stirred at 60 °C for 24 h. Water is added, the aq. layer is extracted with EtOAc and the combined organic layers are washed with brine, dried over MgSO₄, filtered and concentrated. The crude is purified by flash chromatography on silica gel (solid load, eluting with 0% to 50% EtOAc in heptane) to afford **Cpd 117.**

### 1.2.2.2. Method B2:

To a solution of pyrazole intermediate (1 eq.) in DMF is added sodium 2-chloro-2,2-difluoro-acetate (CAS# 1895-39-2; 1.5 eq. to 2.5 eq.) and Cs₂CO₃ (5 eq). The reaction mixture is stirred at 60 °C (or 100 °C) for 1 h to overnight. Water is added, the aq. phase is extracted with EtOAc and the combined organic layers are washed with brine, dried over Na₂SO₄ (or MgSO₄), filtered and concentrated. The crude is purified by flash chromatography on silica gel to afford the expected compound.

### 1.2.2.2.1 Illustrative synthesis of Cpd 152

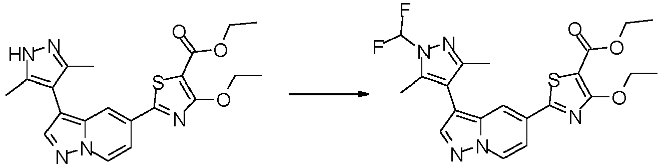

To a solution of **Int 64** (54 mg, 0.131 mmol, 1 eq.) in DMF (2 mL) is added Cs₂CO₃ (213 mg, 0.655 mmol, 5 eq.) and sodium ethyl 2-chloro-2,2-difluoro-acetate (24 mg, 0.157 mmol, 1.2 eq.). The reaction mixture is stirred at 60 °C for 24 h. Water is added, the aq. layer is extracted with EtOAc and the combined organic layers are washed with brine, dried over MgSO₄, filtered and concentrated. The crude is purified by flash chromatography on silica gel (eluting with 0% to 50% EtOAc in heptane) to afford **Cpd 152.**

### 1.2.3. Method C: Pyrazoles synthesis by cyclization with hydrazines

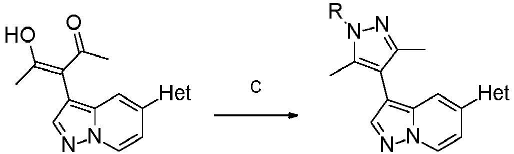

In a sealed tube, to a solution of the acetylacetone intermediate (1 eq.) in EtOH is added the hydrazine (1 eq. to 3.3 eq.) and DIPEA (0 to 6.6 eq.). The reaction mixture is stirred for 1 h to 3 days at 60 °C to 80 °C.
*Alternative work-up 1*: the reaction mixture is concentrated and the crude is purified by preparative HPLC or by flash chromatography to afford expected product.
*Alternative work-up 2*: the reaction mixture is concentrated, water is added, the aq. layer is extracted with EtOAc and the combined organic layers are dried over Na₂SO₄, filtered and concentrated. The filtrate is purified by flash chromatography on silica gel to afford the expected compound.

### 1.2.3.1. Illustrative synthesis of Cpd110

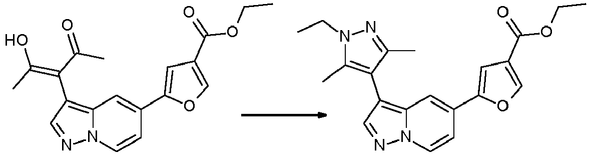

In a sealed tube, to a solution of **Int 19** (35 mg, 0.1 mmol, 1 eq.) in EtOH (1 mL) is added ethylhydrazine oxalate (CAS# 6629-60-3, 16.5 mg, 0.11 mmol, 1.1 eq.) and DIPEA (38 µL, 0.22 mmol, 2.2 eq.). The reaction mixture is stirred for 1 h at 60 °C, and then concentrated. The crude is purified by flash chromatography on silica gel (eluting with 0% to 60% EtOAc in heptane) to afford **Cpd 110.**

### 1.2.4. Method D: Opening of oxazoles with Mo(CO)6

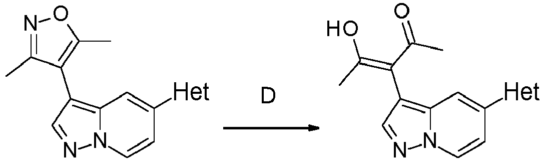

### Step 1:

To a solution of the oxazole intermediate (1 eq.) in a MeCN/water 3/1 mixture is added Mo(CO)₆ (0.4 eq. to 3 eq.). The reaction mixture is stirred at 90 °C for 2 h to 5.5 h.
*Alternative work-up 1:* the reaction mixture is concentrated and the crude is used as such in step 2 *Alternative work-up 2:* the reaction mixture is concentrated, the residue is taken up in a (DCM or
EtOAc)/water mixture and the combined organic layers are dried and concentrated. The residue is used as such in step 2.
*Alternative work-up 3:* The reaction mixture is filtered on decalite, solids are washed with DCM and the filtrate is concentrated. The residue is used without purification in step 2.
*Alternative work-up 4:* The reaction mixture is cooled to 0 °C, the precipitate is filtered, washed and dried *in vacuo* to afford expected product.

### Step 2:

The residue is dissolved in a (THF or EtOH)/water (1/1) mixture and the oxalic acid (3 to 7 eq.) is added. The reaction mixture is stirred at 50 to 70 °C for 1 h to overnight.
*Alternative work up 1*: The reaction mixture is concentrated. The residue is solubilized in DCM and the organic layer is washed with water, then concentrated and the residue is triturated in iPr₂O. The solids are filtrated and dried to afford expected product.
*Alternative work up 2:* THF or EtOH is evaporated and the suspension in water is filtrated, washed with water then dried *in vacuo* to afford expected product.
*Alternative work-up 3:* The reaction mixture is filtered. The solid is dried to afford the expected product. *Alternative work-up 4:* The reaction mixture is concentrated and the crude is purified by flash chromatography on silica gel to afford the expected compound.

### 1.2.4.1. Illustrative synthesis of Int 30

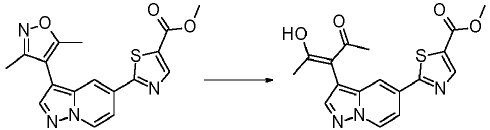

To a solution of **Cpd 107** (140 mg, 0.395 mmol, 1 eq.) in a MeCN/water (8 mL) is added Mo(CO)₆ (105 mg, 0.395 mmol, 1 eq.). The reaction mixture is stirred at 90 °C for 2 h. The reaction mixture is concentrated, the residue is taken up in DCM, the suspension is filtered and the filtrate is concentrated. The crude is dissolved in a THF/water (20 mL) and the oxalic acid (250 mg, 2.76 mmol, 7 eq.) is added. The reaction mixture is stirred at 70 °C for 3 h. The solvent is evaporated, the residue is diluted in DCM and washed with water. The organic layer is dried, then concentrated and the crude is triturated in iPr₂O. The solid is filtrated and dried to afford **Int 30.**

### 1.2.5. Method E: Suzuki coupling

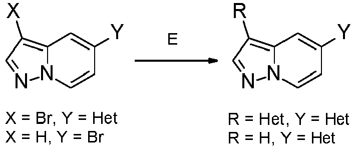

To a solution of the brominated compound (1 eq.) in a degassed mixture of dioxane and water (4/1) are added boronate (1 to 4.6 eq.), Cs₂CO₃ or K₃PO₄ (2 to 3 eq.), and Pd(dppf)Cl₂.DCM or XPhos Pd G3 (0.05 to 0.15 eq.). The reaction mixture is stirred at 80 °C to 100 °C for 1 h to 2 days.
*Alternative work-up 1*: the reaction mixture is concentrated. The residue is directly purified by flash chromatography on silica gel to afford the expected compound.
*Alternative work-up 2:* the reaction mixture is concentrated. Water is added and the aq. phase is extracted with EtOAc or DCM. The combined organic layers are dried over Na₂SO₄, filtered and concentrated. The crude is purified by chromatography on silica gel to afford the expected compound.
*Alternative work-up* 3: the reaction mixture is filtered on Celite^{®}. Solids are washed with EtOAc and the filtrate is concentrated. The residue is used as such.

### 1.2.5.1. Illustrative synthesis of Cpd 93

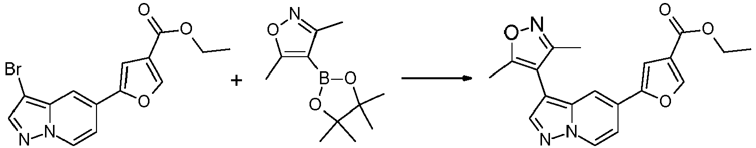

To a solution of **Int 8** (1.4 g, 4.18 mmol, 1 eq.) in a degassed mixture of dioxane/water (20 mL) are added 3,5-dimethyl-4-(4,4,5,5-tetramethyl-1,3,2-dioxaborolan-2-yl)isoxazole (CAS# 832114-00-8; 1.12 g, 5.01 mmol, 1.2 eq.), Cs₂CO₃ (3.4 g, 10.45 mmol, 2.5 eq.) and Pd(dppf)Cl₂.DCM (171 mg, 0.21 mmol, 0.05 eq.). The reaction mixture is stirred at 100 °C for 2 h. Water is then added, the aq. phase is extracted with DCM and the combined organic layers are dried over Na₂SO₄, filtered and concentrated. The crude mixture is purified by chromatography on silica gel eluting with 0 to 100% EtOAc in heptane to afford the expected compound.

### 1.2.6. Method F: Saponification with NaOH

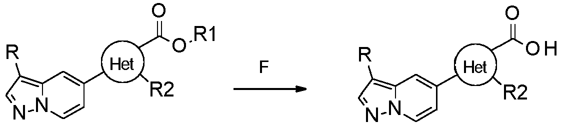

To a solution of the ester intermediate (1 eq.) in a THF/(MeOH or EtOH) mixture (2/1) is added NaOH (2 eq). The reaction mixture is stirred at RT or 50 °C for 1 h to overnight.

The reaction mixture is concentrated. Water is added and the aq. phase is acidified with an aq. solution of 1N HCl. The precipitate is filtrated, washed and dried to afford expected acid compound. Some acids are purified by preparative HPLC.

### 1.2.6.1. Illustrative synthesis of Cpd 35

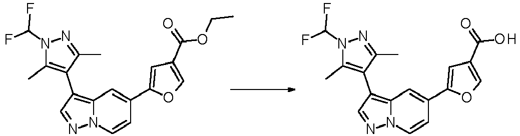

To a solution of **Cpd 117** (1.75 g, 4.37 mmol, 1 eq.) in a THF/MeOH mixture (22 mL), a 1N aq. solution of NaOH (4.4 mL, 2 eq.) is added. The reaction mixture is stirred at 50 °C for 1 h, then concentrated. Water is added and the aq. phase is acidified. The precipitate is filtrated. The solid is triturated in iPrzO and filtrated to afford **Cpd 35.**

### 1.2.7. Method G: 4-hydroxy- and 4-alkyl-thiazoles synthesis from thioamides

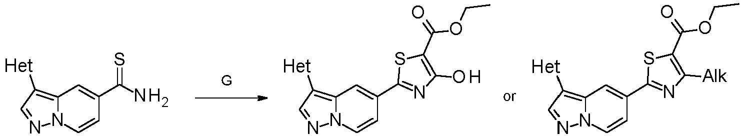

To a solution of thioamide (1 eq.) in EtOH are added the malonate or the β-diketone (1.1 eq. to 1.5 eq.) and pyridine (4 eq.; only when malonates are used). The reaction mixture is heated at reflux for 1 h to overnight.
*Alternative work-up 1* (hydroxythiazoles): the reaction mixture is cooled to RT and poured into an ice/water mixture. The precipitate is filtered and dried *in vacuo* or extracted with EtOAc, concentrated and the residue is purified by chromatography on silica gel to afford the expected compound.
*Alternative work-up 2* (alkylthiazoles): the mixture is concentrated *in vacuo.* The residue is purified by flash chromatography on silica gel to afford the expected compound.

### 1.2.7.1. Illustrative synthesis of Int 56

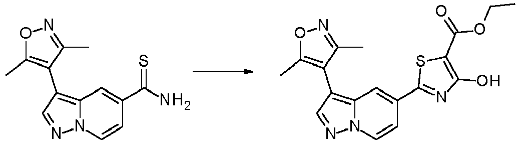

To a solution of **Int 57** (136 mg, 0.5 mmol, 1 eq.) in EtOH (3 mL) are added diethyl 2-bromopropanedioate (CAS# 685-87-0; 126 µL, 0.75 mmol, 1.5 eq.) and pyridine (161 µL, 2 mmol, 4 eq.). The reaction mixture is heated at reflux for 1 h then cooled to RT and poured into an ice/water mixture. The aq. phase is extracted with EtOAc, and the organic layer is concentrated. The crude is purified by flash chromatography on silica gel (eluting with 0 to 40% EtOAc in heptane) to afford **Int 56.**

### 1.2.8. Method H: Alkylation of hydroxythiazoles

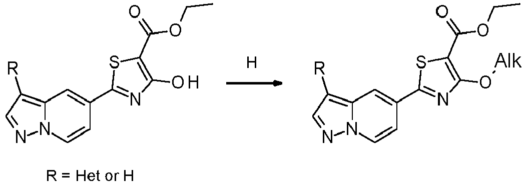

To a solution of the hydroxythiazole intermediate (1 eq.) in DMF are added the alkyl halide (1.5 to 11.5 eq.) or sulfonate (3 eq.) and K₂CO₃ (2 eq. to 4 eq.). The reaction mixture is heated at 60 to 120 °C for 1 h to overnight.
*Alternative work-up 1*: the reaction mixture is cooled to RT and water is added. The aq. phase is extracted with EtOAc and the organic phase is concentrated. The residue is purified by flash chromatography on silica gel to afford the expected compound.
*Alternative work-up 2:* the reaction mixture is poured into an ice/water mixture. The precipitate is filtered, washed with water and dried *in vacuo* to afford expected compound.

### 1.2.8.1. Illustrative synthesis of Cpd 136

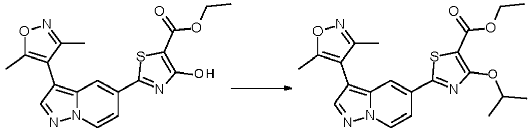

To a solution of **Int 56** (40 mg, 0.104 mmol, 1 eq.) in DMF (2 mL) are added 2-iodopropane (CAS# 75-30-9; 16 µL, 0.156 mmol, 1.5 eq.) and K₂CO₃ (2 9 mg, 0.208 mmol, 2 eq.). The reaction mixture is heated at 80 °C for 1 h. The reaction mixture is poured into an ice/water mixture. The precipitate is filtered, washed with water and dried *in vacuo* to afford **Cpd 136.**

### 1.2.9. Method I: Ullmann reaction

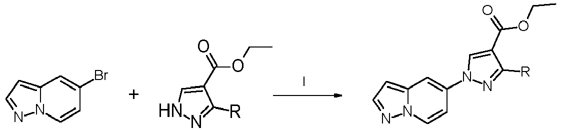

In a sealed tube, to a solution of the 5-bromopyrazolo[1,5-a]pyridine (CAS# 1060812-84-1; 1 eq.) in toluene is added the pyrazole derivative (1 eq.), K₂CO₃ (2.1 eq.) and CuI (0.05 to 0.1 eq.). The reaction mixture is stirred and degassed 10 min. Trans-N,N'-dimethylcyclohexane-1,2-diamine (CAS# 67579-81-1; 0.2 to 0.4 eq.) is added and the reaction mixture is heated at 100 °C for 2 h to overnight. The reaction mixture is cooled and filtered. The solid is washed with a DCM/MeOH (9/1) mixture. The filtrate is concentrated and the residue purified by flash chromatography on silica gel to afford the expected compound.

### 1.2.9.1. Illustrative synthesis of Int 60

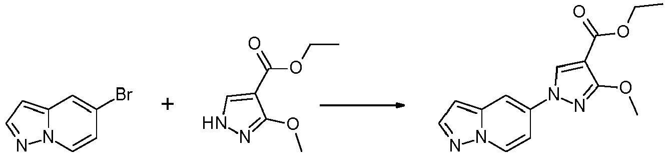

In a sealed tube, to a solution of 5-bromopyrazolo[1,5-a]pyridine (CAS# 1060812-84-1; 500 mg, 2.54 mmol, 1 eq.) in toluene (3 mL) is added ethyl 3-methoxy-1H-pyrazole-4-carboxylate (CAS# 478968-48-8; 432 mg, 2.54 mmol, 1 eq.), K₂CO₃ (736 mg, 5.33 mmol, 2.1 eq.), and CuI (48 mg, 0.254 mmol, 0,1 eq.). The reaction mixture is stirred and degassed 10 min. Trans-N,N'-dimethylcyclohexane-1,2-diamine (CAS# 67579-81-1; 160 µL, 1 mmol, 0.4 eq.) is added and the reaction mixture is heated at 100 °C overnight then cooled and filtered. The solid is washed with a DCM/MeOH (9/1) mixture. The filtrate is concentrated and purified by chromatography on silica gel (eluting with 0 to 50% EtOAc in heptane) to afford **Int 60.**

### 1.2.10. Method J: Alkylation of carboxylic acid with MeI

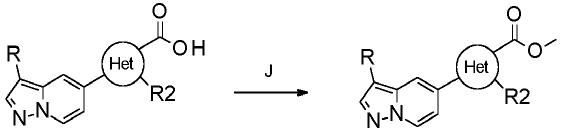

To a solution of acid derivative (1 eq.) in DMF are added iodomethane (1.1 to 2 eq.) and K₂CO₃ (1.5 to 2 eq.). The reaction mixture is stirred at RT or 50 °C for 3 h to 2 days. The reaction mixture is then quenched with water.
*Alternative work-up 1*: the precipitate is filtered, washed with water and dried *in vacuo* to afford the expected product.
*Alternative work-up 2:* extraction with EtOAc or DCM. The two phases are separated and the organic phase is dried over Na₂SO₄. After filtration and concentration, the residue is used as such or purified by flash chromatography to afford expected product.

### 1.2.10.1. Illustrative synthesis of Int 2

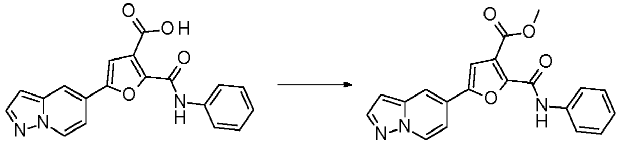

To a solution of **Int 3** (940 mg, 2.7 mmol, 1 eq.) in DMF (10 mL) are added iodomethane (252 µL, 5.41 mmol, 2 eq.) and K₂CO₃ (748 mg, 5.41 mmol, 2 eq.). The reaction mixture is stirred at RT for 4 h and at 50 °C for 2 h. Water is added and the aq. phase is extracted with DCM. The two phases are separated and the organic phase is dried over Na₂SO₄. After filtration and concentration of the organic phase, the residue is purified by flash chromatography on silica gel (eluting with a solution of DCM/MeOH 98/2) to afford **Int 2.**

### 1.2.11. Method K: Suzuki coupling of halogenated heterocycles with Int 22

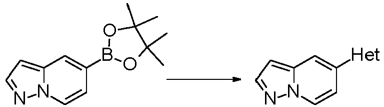

To a solution of halogenated heterocycle (1 eq.) in a degassed mixture of dioxane and water (4/1) are added **Int 22** (1.1 eq.), PdCl₂(dppf).DCM or XPhos Pd G3 (0.05 eq.) and Cs₂CO₃ (3 eq.). The mixture is heated at 110 °C for 1 h to 2 h then cooled to RT. The reaction mixture is concentrated. The residue is diluted in DCM, filtered on Celite^{®}. After concentration, the filtrate is purified by flash chromatography on silica gel to afford expected product.

### 1.2.11.1. Illustrative synthesis of Int 24

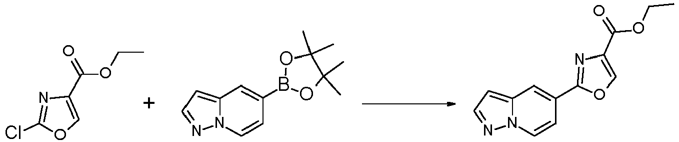

To a solution of ethyl 2-chlorooxazole-4-carboxylate (CAS# 460081-18-9; 900 mg, 5.14 mmol, 1 eq.) in a degassed mixture of dioxane/water 4/1 (22.5 mL) are added **Int 22** (1.38 g, 5.66 mmol, 1.1 eq.), PdCl₂(dppf).DCM (210 mg, 0.257 mmol, 0.05 eq.) and Cs₂CO₃ (5.03 g, 15.4 mmol, 3 eq.). The mixture is heated at 110 °C for 2 h, then concentrated. The residue is diluted in DCM, filtered on Celite^{®}, eluting with EtOAc. After concentration, the filtrate is purified by flash chromatography on silica gel (eluting with heptane/EtOAc 50/50) to afford **Int 24.**

### 1.2.12. Method L: Suzuki coupling on a 2-bromofuran

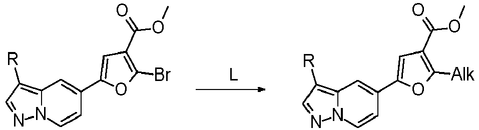

To a solution of bromofuran derivative (1 eq.) in a degassed mixture dioxane/water (4/1) are added the boronate, boronic acid, or boroxine (2 eq.), PdCl₂(dppf).DCM (0.05 eq.) and Cs₂CO₃ (3 eq.). The reaction mixture is heated at 90 °C for 2 h then concentrated. The residue is purified by flash chromatography on silica gel to afford expected product.

### 1.2.12.1. Illustrative synthesis of Cpd 99

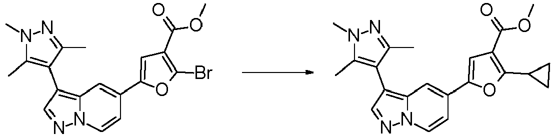

To a solution of **Int 18** (86 mg, 0.2 mmol, 1 eq.) in a degassed dioxane/water (4/1) mixture (2 mL) are added 2-cyclopropyl-4,4,5,5-tetramethyl-1,3,2-dioxaborolane (CAS# 126689-01-8; 68 mg, 0.4 mmol, 2 eq.), PdCl₂(dppf).DCM (8 mg, 0.01 mmol, 0.05 eq.) and Cs₂CO₃ (196 mg, 0.6 mmol, 3 eq.). The reaction mixture is heated at 90 °C for 2 h then concentrated. The residue is purified by flash chromatography on silica gel (eluting with EtOAc 100%) to afford **Cpd 99.**

### 1.2.13. Method M: Amide synthesis from aqueous ammonia

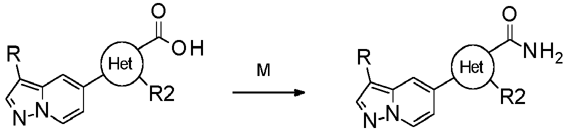

To a suspension of the acid derivative (1 eq.) in anhydrous THF is added CDI (1.5 eq.). The reaction mixture is stirred at RT for 1 h then a solution of ammonia in water (20%) is added (5.0 eq.). The mixture is stirred at RT for 30 min to 1 h.
*Alternative work-up 1*: the reaction mixture is concentrated *in vacuo.* The residue is purified by flash chromatography on silica gel to afford the expected compound (**Cpd 43**).
*Alternative work-up 2:* water is added to the reaction mixture. The precipitate is filtered, washed with water then dried *in vacuo* to afford the expected compound (**Cpd 82, Cpd 83**).

### 1.2.13.1. Illustrative synthesis of Cpd 43

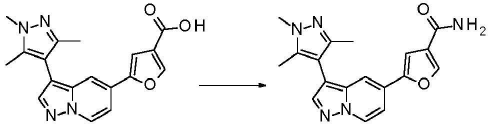

In a 10 mL RBF is charged **Cpd 2** (30.0 mg, 0.089 mmol, 1.0 eq.). Anhydrous THF is added (0.6 mL) followed by CDI (CAS# 530-62-1; 22.0 mg, 0.133 mmol, 1.5 eq.). The reaction mixture is stirred at RT for 1 h then a 20% aq. solution of NH₄OH (0.084 mL, 0.445 mmol, 5 eq.) is added rapidly at RT. The reaction mixture is stirred for 30 min at RT then concentrated *in vacuo.* The residue is purified by flash chromatography on silica gel (dry loading, elution with a DCM - DCM/MeOH 90/10 0%to 100% gradient). After concentration of the fractions containing expected product, the solid is triturated in water to afford **Int 26.**

### 1.2.14. Method N: Synthesis of thioamides from nitriles

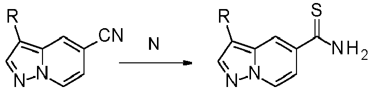

To a solution of the cyano derivative (1 eq.) in pyridine are added (NH₄)₂S (solution in water 40-48 wt%; 1.1 eq.) and TEA (1 eq.). The reaction mixture is heated at 55 °C for 1.5 h to 2 h then flushed with an N₂ stream. Water is added and the aq. phase is extracted with EtOAc. The two phases are separated, the organic layer is dried over Na₂SO₄ and dried *in vacuo* to afford the expected product.

### 1.2.14.1. Illustrative synthesis of Int 15

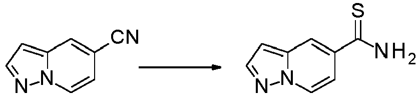

To a solution of pyrazolo[1,5-a]pyridine-5-carbonitrile (CAS# 1352903-96-8; 584 mg, 4 mmol, 1 eq.) in pyridine (2.6 mL) are added (NH₄)₂S (solution in water 40-48 wt%; 750 µL) and TEA (540 µL, 4 mmol, 1 eq.). The reaction mixture is heated at 55 °C for 2 h then flushed with an N₂ stream. Water is added and the aq. phase is extracted with EtOAc. The two phases are separated, the organic layer is dried over Na₂SO₄ and dried *in vacuo* to afford **Int 15.**

### 1.2.15. Method O: Metal-catalyzed borylation reaction

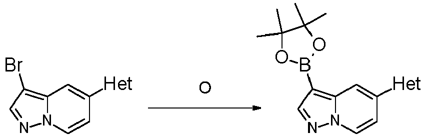

To a solution of brominated derivative (1 eq.) in degassed DME are added B₂pin₂ (1.5 eq. to 3 eq.), Pd(OAc)₂ (0.05 eq. to 0.1 eq.), PCy₃.HBF₄ (CAS# 58656-04-5; 0.1 eq.) and K₂CO₃ (2 eq.). The mixture is heated at 90 to 100 °C for 45 min to 1.5 h.
*Alternative work-up 1:* the reaction mixture is diluted in EtOAc and THF. The mixture is filtered on Celite^{®}, the solids are washed with THF or EtOAc. The filtrate is concentrated under vacuum to afford the expected product.
*Alternative work-up 2:* the reaction mixture is concentrated and the residue is used as such in the next step.

### 1.2.15.1. Illustrative synthesis of Int 25

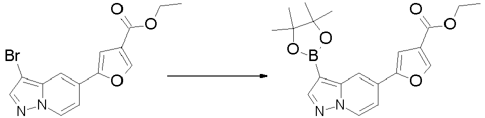

To a solution *of* ***Int* 8** (300 mg, 0.90 mmol, 1 eq.) in degassed DME (3 mL) are added Bzpinz (341 mg, 1.34 mmol, 1.5 eq.), Pd(OAc)₂ (10.0 mg, 0.045 mmol, 0.05 eq.), PCy₃.HBF₄ (CAS# 58656-04-5; 33.0 mg, 0.09 mmol, 0.1 eq.) and K₂CO₃ (249 mg, 1.80 mmol, 2 eq.). The mixture is heated at 90 °C for 45 min then diluted in EtOAc and THF. The mixture is filtered, solids are washed with THF. The filtrate is concentrated. The residue is triturated in MeCN. The solid is filtered and dried *in vacuo* to afford **Int 25.**

### 1.2.16. Method P: Chlorination of β-diketones and malonates with SO₂Cl₂

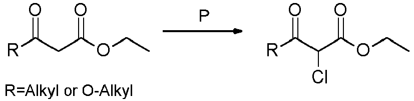

To a cooled solution of malonate or β-diketone (1 eq.) in DCM is slowly added SO₂Cl₂ (1 eq.). The reaction mixture is allowed to warm up to RT and then stirred for 1 h. The reaction mixture is then refluxed for 2 h. The reaction mixture is concentrated and the crude is taken up in toluene and then concentrated to afford the expected compound.

### 1.2.16.1. Illustrative synthesis of Int 46

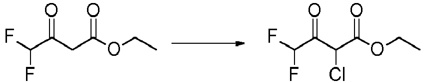

To a cooled solution of ethyl 4,4-difluoroacetoacetate (CAS# 352-24-9; 0.65 mL, 5 mmol, 1 eq.) in DCM (5 mL) is slowly added SO₂Cl₂ (0.41 mL, 5 mmol, 1 eq.). The reaction mixture is allowed to warm up to RT and stirred for 1 h. The reaction mixture is then refluxed for 2 h, concentrated and the crude is taken up in toluene and then concentrated to afford **Int 46.**

### 1.2.17. Method Q: Hydrogenation

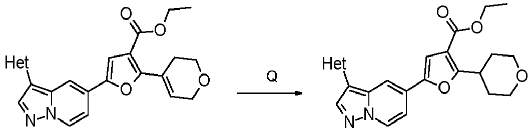

To a solution of the alkene intermediate (1 eq.) in EtOAc/EtOH (3/1 or 1/1) is added Pd/C or Pd(OH)₂/C. The reaction mixture is flushed with H₂ and stirred at RT for 48 h. The reaction mixture is filtered on Celpure^{®} P65, the filtrate is concentrated *in vacuo* to afford the expected compound.

### 1.2.17.1. Illustrative synthesis of Cpd 124

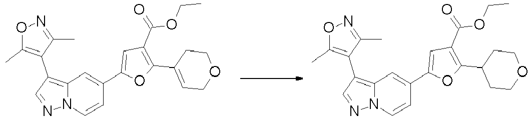

To a solution of **Cpd 122** (55 mg, 0.127 mmol, 1 eq.) in EtOAc/EtOH (4 mL) is added Pd/C. The reaction mixture is flushed with Hz and stirred at RT for 48 h. The reaction mixture is filtered on Celpure^{®} P65, the filtrate is concentrated *in vacuo* to afford **Cpd 124.**

### Example 2. Preparation of the compounds of the invention

### 2.1. Int 3

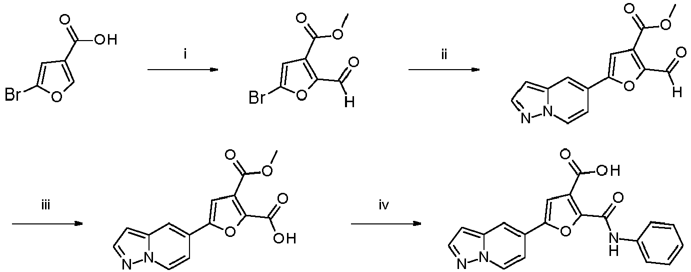

### 2.1.1. Step i: Int 6

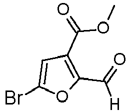

To a solution of 5-bromofuran-3-carboxylic acid (CAS# 58832-36-3; 1.2 g, 6.28 mmol, 1 eq.) in THF (25 mL) at -78 °C, is added LDA 2M in THF (6.9 mL, 13.8 mmol, 2.2 eq.). The reaction mixture is warmed up to -30 °C and stirred for 1.5 h. The reaction mixture is cooled down to -78 °C then DMF (1.45 mL, 3 eq.) is added and the reaction mixture is stirred at -78 °C for 1 h. The reaction mixture is quenched by addition of HCl 1N and extracted with DCM. The organic phase is concentrated to dryness and the residue is dissolved in DMF (15 mL). Iodomethane (0.785 mL, 12.6 mmol, 2 eq.) and K₂CO₃ (1.74 g, 12.6 mmol, 2 eq.) are added and the reaction mixture is stirred at RT for 3 h. Water is added and the reaction mixture is extracted with EtOAc. The organic layer is concentrated. The residue is purified by flash chromatography on silica gel (eluting with a heptane/EtOAc 80/20 mixture) to afford **Int 6.**

### 2.1.2. Step ii: Int 5

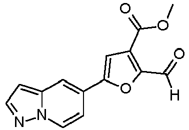

To a solution of **Int 6** (0.8 g, 3.43 mmol, 1 eq.) in a degassed mixture of dioxane/H₂O 4/1 (17 mL) is added **Int 22** (1 g, 4.1 mmol, 1.2 eq.), Pd(OAc)₂ (0.039 g, 0.172 mmol, 0.05 eq.), SPhos (0.176 g, 0.43 mmol, 0.125 eq.), K₃PO₄ (2.2 g, 10.3 mmol, 3 eq.). The reaction mixture is stirred at RT for 3 h. Water is added and the precipitate is filtered to afford **Int 5.**

### 2.1.3. Step iii: Int 4

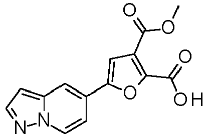

To a solution of **Int** 5 (0.930 g, 3.44 mmol, 1 eq.) in MeCN (20 mL) and a mixture *t*BuOH/H₂O 7/3 (30 mL) is added NaClO₂ (1.32 g, 14.67 mmol, 4.3 eq.), NaH₂PO₄ (1.76 g, 14.67 mmol, 4.3 eq.) and 2-methyl-2-butene (2 mL, 18.9 mmol, 5.5 eq.). The reaction mixture is stirred 2 h at RT then diluted with water and acidified with a solution of HCl 2N. The reaction mixture is extracted with DCM and the organic layer is concentrated. The residue is taken up in a mixture Et₂O/pentane and the solid is filtered and dried under vaccum to afford **Int 4.**

### 2.1.4. Step iv: Int 3

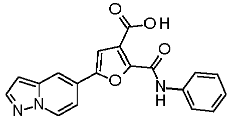

To a solution of **Int 4** (0.750 g, 2.62 mmol, 1 eq.) in DMF (10 mL) is added aniline (CAS# 62-53-3; 0.263 mL, 2.88 mmol, 1.1 eq.), HATU (1.1 g, 2.88 mmol, 1.1 eq.) and DIPEA (2.3 mL, 13.1 mmol, 5 eq.). The reaction mixture is stirred at RT for 12 h. Water is added and the precipitate is filtered. The solid is dissolved in a THF/MeOH mixture (10 mL) and an aq. solution of NaOH 1N (5.25 mL, 5.24 mmol, 2 eq.) is added. The reaction mixture is stirred 2 h at RT then concentrated. Water and then an aq. solution of HCl 1N are added. The precipitate is filtered to afford **Int 3.**

### 2.2. Int 7

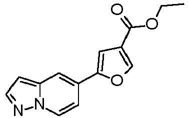

Ethyl furan-3-carboxylate (CAS# 614-98-2; 50.0 g, 357 mmol, 2.38 eq.), Bzpinz (38.0 g, 150 mmol, 1.00 eq), BBBPY (0.82 g, 3.0 mmol, 0.02 eq.), and [Ir(OMe)(1,5-cod)]z (1.00 g, 1.48 mmol, 0.01 eq.) are added to THF (275 mL). The reaction mixture is then refluxed for 1 h. The reaction mixture is then cooled to RT. 5-Bromopyrazolo[1,5-a]pyridine (CAS# 1060812-84-1; 53.0 g, 269 mmol, 0.9 eq.), K₃PO₄ (117 g, 540.2 mmol, 2.00 eq.), Pd(OAc)₂ (0.65 g, 2.8 mmol, 0.01 eq.) and tri(o-tolyl)phosphine (CAS# 6163-58-2; 1.67 g, 5.38 mmol, 0.02 eq) are added. Water (75 mL) is then slowly added keeping the reaction temperature below 30 °C. The reaction mixture is then heated at 55 °C for 1 h and cooled to RT. Water (300 mL) and EtOAc (250 mL) are added. The aq. phase is extracted with EtOAc (100 mL). The combined organic phases are washed with 20% aq. NaCl. A solvent exchange is performed with EtOH to induce crystallization; the suspension is concentrated till a weight of 300 g and stirred at RT for 30 min. The suspension is filtered and the solid is washed successively with EtOH and heptane. The solid is dried under reduced pressure to afford **Int 7.**

### 2.3. Int 8

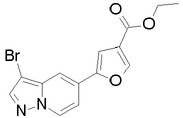

NBS (12.5 g, 69.5 mmol, 1.10 eq.) is added portionwise to a suspension of **Int 7** (16.2 g, 63.2 mmol, 1.00 eq.) in 1-methyl-2-pyrrolidinone (80 mL) keeping the temperature below 30 °C. The reaction mixture is stirred at RT for 15 min and then water (80 mL) is slowly added keeping the temperature below 30 °C. The suspension is stirred at RT for 30 min, filtered and the solid is washed with water. The solid is dried under reduced pressure to afford **Int 8.**

### 2.4. Int 9

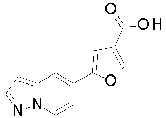

To a solution of 5-bromopyrazolo[1,5-a]pyridine (CAS# 1060812-84-1; 3.76 g, 19.1 mmol, 1 eq.) in a degassed mixture of dioxane/water 4/1 (100 mL) are added 5-(4,4,5,5-tetramethyl-1,3,2-dioxaborolan-2-yl)furan-3-carboxylic acid (CAS# 1073354-94-5; 5 g, 21 mmol, 1.1 eq.), PdCl₂(dppf).DCM (0.780 g, 0.955 mmol, 0.05 eq.), and Cs₂CO₃ (18.6 g, 57 mmol, 3 eq.). The reaction mixture is heated at 100 °C for 2 h. 5-(4,4,5,5-tetramethyl-1,3,2-dioxaborolan-2-yl)furan-3-carboxylic acid (5 g, 21 mmol , 1.1 eq.) is added for a second time, then Pd(PPh₃)₄ (1.10 g, 0.955 mmol, 0.05 eq.). The reaction mixture is heated at 100 °C overnight and then concentrated in vacuum. Water is added to the residue. The aq. phase is acidified and the precipitate is recovered to afford **Int 9.**

### 2.5. Int 18

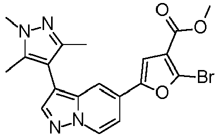

To a solution of **Cpd 5** (280 mg, 0.8 mmol, 1 eq.) in a mixture of DCM/DMF 6/1 (7 mL) at RT is added NBS (150 mg, 0.84 mmol, 1.05 eq.). The reaction mixture is stirred 6 h at RT, then quenched with water. The aq. layer is extracted with DCM. The combined organic layers are dried over anhydrous Na₂SO₄, filtered, and concentrated *in vacuo.* The residue is purified by flash chromatography on silica gel (eluting with EtOAc) to afford ***Int 18.***

### 2.6. Int 22

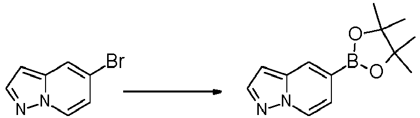

A RBF is charged with potassium acetate (14.94 g, 152.24 mmol, 3 eq.). The whole system is dried and flushed with N₂. Anhydrous dioxane (200 mL) is added and the resulting suspension is degassed with N₂ (bubbling for 20 min). 5-bromopyrazolo[1,5-a]pyridine (CAS# 1060812-84-1; 10 g, 50.75 mmol, 1 eq.), Bzpinz (14.17 g, 55.82 mmol, 1.1 eq.) and Pd(dppf)Cl₂.DCM (4.14 g, 5.07 mmol, 0.1 eq.) are introduced into the mixture at RT. The RBF is equipped with a reflux condenser and heated to 105-110 °C for 1.5 h. The mixture is cooled down to RT, diluted in EtOAc (100 mL) and filtered on Celpure^{®} P65. The solids are washed with EtOAc. The filtrate is washed with water and brine (100 mL + 25 mL), then brine and concentrated under vacuum. The residue is purified by flash chromatography (eluting with heptane/EtOAc, 80/20 + 0.5% AcOH) to afford **Int 22.**

### 2.7. Int 33

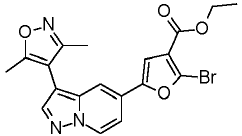

To a solution of **Cpd 93** (473 mg, 1.35 mmol, 1 eq.) in DMF at 0 °C is added NBS (252 mg, 1.41 mmol, 1.05 eq.). The reaction mixture is heated at 50 °C for 1 h, then water is added and the precipitate is filtered, washed with water and dried under vacuum to afford **Int 33.**

### 2.8. Int 36

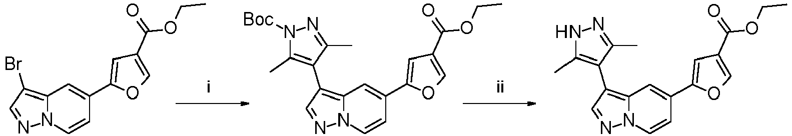

### 2.8.1. Step i: tert-butyl 4-[5-(4-ethoxycarbonyl-2-furyl)pyrazolo[1,5-a]pyridin-3-yl]-3,5-dimethyl-pyrazole-1-carboxylate

**Int 8** is reacted with 1-Boc-3,5-dimethylpyrazole-4-boronic acid pinacol ester (CAS# 1073354-70-7) following general method E.

### 2.8.2. Step ii: Int 36

To a solution of the crude pyrazole from step i in DCM (50 mL) is added TFA (10 mL). The reaction mixture is stirred at RT for 2 h. The reaction mixture is concentrated *in vacuo.* Brine is added to the residue and the aq. solution is extracted with DCM. The two phases are separated, the organic phase is dried over Na₂SO₄ and filtrated. The filtrate is concentrated and the residue is purified by flash chromatography (elution with a DCM / MeCN gradient 95/5 to 50/50). The obtained solid is triturated in iPr₂O and filtrated to afford **Int 36.**

### 2.9. Int 39

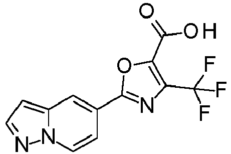

To a solution of ethyl 2-bromo-4-(trifluoromethyl)oxazole-5-carboxylate (CAS# 1227934-69-1; 0.500 g, 2.137 mmol, 1 eq.) in a degassed mixture of dioxane/water 4/1 (10 mL) are added **Int 22** (0.574 g, 2.35 mmol, 1.1 eq.), Cs₂CO₃ (2.09 g, 6.41 mmol, 3 eq.) and XPhos Pd G3 (0.090 g, 0.107 mmol, 0.05 eq.). The reaction mixture is stirred at 110 °C for 4 h, then filtered on Celite^{®}. Solids are washed with EtOAc. The filtrate is concentrated. The residue is triturated in DCM, the solid is filtered, washed with DCM and dried to afford **Int 39.**

### 2.10. Int 53

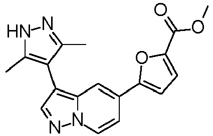

To a solution of **Int 50** (60 mg, 0.176 mmol, 1 eq.) in EtOH (1.2 mL) are added DIPEA (72 µL, 54 mg, 2.2 eq.) and a tetrahydropyran-4-ylhydrazine dihydrochloride (CAS# 1187974-47-5)/hydrazine mixture (37 mg). The reaction mixture is heated at 60 °C for 1 h, then concentrated. The residue is purified by flash chromatography, eluting with a heptane/EtOAc 0 to 100% gradient, to afford **Int 53.**

### 2.11. Int 58

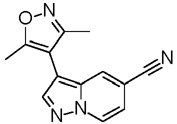

To a solution of 3-bromopyrazolo[1,5-a]pyridine-5-carbonitrile (CAS# 1427501-82-3; 144 mg, 0.649 mmol, 1 eq.) in a degassed dioxane/water mixture (4/1; 2 mL) are added 3,5-dimethyl-4-(4,4,5,5-tetramethyl-1,3,2-dioxaborolan-2-yl)isoxazole (CAS# 832114-00-8; 174 mg, 0.778 mmol, 2 eq.), Cs₂CO₃ (634 mg, 1.95 mmol, 3 eq.) and XPhos Pd G3 (27 mg, 0.032 mmol, 0.05 eq.). The reaction mixture is stirred at 100 °C for 1.5 h, and then concentrated. The residue is diluted in EtOAc, washed with water and brine. The organic phase is dried over MgSO₄, filtered and the filtrate is concentrated. The residue is purified by flash chromatography on silica gel (eluting with a heptane/EtOAc gradient 0 to 40%) to afford **Int 58.**

### 2.12. Int 67

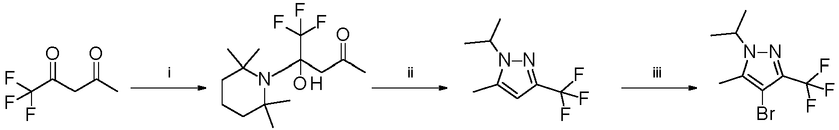

### 2.12.1. Step i: Int 69

To a solution of 1,1,1-trifluoro-2,4-pentanedione (CAS# 367-57-7; 0.75 mL, 6 mmol, 1 eq.) in heptane (10 mL) at 0 °C under N₂, is added slowly 2,2,6,6-tetramethylpiperidine (CAS# 768-66-1; 1 mL, 6 mmol, 1 eq.). The reaction mixture is stirred at 0 °C for 30 min then filtered to afford **Int 69.**

### 2.12.2. Step ii: Int 68

To a suspension of isopropylhydrazine hydrochloride (CAS# 16726-41-3; 210 mg, 1.9 mmol, 1 eq.) in dry DCM (2 mL) is added DIPEA (331 µL, 1.9 mmol, 1 eq.). The reaction mixture is stirred 10 min at RT. To a cooled suspension of **Int 69** (560 mg, 1.9 mmol, 1 eq.) in dry THF (4 mL) is slowly added the above solution of hydrazine. The reaction mixture is then stirred 12 h from 0 °C to RT. A 2N solution of HCl in water (2 mL) is added and the reaction mixture is stirred 30 min at RT. DCM and water are added. The organic phase is recovered and washed with a saturated solution of NaHCO₃, dried over Na₂SO₄, filtered and concentrated *in vacuo* to afford the **Int 68** mixture.

### 2.12.3. Step iii: Int 67

To a solution of the **Int 68** mixture (268 mg, 1.39 mmol, 1 eq.) in DMF (5 mL) is added NBS (261 mg, 1.46 mmol, 1.05 eq.) at 0 °C. The reaction mixture is stirred at 0 °C for 1 h and then heated to 80 °C for 2 h. Water is added and the aq. solution is extracted with EtOAc. The organic layer is dried over Na₂SO₄, filtered and concentrated *in vacuo.* The residue is purified by flash chromatography on silica gel (eluting with heptane/EtOAc 90/10) to afford **Int 67** as the fast eluting compound.

### 2.13. Int 78

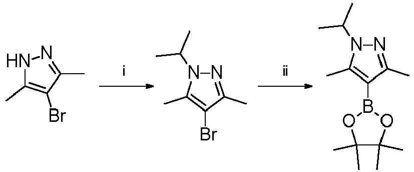

### 2.13.1. Step i: 4-bromo-1-isopropyl-3,5-dimethyl pyrazole

4-Bromo-3,5-dimethyl-1H-pyrazole (CAS# 3398-16-1; 750 g, 4.156 mol, 1.0 eq.) and KOH (549 g, 8.32 mol, 2.0 eq.) are added to MeCN (3750 mL). The reaction mixture is stirred at RT for 5 min before 2-bromopropane (780 mL, 8.31 mol, 2.0 eq.) is added in one portion. The reaction mixture is stirred at 55 °C for 4 h and cooled to RT. MTBE (2 L) and water (2 L) are added. The organic phase is washed with 20% NaCl solution and concentrated to remove 4.7 L of solvent. The solution is then dried over Na₂SO₄, filtered and concentrated to dryness to afford the desired compound.

### 2.13.2. Step ii: Int 78

4-Bromo-1-isopropyl-3,5-dimethyl-pyrazole (50.0 g, 230 mmol, 1.00 eq.), pinacolborane (44 mL, 294 mmol, 1.30 eq.), Pd₂(dba)₃ (CAS# 51364-51-3; 520 mg, 0.57 mmol, 0.0025 eq.), XPhos (CAS# 564483-18-7; 550 mg, 1.13 mmol, 0.005 eq.) and TEA (60 mL, 428 mmol, 1.90 eq.) are added to EtOAc (300 mL). The reaction mixture is then stirred at 80 °C for 1 h and then cooled to RT. The reaction mixture is filtered on Whatman^{®} grade 50 filter paper, the cake is washed with EtOAc (150 mL) and water (300 mL). The organic phase is extracted and concentrated to dryness to afford **Int 78.**

### 2.14. Cpd 7

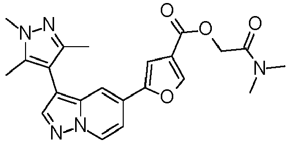

To a solution of Cpd 2 (80 mg, 0.24 mmol, 1 eq.) in DMF (0.5 mL) are added a catalytic amount of NaI, TEA (39 µL, 0.284 mmol, 1.2 eq.) and 2-chloro-N,N-dimethyl-acetamide (CAS# 2675-89-0, 29 µL, 0.284 mmol, 1.2 eq.). The reaction mixture is stirred at RT for 3 h. Water is added, followed by a 2N Na₂S₂O₃ solution. The precipitate is filtered. The solid is triturated in a saturated solution of NaHCO₃, then filtered and dried *in vacuo* to afford **Cpd 7.**

### 2.15. Cpd 11

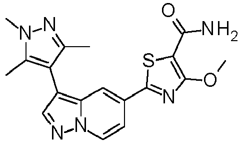

To a solution of **Cpd** 8 (30 mg, 0.078 mmol, 1 eq.) are added HATU (38 mg, 0.1 mmol, 1.2 eq.), DIPEA (82 µL, 0.468 mmol, 6 eq.) and NH₄Cl (21 mg, 0.39 mmol, 5 eq.). The reaction mixture is stirred at RT for 12 h. Water is added and the precipitate is filtered. The solid is dried *in vacuo* to afford **Cpd 11.**

### 2.16. Cpd 13

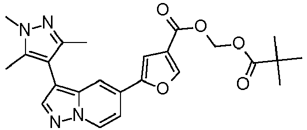

To a solution of **Cpd 2** (80 mg, 0.24 mmol, 1 eq.) in DMF (0.5 mL) are added a catalytic amount of NaI, TEA (52 µL, 0.378 mmol, 1.6 eq.) and chloromethyl 2,2-dimethylpropanoate (CAS# 18997-19-8; 54 µL, 0.378 mmol, 1.6 eq.). The reaction mixture is stirred at RT for 3 h. Water is added, followed by a 2N Na₂S₂O₃ solution, then a saturated solution of NaHCO₃. The precipitate is filtered and dried in vacuum to afford **Cpd 13.**

### 2.17. Cpd 21

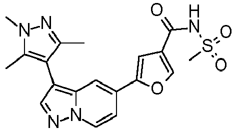

A RBF is charged with **Cpd 2** (30 mg, 0.089 mmol, 1.0 eq.), methanesulfonamide (CAS# 3144-09-0; 17 mg, 0.178 mmol, 2.0 eq.) and DMAP (12 mg, 0.098 mmol, 1.1 eq.). Anhydrous DCM (0.75 mL) is added and the reaction mixture is stirred for 15 min at RT. EDCI (23 mg, 0.115 mmol, 1.3 eq.) is then added and the reaction mixture is stirred 12 h at RT. The reaction mixture is quenched with a solution of HCl 1M (0.1 mL, 1.1 eq.) and diluted in DCM. The two phases are separated and the aq. phase is extracted with DCM. The combined organic phases are dried over MgSO₄, filtered and concentrated *in vacuo.* The residue is suspended in DCM and the solid is filtered and dried to afford **Cpd 21.**

### 2.18. Cpd 22

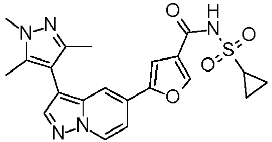

A RBF is charged with **Cpd 2** (30 mg, 0.089 mmol, 1.0 eq.), cyclopropane sulfonamide (CAS# 154350-29-5; 25 mg, 0.178 mmol, 2.0 eq.) and DMAP (12 mg, 0.098 mmol, 1.1 eq.). Anhydrous DCM (0.75 mL) is added and the reaction mixture is stirred at RT for 15 min. EDCI (28 mg, 0.140 mmol, 1.6 eq.) is then added and the reaction mixture is stirred at RT for 48 h. The reaction mixture is then quenched with a solution of HCl 1M (0.1 mL, 1.1 eq.). The precipitate is filtered and the solid is washed with DCM, then with water. The solid is dried *in vacuo* to afford **Cpd 22.**

### 2.19. Cpd 36

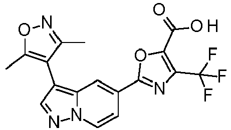

**Cpd 36** is prepared from **Int 37** according to general method E. Saponification occurs during the Suzuki reaction. The reaction mixture is filtered on Celite^{®}, eluting with EtOAc. The filtrate is concentrated to dryness. The residue is dissolved in DCM and purified on a Biotage^{®} ISOLUTE^{®} PE-AX column, eluting with a DCM/MeCN 1/1 + 5% AcOH mixture to afford **Cpd 36.**

### 2.20. Cpd 61

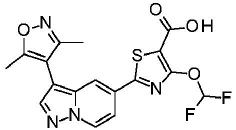

To a solution of **Int 56** (40 mg, 0.104 mmol, 1 eq.) in DMF (2 mL) are added ethyl 2-chloro-2,2-difluoro-acetate (CAS# 383-62-0; 16 µL, 0.125 mmol, 1.2 eq.) and Cs₂CO₃ (169 mg, 0.52 mmol, 5 eq.).The reaction mixture is heated at 60 °C for 12 h, and then evaporated to dryness. The residue is dissolved in DMSO, the solid is filtered and the filtrate is purified by HPLC preparative to afford **Cpd 61.**

### 2.21. Cpd 102

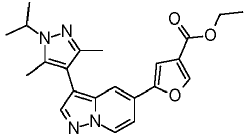

**Int 8** (14.065 g, 41.96 mmol, 1.00 eq.), **Int 78** (16.265 g, 58.49 mmol, 1.39 eq.), Pd(OAc)₂ (140 mg, 0.63 mmol, 0.015 eq.), Xantphos (726 mg, 1.25 mmol, 0.03 eq.) and K₃PO₄ (18.65 g, 85.25 mmol, 2.03 eq.) are added to a dioxane/water (55 mL/14 mL) mixture. The reaction mixture is refluxed for 18 h and then cooled to RT. EtOAc (28 mL) and water (28 mL) are added. The solution is filtered on a pad of Celite^{®}, the cake being washed with water. The solution is diluted with EtOAc (150 mL) and a 20% NaCl aq. solution (150 mL). The organic phase is concentrated. The crude residue is dissolved in iPrzO (50 mL) and stirred at RT for 1 h. The suspension is filtered and the solid is dried to afford **Cpd 102.**

### 2.22. Cpd 121

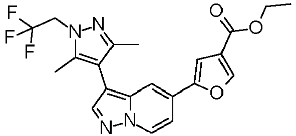

In a RBF is charged **Int 19** (22 g, 62.08 mmol, 1 eq.). EtOH (220 mL) is added at RT followed by 2,2,2-trifluoroethylhydrazine (CAS# 5042-30-8; 10.9 mL, 86.91 mmol, 1.4 eq.). The reaction mixture is stirred at reflux for 2.5 h, then cooled to RT and concentrated. The residue is taken up in DCM, washed with water, then with brine, dried over MgSO₄, filtered and concentrated *in vacuo.* The residue is purified by flash chromatography on silica gel (dry loading, eluting with a DCM : DCM/MeCN + 0.5% MeOH gradient). The solid obtained is triturated in iPrzO, stirred at RT for 1 h, then filtered, washed and dried to afford **Cpd 121.**

**Table II. Intermediates used towards the compounds of the invention**

| **Int#** | **Structure** | **Name** | **SM** | **Mtd** | **MW** | **MS Mes'd** |
|---|---|---|---|---|---|---|
| 1 | 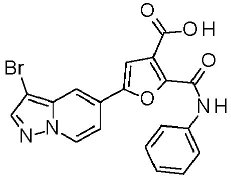 | 5-(3-bromopyrazolo [1,5-a]pyridin-5-yl)-2-(phenylcarbamoyl)furan-3-carboxylic acid | Int 2 | F+A | 426.2 | 426.4 + 428.3 |
| 2 | 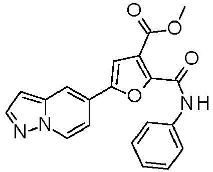 | methyl 2-(phenylcarbamoyl)-5- pyrazolo[1,5-a]pyridin-5-yl-furan-3-carboxylate | Int 3 | J | 361.4 | 362.7 |
| 3 | 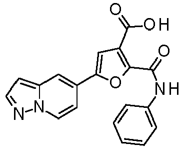 | 2-(phenylcarbamoyl)-5-pyrazolo[1,5-a]pyridin-5-yl-furan-3-carboxylic acid | Int 4 | Ex. 2.1.4 | 347.3 | 348.5 |
| 4 | 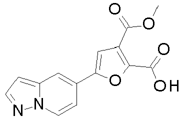 | 3-methoxycarbonyl-5-pyrazolo[1,5-a]pyridin-5-yl-furan-2-carboxylic acid | Int 5 | Ex. 2.1.3 | 286.2 | 287.5 |
| 5 | 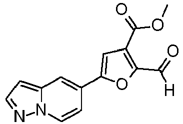 | methyl 2-formyl-5-pyrazolo[1,5-a]pyridin-5-yl-furan-3-carboxylate | Int 6 + Int 22 | Ex. 2.1.2 | 270.2 | 271.4 |
| 6 | 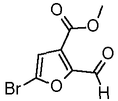 | methyl 5-bromo-2-formyl-furan-3-carboxylate | CAS# 58832-36-3 | Ex. 2.1.1 | 233.0 | 233.1 + 235.1 |
| 7 | 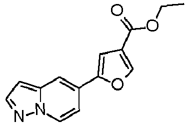 | ethyl 5-pyrazolo[1,5-a]pyridin-5-ylfuran-3- carboxylate | CAS# 614-98-2 + CAS# 1060812-84-1 | Ex. 2.2 | 256.3 | 257.1 |
| 8 | 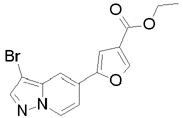 | ethyl5-(3-bromopyrazolo[1,5-a]pyridin-5-yl)furan-3- carboxylate | Int 7 | Ex. 2.3 | 335.2 | 335.0 + 336.9 |
| 9 | 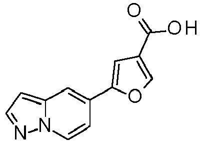 | 5-pyrazolo[1,5-a]pyridin-5-ylfuran-3-carboxylic acid | CAS# 1073354-94-5 + CAS# 1060812-84-1 | Ex. 2.4 | 228.2 | NA |
| 10 | 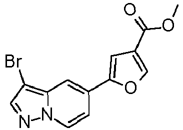 | methyl 5-(3-bromopyrazolo[1,5-a]pyridin-5-yl)furan-3-carboxylate | Int 11 | J | 321.1 | 321.1 + 323.1 |
| 11 | 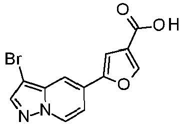 | 5-(3-bromopyrazolo[1,5-a]pyridin-5-yl)furan-3-carboxylic acid | Int 9 | A | 307.1 | 307.3 + 309.2 |
| 12 | 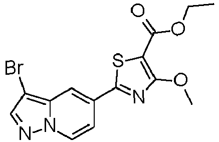 | ethyl 2-(3-bromopyrazolo [1,5-a]pyridin-5-yl)-4-methoxy-thiazole-5-carboxylate | Int 13 | A | 382.2 | 382.4 + 384.3 |
| 13 | 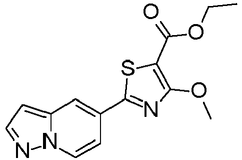 | ethyl 4-methoxy-2-[3-(1,3,5-trimethylpyrazol-4-yl)pyrazolo[1,5-a]pyridin-5-yl]thiazole-5-carboxylate | Int 14 | H | 303.3 | 304.7 |
| 14 | 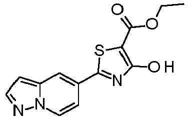 | ethyl 4-hydroxy-2-pyrazolo[1,5-a]pyridin-5-yl-thiazole-5-carboxylate | Int 15 + CAS# 685-87-0 | G | 289.3 | 290.5 |
| 15 | 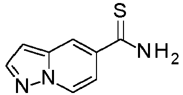 | pyrazolo[1,5-a]pyridine-5-carbothioamide | CAS# 1352903-96-8 | N | 177.2 | 178.3 |
| 16 | 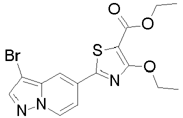 | ethyl 2-(3-bromopyrazolo[1,5-a]pyridin-5-yl)-4-ethoxy-thiazole-5-carboxylate | Int 17 | A | 396.3 | 396.4 + 398.3 |
| 17 | 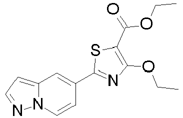 | ethyl 4-ethoxy-2-pyrazolo[1,5-a]pyridin-5-yl-thiazole-5-carboxylate | Int 14 | H | 317.4 | 318.8 |
| 18 | 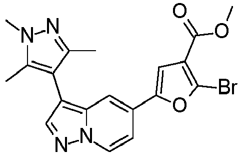 | methyl 2-bromo-5-[3-(1,3,5-trimethylpyrazol-4-yl)pyrazolo[1,5-a]pyridin-5-yl]furan-3-carboxylate | Cpd 5 | Ex. 2.3 | 429.3 | 429.1 + 431.1 |
| 19 | 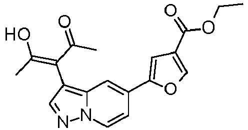 | ethyl 5-[3-[(Z)-1-acetyl-2-hydroxy-prop-1-enyl]pyrazolo[1,5-a]pyridin-5-yl]furan-3- carboxylate | Cpd 93 | C | 354.4 | 355.7 |
| 20 | 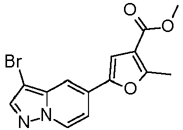 | methyl 5-(3-bromopyrazolo[1,5-a]pyridin-5-yl)-2-methylfuran-3-carboxylate | Int 21 | A | 335.2 | NA |
| 21 | 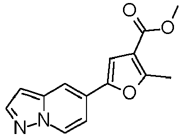 | methyl 2-methyl-5-pyrazolo[1,5-a]pyridin-5-yl-furan-3-carboxylate | Int 22 + CAS# 345891-28-3 | K | 256.3 | 257.2 |
| 22 | 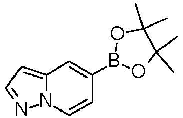 | 5-(4,4,5,5-tetramethyl-1,3,2-dioxaborolan-2-yl)pyrazolo[1,5-a]pyridine | CAS# 1060812-84-1 | Ex. 2.6 | 244.1 | 245.6 |
| 23 | 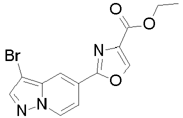 | ethyl 2-(3-bromopyrazolo[1,5-a]pyridin-5-yl)oxazole-4-carboxylate | Int 24 | A | 336.1 | 336.5 + 338.5 |
| 24 | 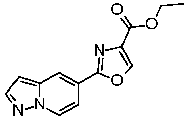 | ethyl 2-pyrazolo[1,5-a]pyridin-5-yloxazole-4-carboxylate | Int 22 + CAS# 460081-18-9 | K | 257.2 | 258.6 |
| 25 | 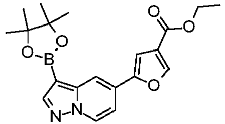 | ethyl 5-[3-(4,4,5,5-tetramethyl-1,3,2-dioxa borolan-2-yl) pyrazolo[1,5-a]pyridin-5-yl]furan-3-carboxylate | Int 8 + CAS# 73183-34-3 | O | 382.2 | 383.2 |
| 26 | 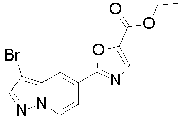 | ethyl 2-(3-bromopyrazolo[1,5-a]pyridin-5-yl)oxazole-5-carboxylate | Int 27 | A | 336.1 | 336.6 + 338.5 |
| 27 | 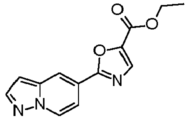 | ethyl 2-pyrazolo[1,5-a]pyridin-5-yloxazole-5-carboxylate | Int 22 + CAS# 862599-47-1 | K | 257.2 | 258.6 |
| 28 | 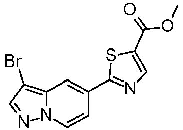 | methyl 2-(3-bromopyrazolo[1,5-a]pyridin-5-yl)thiazole-5-carboxylate | Int 29 | A | 338.2 | 338.0 + 340.0 |
| 29 | 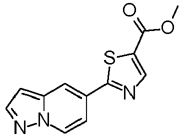 | methyl 2-pyrazolo[1,5-a]pyridin-5-ylthiazole-5-carboxylate | Int 22 + CAS# 54045-74-8 | K | 259.3 | 260.1 |
| 30 | 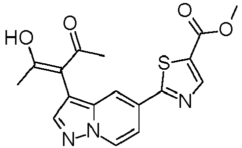 | methyl 2-[3-[(Z)-1-acetyl-2-hydroxy-prop-1-enyl]pyrazolo[1,5-a]pyridin-5-yl]thiazole-5-carboxylate | Cpd 107 | D | 357.4 | 358.1 |
| 31 | 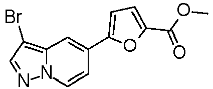 | methyl 5-(3-bromopyrazolo[1,5-a]pyridin-5-yl)furan-2-carboxylate | Int 32 | A | 321.1 | 321.0 + 323.1 |
| 32 | 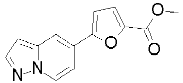 | methyl 5-pyrazolo[1,5-a]pyridin-5-ylfuran-2-carboxylate | CAS# 1060812-84-1 + CAS# 876189-20-7 | K | 242.2 | 243.1 |
| 33 | 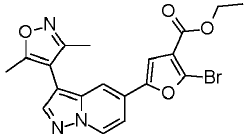 | ethyl 2-bromo-5-[3-(3,5-dimethylisoxazol-4-yl)pyrazolo[1,5-a]pyridin-5-yl]furan-3-carboxylate | Cpd 93 | Ex. 2.7 | 430.3 | 430.6 + 432.5 |
| 34 | 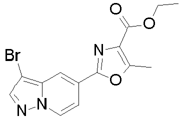 | ethyl 2-(3-bromopyrazolo[1,5-a]pyridin-5-yl)-5-methyloxazole-4-carboxylate | Int 35 | A | 350.2 | 350.1 + 352.1 |
| 35 | 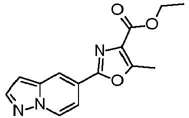 | ethyl 5-methyl-2-pyrazolo[1,5-a]pyridin-5-yl-oxazole-4-carboxylate | Int 22 + CAS# 1187582-59-7 | K | 271.3 | 272.1 |
| 36 | 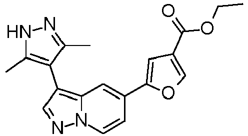 | ethyl 5-[3-(3,5-dimethyl-1H-pyrazol-4-yl)pyrazolo[1,5-a]pyridin-5-yl]furan-3-carboxylate | Int 8 + CAS# 1073354-70-7 | Ex. 2.8 | 350.4 | 351.7 |
| 37 | 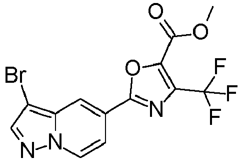 | methyl 2-(3-bromopyrazolo[1,5-a]pyridin-5-yl)-4-(trifluoromethyl)oxazole-5-carboxylate | Int 38 | A | 390.1 | 391.4 |
| 38 | 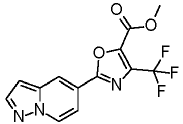 | methyl 2-pyrazolo[1,5-a]pyridin-5-yl-4-(trifluoromethyl)oxazole-5-carboxylate | Int 39 | J | 311.2 | NA |
| 39 | 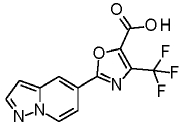 | 2-pyrazolo[1,5-a]pyridin-5-yl-4-(trifluoromethyl)oxazole-5-carboxylic acid | Int 22 + CAS# 1227934-69-1 | Ex. 2.9 | 297.2 | 298.1 |
| 40 | 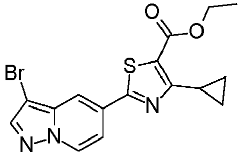 | ethyl 2-(3-bromopyrazolo[1,5-a]pyridin-5-yl)-4-cyclopropyl-thiazole-5- carboxylate | Int 41 | A | 392.3 | 392.6 + 394.5 |
| 41 | 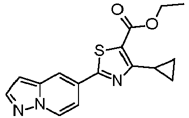 | ethyl 4-cyclopropyl-2-pyrazolo[1,5-a]pyridin-5-yl-thiazole-5-carboxylate | Int 15 + Int 42 | G | 313.4 | 314.6 |
| 42 | 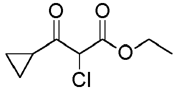 | ethyl 2-chloro-3-cyclopropyl-3-oxo-propanoate | CAS# 24922-02-9 | P | 190.6 | NA |
| 43 | 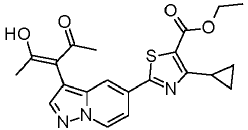 | ethyl 2-[3-[(Z)-1-acetyl-2-hydroxy-prop-1-enyl]pyrazolo[1,5-a]pyridin-5-yl]-4-cyclopropyl-thiazole-5- carboxylate | Cpd 118 | D | 411.5 | 412.8 |
| 44 | 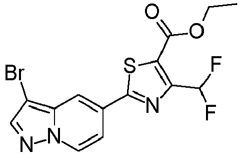 | ethyl 2-(3-bromopyrazolo [1,5-a]pyridin-5-yl)-4-(difluoromethyl)thiazole-5-carboxylate | Int 45 | A | 402.2 | 402.5 + 404.5 |
| 45 | 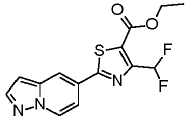 | ethyl 4-(difluoromethyl)-2-pyrazolo[1,5-a]pyridin-5-yl-thiazole-5-carboxylate | Int 15 + Int 46 | G | 323.3 | 324.2 |
| 46 | 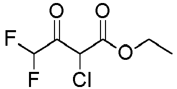 | ethyl 2-chloro-4,4-difluoro-3-oxo-butanoate | CAS# 352-24-9 | P | 200.6 | NA |
| 47 | 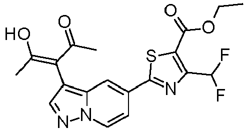 | ethyl 2-[3-[(Z)-1-acetyl-2-hydroxy-prop-1-enyl]pyrazolo[1,5-a]pyridin-5-yl]-4-(difluoromethyl)thiazole-5-carboxylate | Cpd 125 | D | 421.4 | 422.2 |
| 48 | 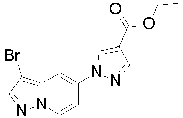 | ethyl 1-(3-bromopyrazolo [1,5-a]pyridin-5-yl)pyrazole-4-carboxylate | Int 49 | A | 335.2 | 335.5 + 337.5 |
| 49 | 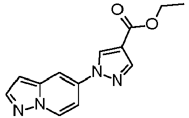 | ethyl 1-pyrazolo[1,5-a]pyridin-5-ylpyrazole-4-carboxylate | CAS# 1060812-84-1 + CAS# 37622-90-5 | I | 256.3 | 257.6 |
| 50 | 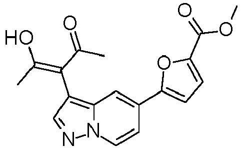 | methyl 5-[3-[(Z)-1-acetyl-2-hydroxy-prop-1-enyl]pyrazolo[1,5-a]pyridin-5-yl]furan-2-carboxylate | Cpd 113 | D | 340.3 | 341.7 |
| 51 | 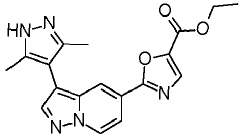 | ethyl 2-[3-(3,5-dimethyl-1H-pyrazol-4-yl)pyrazolo[1,5-a]pyridin-5-yl]oxazole-5-carboxylate | Int 26 + CAS# 1073354-70-7 | E | 351.4 | 352.7 |
| 52 | 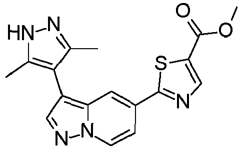 | methyl 2-[3-(3,5-dimethyl-1H-pyrazol-4-yl)pyrazolo[1,5-a]pyridin-5-yl]thiazole-5-carboxylate | Int 28 + CAS# 1073354-70-7 | E | 353.4 | 354.7 |
| 53 | 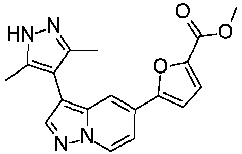 | methyl 5-[3-(3,5-dimethyl-1H-pyrazol-4-yl)pyrazolo[1,5-a]pyridin-5-yl]furan-2-carboxylate | Int 50 + CAS# 1187974-47-5 | Ex. 2.10 | 336.3 | 337.6 |
| 54 | 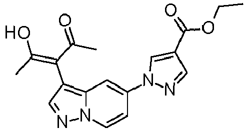 | ethyl 1-[3-[(Z)-1-acetyl-2-hydroxy-prop-1-enyl]pyrazolo[1,5-a]pyridin-5-yl]pyrazole-4- carboxylate | Cpd 127 | D | 354.4 | 355.5 |
| 55 | 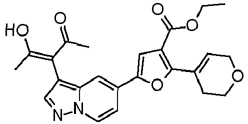 | ethyl 5-[3-[(Z)-1-acetyl-2-hydroxy-prop-1-enyl]pyrazolo[1,5-a]pyridin-5-yl]-2-(3,6-dihydro-2H-pyran-4-yl)furan-3-carboxylate | Cpd 122 | D | 436.5 | 437.6 |
| 56 | 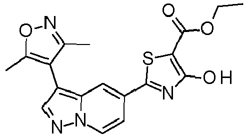 | ethyl 2-[3-(3,5-dimethylisoxazol-4-yl)pyrazolo[1,5-a]pyridin-5-yl]-4-hydroxy-thiazole-5-carboxylate | Int 57 + CAS# 685-87-0 | G | 384.4 | 385.5 |
| 57 | 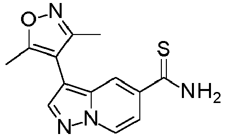 | 3-(3,5-dimethylisoxazol-4-yl)pyrazolo[1,5-a]pyridine-5-carbothioamide | Int 58 | N | 272.3 | 273.2 |
| 58 | 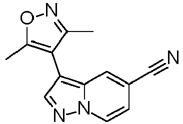 | 3-(3,5-dimethylisoxazol-4-yl)pyrazolo[1,5-a]pyridine-5-carbonitrile | CAS# 1427501-82-3 + CAS# 832114-00-8 | Ex. 2.11 | 238.2 | 239.1 |
| 59 | 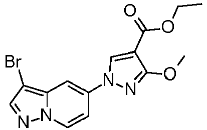 | ethyl 1-(3-bromopyrazolo [1,5-a]pyridin-5-yl)-3-methoxy-pyrazole-4-carboxylate | Int 60 | A | 365.2 | 365.5 + 367.4 |
| 60 | 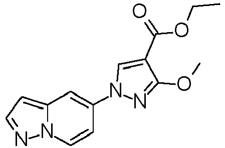 | ethyl 3-methoxy-1-pyrazolo[1,5-a]pyridin-5-yl-pyrazole-4-carboxylate | CAS# 1060812-84-1 + CAS# 478968-48-8 | I | 286.3 | 287.7 |
| 61 | 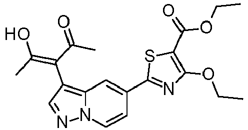 | ethyl 2-[3-[(Z)-1-acetyl-2-hydroxy-prop-1-enyl]pyrazolo[1,5-a]pyridin-5-yl]-4-ethoxy-thiazole-5-carboxylate | Cpd 101 | D | 415.5 | 416.3 |
| 62 | 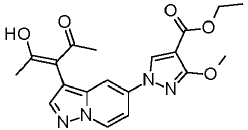 | ethyl 1-[3-[(Z)-1-acetyl-2-hydroxy-prop-1-enyl]pyrazolo[1,5-a]pyridin-5-yl]-3-methoxy-pyrazole-4-carboxylate | Cpd 143 | D | 384.4 | 385.5 |
| 63 | 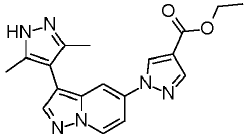 | ethyl 1-[3-(3,5-dimethyl-1H-pyrazol-4-yl)pyrazolo[1,5-a]pyridin-5-yl]pyrazole-4-carboxylate | Int 54 + CAS# 7803-57-8 | C | 350.4 | 351.3 |
| 64 | 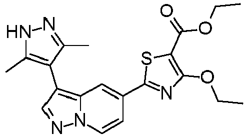 | ethyl 2-[3-(3,5-dimethyl-1H-pyrazol-4-yl)pyrazolo[1,5-a]pyridin-5-yl]-4-ethoxy-thiazole-5-carboxylate | Int 61 + CAS# 7803-57-8 | C | 411.5 | 412.6 |
| 65 | 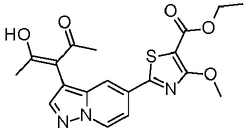 | ethyl 2-[3-[(Z)-1-acetyl-2-hydroxy-prop-1-enyl]pyrazolo[1,5-a]pyridin-5-yl]-4-methoxy-thiazole-5-carboxylate | Cpd 155 | D | 401.4 | 402.5 |
| 66 | 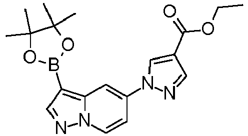 | ethyl 1-[3-(4,4,5,5-tetramethyl-1,3,2-dioxaborolan-2-yl)pyrazolo[1,5-a]pyridin-5-yl]pyrazole-4-carboxylate | Int 48 + CAS# 73183-34-3 | O | 382.2 | 383.5 |
| 67 | 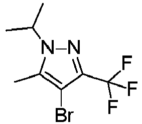 | 4-bromo-1-isopropyl-5- methyl-3-(trifluoromethyl)pyrazole | Int 68 | Ex. 2.12 | 271.1 | 271.3 + 273.1 |
| 68 | 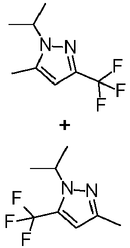 | 1-isopropyl-5-methyl-3-(trifluoromethyl)pyrazole / 1-isopropyl-3-methyl-5-(trifluoromethyl)pyrazole mixture | Int 69 + CAS# 16726-41-3 | Ex. 2.12.2 | 192.2 | 193.2 |
| 69 | 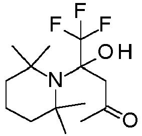 | 5,5,5-trifluoro-4-hydroxy-4-(2,2,6,6-tetramethyl-1-piperidyl)pentan-2-one | CAS# 367-57-7 + CAS# 768-66-1 | Ex. 2.12.1 | 295.3 | NA |
| 70 | 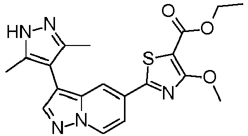 | ethyl 2-[3-(3,5-dimethyl-1H-pyrazol-4-yl)pyrazolo[1,5-a]pyridin-5-yl]-4-methoxy-thiazole-5-carboxylate | Int 65 + CAS# 7803-57-8 | C | 397.5 | 398.3 |
| 71 | 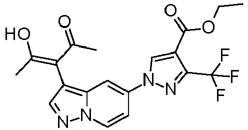 | ethyl 1-[3-[(Z)-1-acetyl-2-hydroxy-prop-1-enyl]pyrazolo[1,5-a]pyridin-5-yl]-3-(trifluoromethyl)pyrazole-4-carboxylate | Cpd 162 | D | 422.4 | 423.3 |
| 72 | 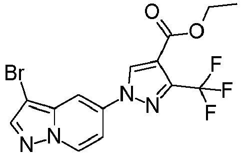 | ethyl 1-(3-bromopyrazolo [1,5-a]pyridin-5-yl)-3-(trifluoromethyl)pyrazole-4-carboxylate | Int 73 | A | 403.2 | 403.2 + 405.1 |
| 73 | 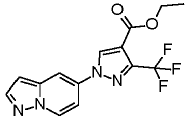 | ethyl 1-pyrazolo[1,5-a]pyridin-5-yl-3-(trifluoromethyl)pyrazole-4-carboxylate | CAS# 1060812-84-1 + CAS# 155377-19-8 | I | 324.3 | 325.3 |
| 74 | 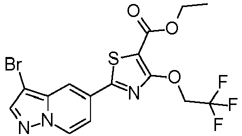 | ethyl 2-(3-bromopyrazolo[1,5-a]pyridin-5-yl)-4-(2,2,2-trifluoroethoxy)thiazole-5-carboxylate | Int 75 | A | 450.2 | 450.3 + 452.4 |
| 75 | 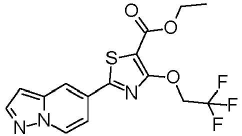 | ethyl2-pyrazolo[1,5-a]pyridin-5-yl-4-(2,2,2-trifluoroethoxy)thiazole-5-carboxylate | Int 14 + CAS# 433-06-7 | H | 371.3 | 372.2 |
| 76 | 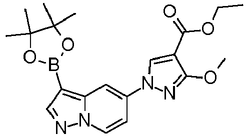 | ethyl 3-methoxy-1-[3-(4,4,5,5-tetramethyl-1,3,2-dioxaborolan-2-yl)pyrazolo[1,5-a]pyridin-5-yl]pyrazole-4-carboxylate | Int 59 | O | 412.3 | 413.4 |
| 77 | 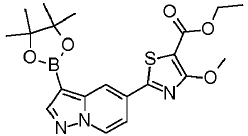 | ethyl 4-methoxy-2-[3-(4,4,5,5-tetramethyl-1,3,2-dioxaborolan-2-yl)pyrazolo[1,5-a]pyridin-5-yl]thiazole-5-carboxylate | Int 12 | O | 429.3 | 430.4 |
| 78 | 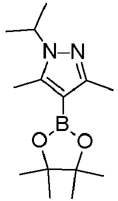 | 1-isopropyl-3,5-dimethyl-4-(4,4,5,5-tetramethyl-1,3,2-dioxaborolan-2-yl)pyrazole | CAS# 3398-16-1 | Ex. 2.13 | 264.2 | 265.2 |

| | | | | | | |
|---|---|---|---|---|---|---|
| SM = Starting Material, Mtd = Method, MS Mes'd = Mesured mass | | | | | | |

**Table III. Illustrative compounds of the invention**

| **Cpd#** | **Structure** | **Name** | **SM** | **Mtd** | **MW** | **MS Mes'd** |
|---|---|---|---|---|---|---|
| 1 | 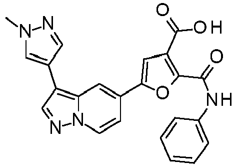 | 5-[3-(1-methylpyrazol-4-yl)pyrazolo[1,5-a]pyridin-5- yl]-2-(phenylcarbamoyl) furan-3-carboxylic acid | Int 1 + CAS# 761446-44-0 | E | 427.4 | 428.4 |
| 2 | 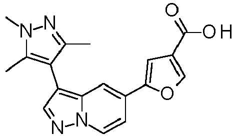 | 5-[3-(1,3,5-trimethylpyrazol-4-yl)pyrazolo[1,5-a]pyridin-5-yl]furan-3-carboxylic acid | Int 11 + CAS# 847818-62-6 | E | 336.3 | 337.5 |
| 3 | 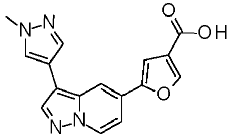 | 5-[3-(1-methylpyrazol-4-yl)pyrazolo[1,5-a]pyridin-5-yl]furan-3-carboxylic acid | Cpd 91 | F | 308.3 | 309.5 |
| 4 | 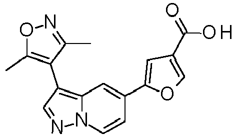 | 5-[3-(3,5-dimethylisoxazol-4-yl)pyrazolo[1,5-a]pyridin-5-yl]furan-3-carboxylic acid | Cpd 92 | F | 323.3 | 324.5 |
| 5 | 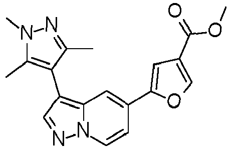 | methyl 5-[3-(1,3,5-trimethylpyrazol-4-yl)pyrazolo[1,5-a]pyridin-5-yl]furan-3-carboxylate | Int 10 + CAS# 847818-62-6 | E | 350.4 | 351.3 |
| 6 | 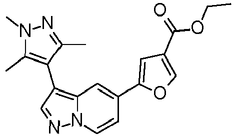 | ethyl 5-[3-(1,3,5-trimethylpyrazol-4-yl)pyrazolo[1,5-a]pyridin-5- yl]furan-3-carboxylate | Int 8 + CAS# 847818-62-6 | E | 364.4 | 365.9 |
| 7 | 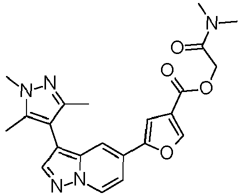 | [2-(dimethylamino)-2-oxoethyl] 5-[3-(1,3,5-trimethylpyrazol-4-yl)pyrazolo[1,5-a]pyridin-5 - yl]furan-3-carboxylate | Cpd 2 + CAS# 2675-89-0 | Ex. 2.14 | 421.4 | 422.9 |
| 8 | 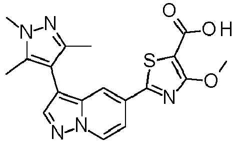 | 4-methoxy-2-[3-(1,3,5-trimethylpyrazol-4-yl)pyrazolo[1,5-a]pyridin- 5-yl]thiazole-5-carboxylic acid | Cpd 94 | F | 383.4 | 384.7 |
| 9 | 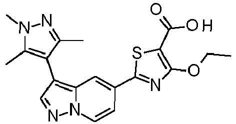 | 4-ethoxy-2-[3-(1,3,5-trimethylpyrazol-4-yl)pyrazolo[1,5-a]pyridin- 5-yl]thiazole-5-carboxylic acid | Cpd 95 | F | 397.5 | 398.8 |
| 10 | 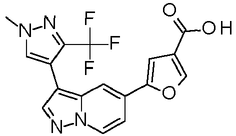 | 5-[3-[1-methyl-3-(trifluoromethyl)pyrazol-4-yl]pyrazolo[1,5-a]pyridin-5-yl]furan-3-carboxylic acid | Cpd 96 | F | 376.3 | 377.2 |
| 11 | 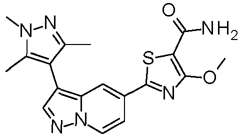 | 4-methoxy-2-[3-(1,3,5-trimethylpyrazol-4-yl)pyrazolo[1,5-a]pyridin- 5-yl]thiazole-5-carboxamide | Cpd 8 | Ex. 2.15 | 382.4 | 383.9 |
| 12 | 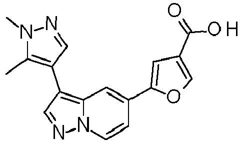 | 5-[3-(1,5-dimethylpyrazol-4-yl)pyrazolo[1,5-a]pyridin-5 - yl]furan-3-carboxylic acid | Cpd 97 | F | 322.3 | 323.2 |
| 13 | 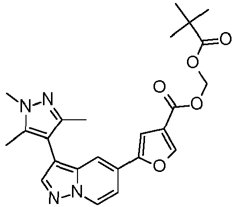 | 2,2-dimethylpropanoyloxymethyl 5-[3-(1,3,5-trimethylpyrazol-4-yl)pyrazolo[1,5-a]pyridin-5-yl]furan-3-carboxylate | Cpd 2 + CAS# 18997-19-8 | Ex. 2.16 | 450.5 | 451.3 |
| 14 | 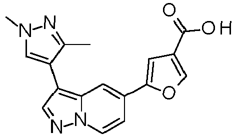 | 5-[3-(1,3-dimethylpyrazol-4-yl)pyrazolo[1,5-a]pyridin-5- yl]furan-3-carboxylic acid | Cpd 98 | F | 322.3 | 323.3 |
| 15 | 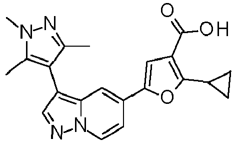 | 2-cyclopropyl-5-[3-(1,3,5- trimethylpyrazol-4-yl)pyrazolo[1,5-a]pyridin-5- yl]furan-3-carboxylic acid | Cpd 99 | F | 376.4 | 377.2 |
| 16 | 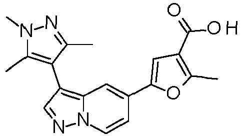 | 2-methyl-5-[3-(1,3,5-trimethylpyrazol-4-yl)pyrazolo[1,5-a]pyridin-5- yl]furan-3-carboxylic acid | Cpd 100 | F | 350.4 | 351.2 |
| 17 | 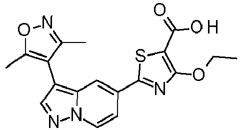 | 2-[3-(3,5-dimethylisoxazol-4-yl)pyrazolo[1,5-a]pyridin-5-yl]-4-ethoxy-thiazole-5- carboxylicacid | Cpd 101 | F | 384.4 | 385.2 |
| 18 | 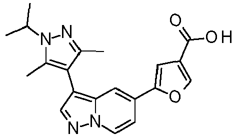 | 5-[3-(1-isopropyl-3,5-dimethyl-pyrazol-4-yl)pyrazolo[1,5-a]pyridin-5-yl]furan-3-carboxylic acid | Cpd 102 | F | 364.4 | 365.4 |
| 19 | 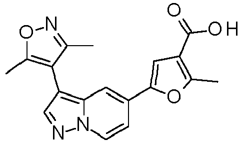 | 5-[3-(3,5-dimethylisoxazol-4-yl)pyrazolo[1,5-a]pyridin-5-yl]-2-methyl-furan-3- carboxylic acid | Cpd 103 | F | 337.3 | 338.6 |
| 20 | 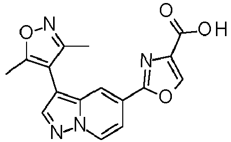 | 2-[3-(3,5-dimethylisoxazol-4-yl)pyrazolo[1,5-a]pyridin-5-yl]oxazole-4-carboxylic acid | Cpd 104 | F | 324.3 | 325.6 |
| 21 | 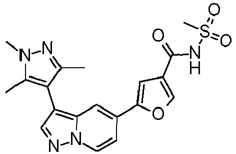 | N-methylsulfonyl-5-[3-(1,3,5-trimethylpyrazol-4-yl)pyrazolo[1,5-a]pyridin- 5-yl] furan-3-carboxamide | Cpd 2 + CAS# 3144-09-0 | Ex. 2.17 | 413.5 | 414.7 |
| 22 | 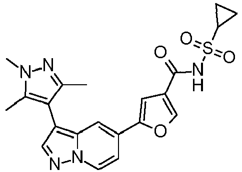 | N-cyclopropylsulfonyl-5-[3 - (1,3,5-trimethylpyrazol-4-yl)pyrazolo[1,5-a]pyridin-5-yl] furan-3-carboxamide | Cpd 2 + CAS# 154350-29-5 | Ex. 2.18 | 439.5 | 440.3 |
| 23 | 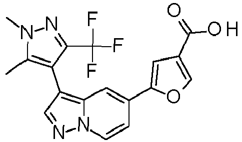 | 5-[3-[1,5-dimethyl-3-(trifluoromethyl)pyrazol-4-yl]pyrazolo[1,5-a]pyridin- 5-yl]furan-3-carboxylic acid | Cpd 105 | F | 390.3 | 391.6 |
| 24 | 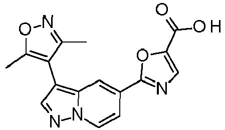 | 2-[3-(3,5-dimethylisoxazol-4-yl)pyrazolo[1,5-a]pyridin-5-yl]oxazole-5-carboxylic acid | Cpd 106 | F | 324.3 | 325.2 |
| 25 | 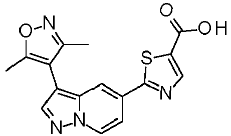 | 2-[3-(3,5-dimethylisoxazol-4-yl)pyrazolo[1,5-a]pyridin-5-yl]thiazole-5-carboxylic acid | Cpd 107 | F | 340.4 | 341.6 |
| 26 | 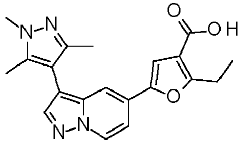 | 2-ethyl-5-[3-(1,3,5-trimethylpyrazol-4-yl)pyrazolo[1,5-a]pyridin-5- yl]furan-3-carboxylic acid | Cpd 108 | F | 364.4 | 365.2 |
| 27 | 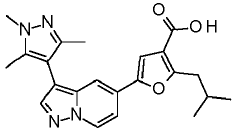 | 2-isobutyl-5-[3-(1,3,5-trimethylpyrazol-4-yl)pyrazolo[1,5-a]pyridin-5- yl]furan-3-carboxylic acid | Cpd 109 | F | 392.5 | 393.3 |
| 28 | 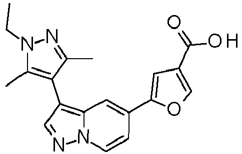 | 5-[3-(1-ethyl-3,5-dimethyl-pyrazol-4-yl)pyrazolo[1,5- a]pyridin-5-yl]furan-3-carboxylic acid | Cpd 110 | F | 350.4 | 351.2 |
| 29 | 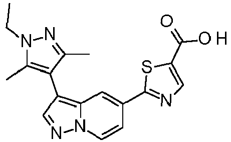 | 2-[3-(1-ethyl-3,5-dimethyl-pyrazol-4-yl)pyrazolo[1,5-a]pyridin-5-yl]thiazole-5- carboxylic acid | Cpd 111 | F | 367.4 | 368.2 |
| 30 | 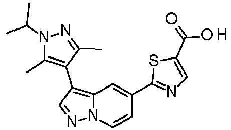 | 2-[3-(1-isopropyl-3,5-dimethyl-pyrazol-4-yl)pyrazolo[1,5-a]pyridin-5-yl]thiazole-5-carboxylic acid | Cpd 112 | F | 381.5 | 382.2 |
| 31 | 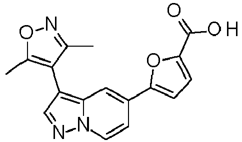 | 5-[3-(3,5-dimethylisoxazol-4-yl)pyrazolo[1,5-a]pyridin-5-yl]furan-2-carboxylic acid | Cpd 113 | F | 323.3 | 324.6 |
| 32 | 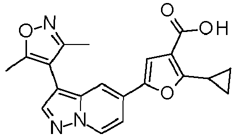 | 2-cyclopropyl-5-[3-(3,5-dimethylisoxazol-4-yl)pyrazolo[1,5-a]pyridin- 5 - yl]furan-3-carboxylic acid | Cpd 114 | F | 363.4 | 364.7 |
| 33 | 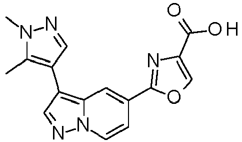 | 2-[3-(1,5-dimethylpyrazol-4-yl)pyrazolo[1,5-a]pyridin-5- yl]oxazole-4-carboxylic acid | Cpd 115 | F | 323.3 | 324.2 |
| 34 | 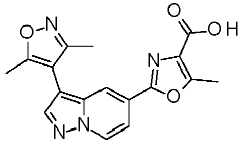 | 2-[3-(3,5-dimethylisoxazol-4-yl)pyrazolo[1,5-a]pyridin-5-yl]-5-methyl-oxazole-4-carboxylic acid | Cpd 116 | F | 338.3 | 339.6 |
| 35 | 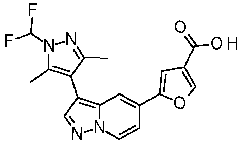 | 5-[3-[1-(difluoromethyl)-3,5-dimethyl-pyrazol-4-yl]pyrazolo[1,5-a]pyridin- 5-yl]furan-3-carboxylic acid | Cpd 117 | F | 372.3 | 373.7 |
| 36 | 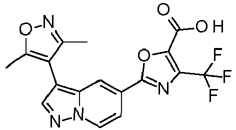 | 2-[3-(3,5-dimethylisoxazol-4-yl)pyrazolo[1,5-a]pyridin-5-yl]-4-(trifluoromethyl) oxazole-5-carboxylic acid | Int 37 + CAS# 832114-00-8 | Ex. 2.19 | 392.3 | 393.7 |
| 37 | 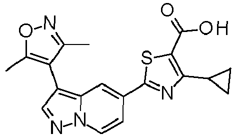 | 4-cyclopropyl-2-[3-(3,5-dimethylisoxazol-4-yl)pyrazolo[1,5-a]pyridin- 5-yl]thiazole-5-carboxylic acid | Cpd 118 | F | 380.4 | 381.7 |
| 38 | 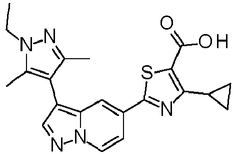 | 4-cyclopropyl-2-[3-(1-ethyl-3,5-dimethyl-pyrazol-4-yl)pyrazolo[1,5-a]pyridin-5-yl]thiazole-5-carboxylic acid | Cpd 119 | F | 407.5 | 408.5 |
| 39 | 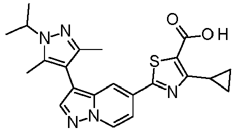 | 4-cyclopropyl-2-[3-(1-isopropyl-3,5-dimethyl-pyrazol-4-yl)pyrazolo[1,5-a]pyridin-5-yl]thiazole-5-carboxylic acid | Cpd 120 | F | 421.5 | 422.5 |
| 40 | 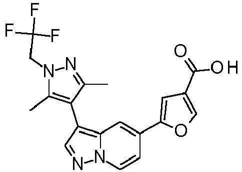 | 5-[3-[3,5-dimethyl-1-(2,2,2-trifluoroethyl)pyrazol-4-yl]pyrazolo[1,5-a]pyridin- 5-yl]furan-3-carboxylic acid | Cpd 121 | F | 404.3 | 405.7 |
| 41 | 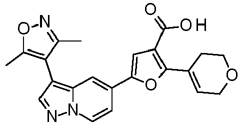 | 2-(3,6-dihydro-2H-pyran-4-yl)-5-[3-(3,5-dimethyl isoxazol-4-yl)pyrazolo[1,5-a] pyridin-5-yl]furan-3-carboxylic acid | Cpd 122 | F | 405.4 | 406.7 |
| 42 | 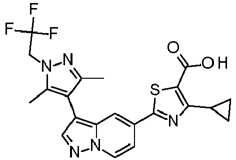 | 4-cyclopropyl-2-[3-[3,5-dimethyl-1-(2,2,2-trifluoroethyl)pyrazol-4-yl]pyrazolo[1,5-a]pyridin- 5-yl]thiazole-5-carboxylic acid | Cpd 123 | F | 461.5 | 462.7 |
| 43 | 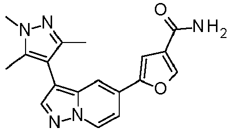 | 5-[3-(1,3,5-trimethylpyrazol-4-yl)pyrazolo[1,5-a]pyridin-5-yl]furan-3-carboxamide | Cpd 2 | M | 335.4 | NA |
| 44 | 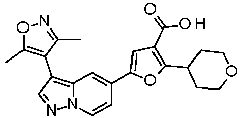 | 5 -[3 -(3,5 -dimethylisoxazol-4-yl)pyrazolo[1,5-a]pyridin-5-yl]-2-tetrahydropyran-4-yl-furan-3-carboxylic acid | Cpd 124 | F | 407.4 | 408.2 |
| 45 | 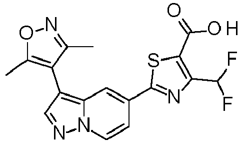 | 4-(difluoromethyl)-2-[3-(3,5 - dimethylisoxazol-4-yl)pyrazolo[1,5-a]pyridin- 5-yl]thiazole-5-carboxylic acid | Cpd 125 | F | 390.4 | 391.1 |
| 46 | 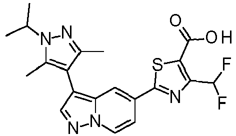 | 4-(difluoromethyl)-2-[3-(1-isopropyl-3,5-dimethyl-pyrazol-4-yl)pyrazolo[1,5-a]pyridin-5-yl]thiazole-5-carboxylic acid | Cpd 126 | F | 431.5 | 432.7 |
| 47 | 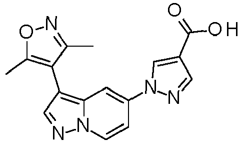 | 1-[3-(3,5-dimethylisoxazol-4-yl)pyrazolo[1,5-a]pyridin-5-yl]pyrazole-4-carboxylic acid | Cpd 127 | F | 323.3 | 324.7 |
| 48 | 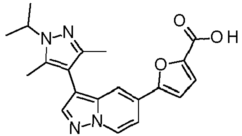 | 5-[3-(1-isopropyl-3,5-dimethyl-pyrazol-4-yl)pyrazolo[1,5-a]pyridin-5- yl]furan-2-carboxylic acid | Cpd 128 | F | 364.4 | 365.7 |
| 49 | 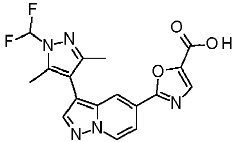 | 2-[3-[1-(difluoromethyl)-3,5-dimethyl-pyrazol-4-yl]pyrazolo[1,5-a]pyridin-5-yl]oxazole-5-carboxylic acid | Cpd 129 | F | 373.3 | 374.6 |
| 50 | 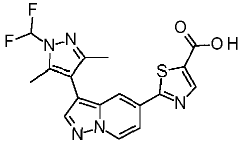 | 2-[3-[1-(difluoromethyl)-3,5-dimethyl-pyrazol-4-yl]pyrazolo[1,5-a]pyridin- 5-yl]thiazole-5-carboxylic acid | Cpd 130 | F | 389.4 | 390.6 |
| 51 | 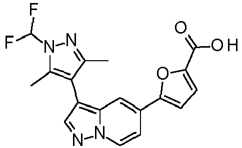 | 5-[3-[1-(difluoromethyl)-3,5-dimethyl-pyrazol-4-yl]pyrazolo[1,5-a]pyridin-5-yl]furan-2-carboxylic acid | Cpd 131 | F | 372.3 | 373.5 |
| 52 | 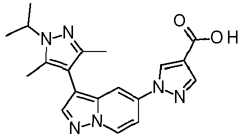 | 1-[3-(1-isopropyl-3,5-dimethyl-pyrazol-4-yl)pyrazolo[1,5-a]pyridin-5 - yl]pyrazole-4-carboxylic acid | Cpd 132 | F | 364.4 | 365.5 |
| 53 | 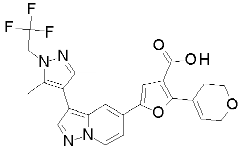 | 2-(3,6-dihydro-2H-pyran-4-yl)-5-[3-[3,5-dimethyl-1-(2,2,2-trifluoroethyl)pyrazol-4-yl]pyrazolo[1,5-a]pyridin-5-yl]furan-3-carboxylic acid | Cpd 133 | F | 486.4 | 487.5 |
| 54 | 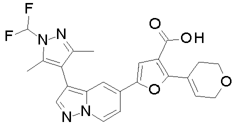 | 5-[3-[1-(difluoromethyl)-3,5-dimethyl-pyrazol-4-yl]pyrazolo[1,5-a]pyridin-5-yl]-2-(3,6-dihydro-2H-pyran-4-yl)furan-3-carboxylic acid | Cpd 134 | F | 454.4 | 455.5 |
| 55 | 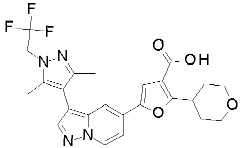 | 5-[3-[3,5-dimethyl-1-(2,2,2-trifluoroethyl)pyrazol-4-yl]pyrazolo[1,5-a]pyridin-5-yl]-2-tetrahydropyran-4-yl-furan-3-carboxylic acid | Cpd 135 | F | 488.5 | 489.8 |
| 56 | 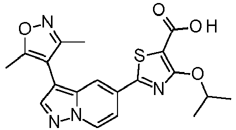 | 2-[3-(3,5-dimethylisoxazol-4-yl)pyrazolo[1,5-a]pyridin-5-yl]-4-isopropoxy-thiazole-5-carboxylic acid | Cpd 136 | F | 398.4 | 399.7 |
| 57 | 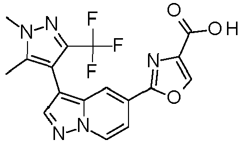 | 2-[3-[1,5-dimethyl-3-(trifluoromethyl)pyrazol-4-yl]pyrazolo[1,5-a]pyridin- 5-yl]oxazole-4-carboxylic acid | Cpd 137 | F | 391.3 | 392.6 |
| 58 | 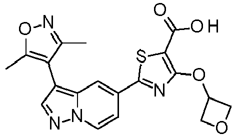 | 2-[3-(3,5-dimethylisoxazol-4-yl)pyrazolo[1,5-a]pyridin-5-yl]-4-(oxetan-3-yloxy) thiazole-5-carboxylic acid | Cpd 138 | F | 412.4 | 413.7 |
| 59 | 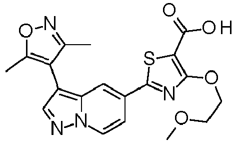 | 2-[3-(3,5-dimethylisoxazol-4-yl)pyrazolo[1,5-a]pyridin-5-yl]-4-(2-methoxyethoxy) thiazole-5-carboxylic acid | Cpd 139 | F | 414.4 | 415.7 |
| 60 | 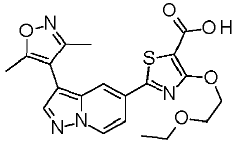 | 2-[3-(3,5-dimethylisoxazol-4-yl)pyrazolo[1,5-a]pyridin-5-yl]-4-(2-ethoxyethoxy) thiazole-5-carboxylic acid | Cpd 140 | F | 428.5 | 429.8 |
| 61 | 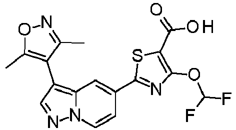 | 4-(difluoromethoxy)-2-[3- (3,5-dimethylisoxazol-4-yl)pyrazolo[1,5-a]pyridin-5 - yl]thiazole-5-carboxylic acid | Int 56 + CAS# 383-62-0 | Ex. 2.20 | 406.4 | 407.4 |
| 62 | 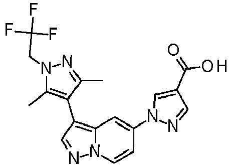 | 1-[3-[3,5-dimethyl-1-(2,2,2-trifluoroethyl)pyrazol-4-yl]pyrazolo[1,5-a]pyridin- 5-yl]pyrazole-4-carboxylic acid | Cpd 142 | F | 404.3 | 405.5 |
| 63 | 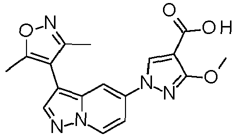 | 1-[3-(3,5-dimethylisoxazol-4-yl)pyrazolo[1,5-a]pyridin-5-yl]-3-methoxy-pyrazole-4-carboxylic acid | Cpd 143 | F | 353.3 | 354.2 |
| 64 | 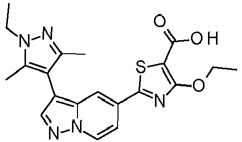 | 4-ethoxy-2-[3-(1-ethyl-3,5-dimethyl-pyrazol-4-yl)pyrazolo[1,5-a]pyridin- 5-yl]thiazole-5-carboxylic acid | Cpd 144 | F | 411.5 | 412.4 |
| 65 | 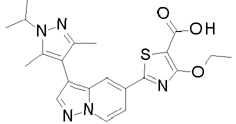 | 4-ethoxy-2-[3-(1-isopropyl-3,5-dimethyl-pyrazol-4-yl)pyrazolo[1,5-a]pyridin- 5-yl]thiazole-5-carboxylic acid | Cpd 145 | F | 425.5 | 426.5 |
| 66 | 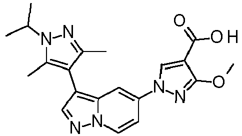 | 1-[3-(1-isopropyl-3,5-dimethyl-pyrazol-4-yl) pyrazolo[1,5-a]pyridin-5-yl]-3-methoxy-pyrazole-4-carboxylic acid | Cpd 146 | F | 394.4 | 395.5 |
| 67 | 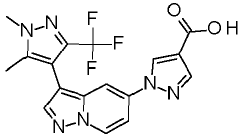 | 1-[3-[1,5-dimethyl-3-(trifluoromethyl)pyrazol-4-yl]pyrazolo[1,5-a]pyridin-5 - yl]pyrazole-4-carboxylic acid | Cpd 147 | F | 390.3 | 391.4 |
| 68 | 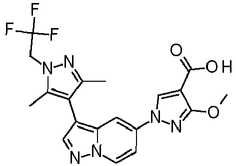 | 1-[3-[3,5-dimethyl-1-(2,2,2-trifluoroethyl)pyrazol-4-yl]pyrazolo[1,5-a]pyridin-5-yl]-3-methoxy-pyrazole-4-carboxylic acid | Cpd 148 | F | 434.4 | 435.3 |
| 69 | 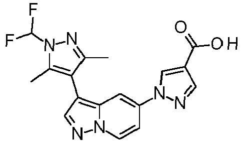 | 1-[3-[1-(difluoromethyl)-3,5-dimethyl-pyrazol-4-yl]pyrazolo[1,5-a]pyridin-5- yl]pyrazole-4-carboxylic acid | Cpd 149 | F | 372.3 | 373.3 |
| 70 | 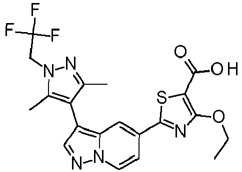 | 2-[3-[3,5-dimethyl-1-(2,2,2-trifluoroethyl)pyrazol-4-yl]pyrazolo[1,5-a]pyridin-5-yl]-4-ethoxy-thiazole-5-carboxylic acid | Cpd 150 | F | 465.4 | |
| 71 | 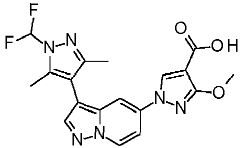 | 1-[3-[1-(difluoromethyl)-3,5-dimethyl-pyrazol-4-yl]pyrazolo[1,5-a]pyridin-5-yl]-3-methoxy-pyrazole-4-carboxylic acid | Cpd 151 | F | 402.4 | 403.4 |
| 72 | 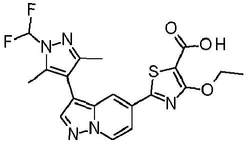 | 2-[3-[1-(difluoromethyl)-3,5-dimethyl-pyrazol-4-yl]pyrazolo[1,5-a]pyridin-5-yl]-4-ethoxy-thiazole-5-carboxylic acid | Cpd 152 | F | 433.4 | 434.4 |
| 73 | 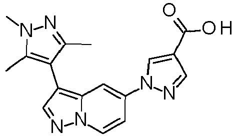 | 1-[3-(1,3,5-trimethylpyrazol-4-yl)pyrazolo[1,5-a]pyridin-5-yl]pyrazole-4-carboxylic acid | Cpd 153 | F | 336.3 | 337.4 |
| 74 | 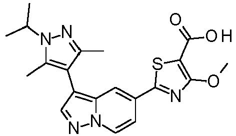 | 2-[3-(1-isopropyl-3,5-dimethyl-pyrazol-4-yl) pyrazolo[1,5-a]pyridin-5-yl]-4-methoxy-thiazole-5-carboxylic acid | Cpd 154 | F | 411.5 | 412.5 |
| 75 | 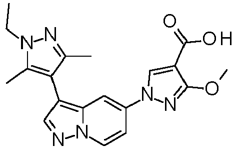 | 1-[3-(1-ethyl-3,5-dimethyl-pyrazol-4-yl)pyrazolo[1,5- a]pyridin-5-yl]-3-methoxy-pyrazole-4-carboxylic acid | Cpd 156 | F | 380.4 | 381.4 |
| 76 | 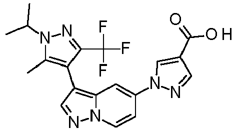 | 1-[3-[1-isopropyl-5-methyl-3-(trifluoromethyl)pyrazol-4-yl]pyrazolo[1,5-a]pyridin-5- yl]pyrazole-4-carboxylic acid | Cpd 157 | F | 418.4 | 419.5 |
| 77 | 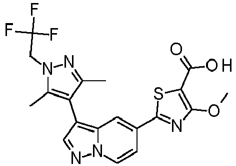 | 2-[3-[3,5-dimethyl-1-(2,2,2-trifluoroethyl)pyrazol-4-yl]pyrazolo[1,5-a]pyridin-5-yl]-4-methoxy-thiazole-5- carboxylic acid | Cpd 158 | F | 451.4 | 452.4 |
| 78 | 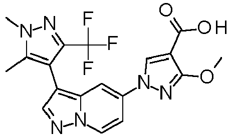 | 1-[3-[1,5-dimethyl-3-(trifluoromethyl)pyrazol-4-yl]pyrazolo[1,5-a]pyridin-5-yl]-3-methoxy-pyrazole-4-carboxylic acid | Cpd 159 | F | 420.3 | 421.3 |
| 79 | 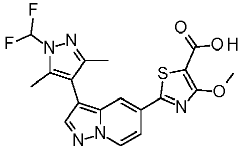 | 2-[3-[1-(difluoromethyl)-3,5-dimethyl-pyrazol-4-yl]pyrazolo[1,5-a]pyridin-5-yl]-4-methoxy-thiazole-5- carboxylic acid | Cpd 160 | F | 419.4 | 420.5 |
| 80 | 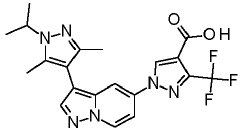 | 1-[3-(1-isopropyl-3,5-dimethyl-pyrazol-4-yl) pyrazolo[1,5-a]pyridin-5-yl]-3-(trifluoromethyl)pyrazole-4-carboxylic acid | Cpd 161 | F | 432.4 | 433.6 |
| 81 | 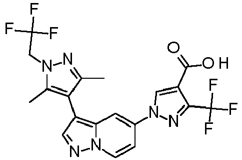 | 1-[3-[3,5-dimethyl-1-(2,2,2-trifluoroethyl)pyrazol-4-yl] pyrazolo[1,5-a]pyridin-5-yl]-3-(trifluoromethyl)pyrazole-4-carboxylic acid | Cpd 163 | F | 472.3 | 473.5 |
| 82 | 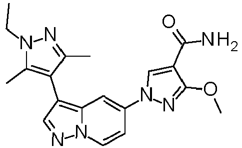 | 1-[3-(1-ethyl-3,5-dimethyl-pyrazol-4-yl)pyrazolo[1,5- a]pyridin-5-yl]-3-methoxy-pyrazole-4-carboxamide | Cpd 75 | M | 379.4 | 380.5 |
| 83 | 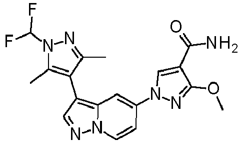 | 1-[3-[1-(difluoromethyl)-3,5-dimethyl-pyrazol-4-yl] pyrazolo[1,5-a]pyridin-5-yl]-3-methoxy-pyrazole-4-carboxamide | Cpd 71 | M | 401.4 | 402.4 |
| 84 | 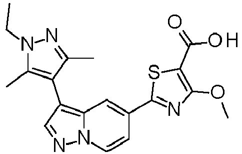 | 2-[3-(1-ethyl-3,5-dimethyl-pyrazol-4-yl)pyrazolo[1,5- a]pyridin-5-yl]-4-methoxy-thiazole-5-carboxylic acid | Cpd 164 | F | 397.5 | 398.4 |
| 85 | 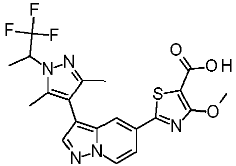 | 2-[3-[3,5-dimethyl-1-(2,2,2-trifluoro-1-methyl-ethyl) pyrazol-4-yl]pyrazolo[1,5-a]pyridin-5-yl]-4-methoxy-thiazole-5-carboxylic acid | Cpd 165 | F | 465.4 | 466.5 |
| 86 | 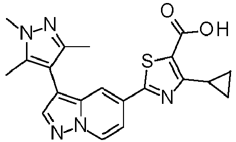 | 4-cyclopropyl-2-[3-(1,3,5-trimethylpyrazol-4-yl)pyrazolo[1,5-a]pyridin- 5-yl]thiazole-5-carboxylic acid | Cpd 166 | F | 393.5 | 394.5 |
| 87 | 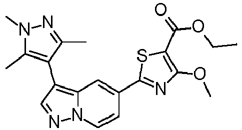 | ethyl 4-methoxy-2-[3-(1,3,5-trimethylpyrazol-4-yl) pyrazolo[1,5-a]pyridin-5- yl]thiazole-5-carboxylate | Int 12 + CAS# 844891-04-9 | E | 411.5 | 412.3 |
| 88 | 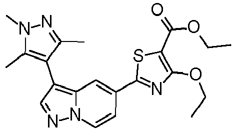 | ethyl 4-ethoxy-2-[3-(1,3,5-trimethylpyrazol-4-yl) pyrazolo[1,5-a]pyridin-5- yl]thiazole-5-carboxylate | Int 16 + CAS# 844891-04-9 | E | 425.5 | 426.3 |
| 89 | 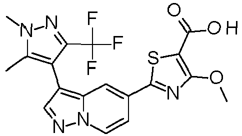 | 2-[3-[1,5-dimethyl-3-(trifluoromethyl)pyrazol-4-yl]pyrazolo[1,5-a]pyridin-5- yl]-4-methoxy-thiazole-5- carboxylic acid | Cpd 167 | F | 437.4 | 438.4 |
| 90 | 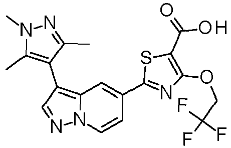 | 4-(2,2,2-trifluoroethoxy)-2-[3-(1,3,5-trimethylpyrazol-4-yl)pyrazolo[1,5-a]pyridin-5 - yl]thiazole-5-carboxylic acid | Cpd 168 | F | 451.4 | 452.3 |
| 91 | 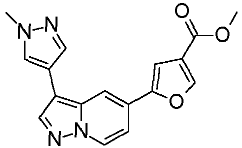 | methyl 5-[3-(1-methylpyrazol-4-yl)pyrazolo[1,5-a]pyridin-5 - yl]furan-3 -carboxylate | Int 10 + CAS# 761446-44-0 | E | 322.3 | 323.3 |
| 92 | 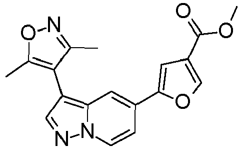 | methyl 5-[3-(3,5-dimethylisoxazol-4-yl)pyrazolo[1,5-a]pyridin-5 - yl]furan-3-carboxylate | Int 10 + CAS# 833114-00-8 | E | 337.3 | 338.3 |
| 93 | 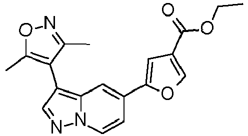 | ethyl 5-[3-(3,5-dimethylisoxazol-4-yl)pyrazolo[1,5-a]pyridin-5-yl]furan-3-carboxylate | Int 8 + CAS# 832114-00-8 | E | 351.4 | NA |
| 94 | 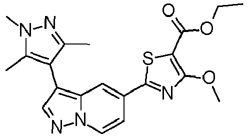 | ethyl 4-methoxy-2-[3-(1,3,5-trimethylpyrazol-4-yl)pyrazolo[1,5-a]pyridin-5- yl]thiazole-5-carboxylate | Int 12 + CAS# 832114-00-8 | E | 411.5 | 412.3 |
| 95 | 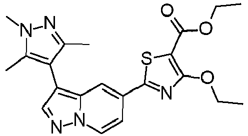 | ethyl 4-ethoxy-2-[3-(1,3,5-trimethylpyrazol-4-yl)pyrazolo[1,5-a]pyridin-5 - yl]thiazole-5 -carboxylate | Int 16 + CAS# 847818-62-6 | E | 425.5 | 427.1 |
| 96 | 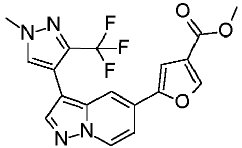 | methyl 5-[3-[1-methyl-3-(trifluoromethyl)pyrazol-4-yl]pyrazolo[1,5-a]pyridin-5-yl]furan-3-carboxylate | Int 10 + CAS# 1218790-53-4 | E | 390.2 | 391.3 |
| 97 | 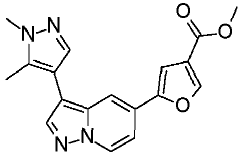 | methyl 5-[3-(1,5-dimethylpyrazol-4-yl)pyrazolo[1,5-a]pyridin-5 - yl]furan-3-carboxylate | Int 10 + CAS# 1036991-40-8 | E | 336.3 | 337.2 |
| 98 | 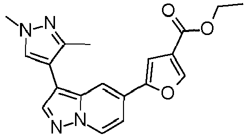 | ethyl 5-[3-(1,3-dimethylpyrazol-4-yl)pyrazolo[1,5-a]pyridin-5 - yl]furan-3-carboxylate | Int 8 + CAS# 1046832-21-6 | E | 350.4 | NA |
| 99 | 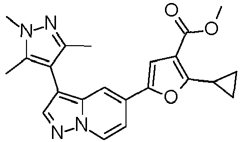 | methyl 2-cyclopropyl-5-[3-(1,3,5-trimethylpyrazol-4-yl)pyrazolo[1,5-a]pyridin-5-yl]furan-3-carboxylate | Int 18 + CAS# 126689-01-8 | L | 390.4 | 391.3 |
| 100 | 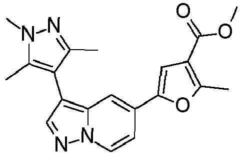 | methyl 2-methyl-5-[3-(1,3,5-trimethylpyrazol-4-yl) pyrazolo[1,5-a]pyridin-5-yl]furan-3-carboxylate | Int 18 + CAS# 823-96-1 | L | 364.4 | 365.3 |
| 101 | 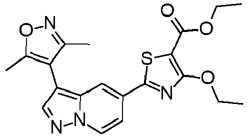 | ethyl 2-[3-(3,5-dimethyl isoxazol-4-yl)pyrazolo[1,5-a]pyridin-5-yl]-4-ethoxy-thiazole-5-carboxylate | Int 16 + CAS# 832114-00-8 | E | 412.5 | 413.3 |
| 102 | 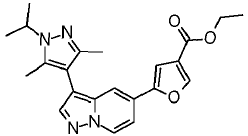 | ethyl 5-[3-(1-isopropyl-3,5-dimethyl-pyrazol-4-yl) pyrazolo[1,5-a]pyridin-5-yl]furan-3-carboxylate | Int 8 + Int 78 | Ex. 2.21 | 392.5 | 393.2 |
| 103 | 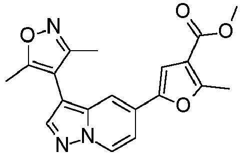 | methyl 5-[3-(3,5-dimethyl isoxazol-4-yl)pyrazolo[1,5-a]pyridin-5-yl]-2-methylfuran-3-carboxylate | Int 20 + CAS# 832114-00-8 | E | 351.4 | 352.3 |
| 104 | 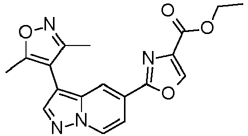 | ethyl 2-[3-(3,5-dimethylisoxazol-4-yl) pyrazolo[1,5-a]pyridin-5- yl]oxazole-4-carboxylate | Int 23 + CAS# 832114-00-8 | E | 352.3 | 353.7 |
| 105 | 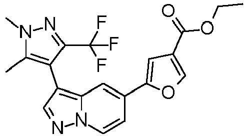 | ethyl 5-[3-[1,5-dimethyl-3-(trifluoromethyl)pyrazol-4-yl]pyrazolo[1,5-a]pyridin-5 - yl]furan-3-carboxylate | Int 25 + CAS# 721402-02-4 | E | 418.4 | 419.5 |
| 106 | 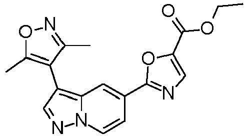 | ethyl 2-[3-(3,5-dimethylisoxazol-4-yl)pyrazolo[1,5-a]pyridin-5-yl]oxazole-5-carboxylate | Int 26 + CAS# 832114-00-8 | E | 352.3 | 353.2 |
| 107 | 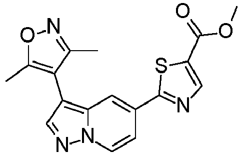 | methyl 2-[3-(3,5-dimethyl isoxazol-4-yl)pyrazolo[1,5-a] pyridin-5-yl]thiazole-5-carboxylate | Int 28 + CAS# 832114-00-8 | E | 354.4 | 355.2 |
| 108 | 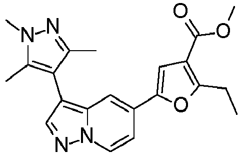 | methyl 2-ethyl-5-[3-(1,3,5-trimethylpyrazol-4-yl) pyrazolo[1,5-a]pyridin-5- yl]furan-3-carboxylate | Int 18 + CAS# 4433-63-0 | L | 378.4 | 379.5 |
| 109 | 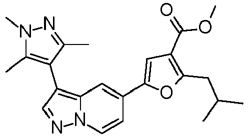 | methyl 2-isobutyl-5-[3-(1,3,5-trimethylpyrazol-4-yl)pyrazolo[1,5-a]pyridin-5 - yl]furan-3-carboxylate | Int 18 + CAS# 84110-40-7 | L | 406.5 | 407.3 |
| 110 | 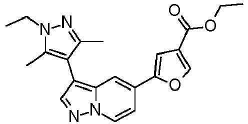 | ethyl 5-[3-(1-ethyl-3,5-dimethyl-pyrazol-4-yl)pyrazolo[1,5-a]pyridin-5 - yl]furan-3-carboxylate | Int 19 + CAS# 6629-60-3 | C | 378.4 | 379.0 |
| 111 | 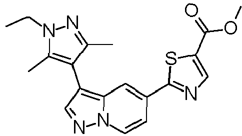 | methyl 2-[3-(1-ethyl-3,5-dimethyl-pyrazol-4-yl)pyrazolo[1,5-a]pyridin-5 - yl]thiazole-5 -carboxylate | Int 30 + CAS# 6629-60-3 | C | 381.5 | 382.5 |
| 112 | 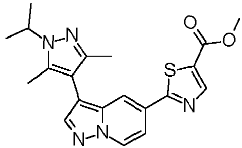 | methyl 2-[3-(1-isopropyl-3,5-dimethyl-pyrazol-4-yl)pyrazolo[1,5-a]pyridin-5-yl]thiazole-5 -carboxylate | Cpd 107 + CAS# 16726-41-3 | C | 395.5 | 396.6 |
| 113 | 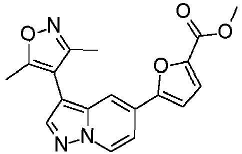 | methyl 5-[3-(3,5-dimethylisoxazol-4-yl)pyrazolo[1,5-a]pyridin-5 - yl] furan-2-carboxylate | Int 31 + CAS# 832114-00-8 | E | 337.3 | 338.7 |
| 114 | 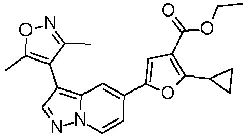 | ethyl 2-cyclopropyl-5-[3-(3,5-dimethylisoxazol-4-yl)pyrazolo[1,5-a]pyridin-5 - yl]furan-3-carboxylate | Int 33 + CAS# 126689-01-8 | L | 391.4 | 392.8 |
| 115 | 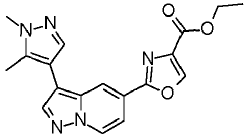 | ethyl 2-[3-(1,2-dimethyl pyrrol-3-yl)pyrazolo[1,5-a]pyridin-5-yl]oxazole-4-carboxylate | Int 23 + CAS# 1036991-40-8 | E | 350.4 | 352.7 |
| 116 | 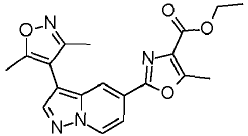 | ethyl 2-[3-(3,5-dimethylisoxazol-4-yl)pyrazolo[1,5-a]pyridin-5-yl]-5-methyl-oxazole-4-carboxylate | Int 34 + CAS# 832114-00-8 | E | 366.4 | NA |
| 117 | 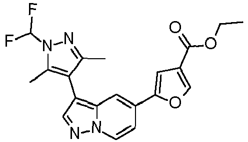 | ethyl 5-[3-[1-(difluoro methyl)-3,5-dimethyl-pyrazol-4-yl]pyrazolo[1,5-a] pyridin-5-yl]furan-3-carboxylate | Int 36 + CAS# 383-62-0 | B1 | 400.4 | 401.7 |
| 118 | 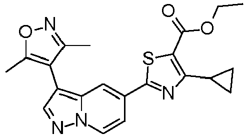 | ethyl 4-cyclopropyl-2-[3-(3,5-dimethylisoxazol-4-yl)pyrazolo[1,5-a]pyridin-5 - yl]thiazole-5-carboxylate | Int 40 + CAS# 832114-00-8 | E | 408.5 | 409.7 |
| 119 | 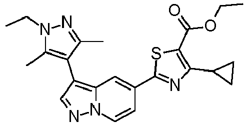 | ethyl 4-cyclopropyl-2-[3-(1-ethyl-3,5-dimethyl-pyrazol-4-yl)pyrazolo[1,5-a]pyridin-5-yl]thiazole-5-carboxylate | Int 43 | C | 435.5 | 436.7 |
| 120 | 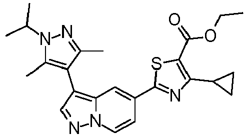 | ethyl 4-cyclopropyl-2-[3-(1-isopropyl-3,5-dimethyl-pyrazol-4-yl)pyrazolo[1,5-a]pyridin-5-yl]thiazole-5-carboxylate | Int 43 + CAS# 16726-41-3 | C | 449.6 | 450.8 |
| 121 | 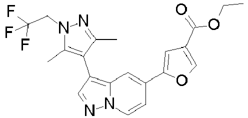 | ethyl 5-[3-[3,5-dimethyl-1-(2,2,2-trifluoroethyl)pyrazol-4-yl]pyrazolo[1,5-a]pyridin-5-yl]furan-3-carboxylate | Int 19 + CAS# 5042-30-8 | C | 432.4 | 433.5 |
| 122 | 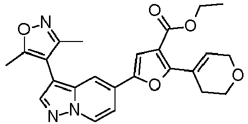 | ethyl 2-(3,6-dihydro-2H-pyran-4-yl)-5-[3-(3,5-dimethylisoxazol-4-yl)pyrazolo[1,5-a]pyridin-5-yl]furan-3-carboxylate | Int 33 + CAS# 287944-16-5 | E | 433.5 | 434.8 |
| 123 | 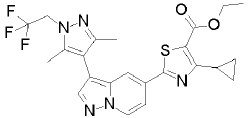 | ethyl 4-cyclopropyl-2-[3-[3,5-dimethyl-1-(2,2,2-trifluoroethyl)pyrazol-4-yl]pyrazolo[1,5-a]pyridin-5-yl]thiazole-5-carboxylate | Int 43 + CAS# 5042-30-8 | C | 489.5 | 490.8 |
| 124 | 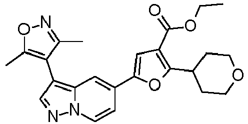 | ethyl 5-[3-(3,5-dimethylisoxazol-4-yl)pyrazolo[1,5-a]pyridin-5-yl]-2-tetrahydropyran-4-yl-furan-3-carboxylate | Cpd 122 | Q | 435.5 | NA |
| 125 | 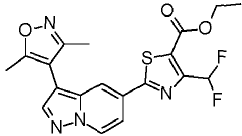 | ethyl 4-(difluoromethyl)-2-[3-(3,5-dimethylisoxazol-4-yl)pyrazolo[1,5-a]pyridin-5-yl]thiazole-5-carboxylate | Int 44 + CAS# 832114-00-8 | E | 418.4 | 419.7 |
| 126 | 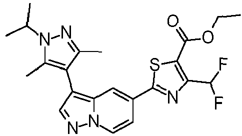 | ethyl 4-(difluoromethyl)-2-[3-(1-isopropyl-3,5-dimethyl-pyrazol-4-yl)pyrazolo[1,5-a]pyridin-5-yl]thiazole-5-carboxylate | Int47 + CAS# 16726-41-3 | C | 459.5 | NA |
| 127 | 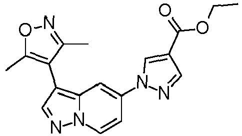 | ethyl 1-[3-(3,5-dimethylisoxazol-4-yl)pyrazolo[1,5-a]pyridin-5- yl]pyrazole-4-carboxylate | Int 48 + CAS# 832114-00-8 | E | 351.4 | 352.7 |
| 128 | 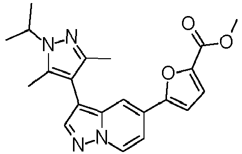 | methyl 5-[3-(1-isopropyl-3,5-dimethyl-pyrazol-4-yl)pyrazolo[1,5-a]pyridin-5-yl] furan-2-carboxylate | Int 50 + CAS# 16726-41-3 | C | 378.4 | 379.7 |
| 129 | 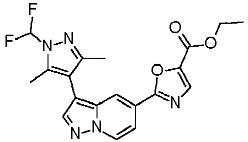 | ethyl 2-[3-[1-(difluoromethyl)-3,5-dimethyl-pyrazol-4-yl]pyrazolo[1,5-a]pyridin-5-yl]oxazole-5-carboxylate | Int 51 + CAS# 383-62-0 | B1 | 401.4 | NA |
| 130 | 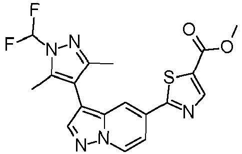 | methyl 2-[3-[1-(difluoromethyl)-3,5-dimethyl-pyrazol-4-yl]pyrazolo[1,5-a]pyridin-5-yl]thiazole-5-carboxylate | Int 52 + CAS# 383-62-0 | B1 | 403.4 | 404.6 |
| 131 | 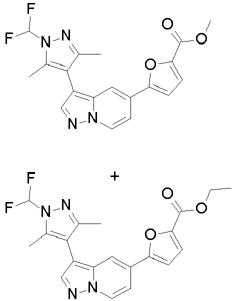 | methyl 5-[3-[1-(difluoro methyl)-3,5-dimethyl-pyra zol-4-yl]pyrazolo[1,5-a] pyridin-5-yl]furan-2-carboxylate / ethyl 5-[3-[1-(difluoromethyl) -3,5-di methyl-pyrazol-4-yl] pyrazolo[1,5-a]pyridin-5- yl]furan-2-carboxylate mixture | Int 53 + CAS# 383-62-0 | B1 | 386.4 + 400.4 | 387.7 + 401.7 |
| 132 | 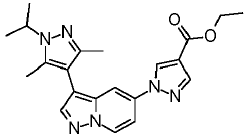 | ethyl 1-[3-(1-isopropyl-3,5-dimethyl-pyrazol-4-yl)pyrazolo[1,5-a]pyridin-5-yl]pyrazole-4-carboxylate | Int 54 + CAS# 16726-41-3 | C | 392.5 | 393.7 |
| 133 | 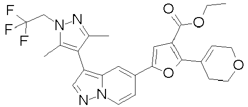 | ethyl 2-(3,6-dihydro-2H-pyran-4-yl)-5-[3-[3,5-dimethyl-1-(2,2,2-trifluoroethyl)pyrazol-4-yl]pyrazolo[1,5-a]pyridin-5-yl]furan-3-carboxylate | Int 55 + CAS# 5042-30-8 | C | 514.5 | NA |
| 134 | 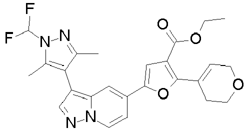 | ethyl 5-[3-[1-(difluoromethyl)-3,5-dimethyl-pyrazol-4-yl]pyrazolo[1,5-a]pyridin-5- yl]-2-(3,6-dihydro-2H-pyran-4-yl)furan-3 -carboxylate | Int 55 + CAS# 5341-61-7 + CAS# 1895-39-2 | C + B | 482.5 | 483.6 |
| 135 | 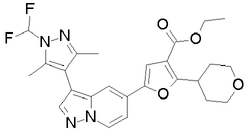 | ethyl 5-[3-[1-(difluorome thyl)-3,5-dimethyl-pyrazol -4-yl]pyrazolo[1,5-a]pyridin-5-yl]-2-tetrahydropyran-4-yl-furan-3-carboxylate | Cpd 134 | Q | 484.5 | NA |
| 136 | 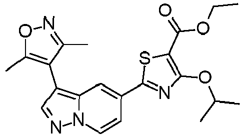 | ethyl 2-[3-(3,5-dimethyl isoxazol-4-yl)pyrazolo[1,5-a]pyridin-5-yl]-4-isopro poxy-thiazole-5-carboxylate | Int 56 + CAS# 75-30-9 | H | 426.5 | 427.2 |
| 137 | 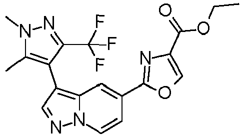 | ethyl 2-[3-[1,5-dimethyl-3-(trifluoromethyl)pyrazol-4-yl]pyrazolo[1,5-a]pyridin-5-yl]oxazole-4-carboxylate | Int 25 + CAS# 51294-75-8 | E | 419.4 | NA |
| 138 | 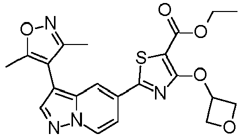 | ethyl 2-[3-(3,5-dimethylisoxazol-4-yl)pyrazolo[1,5-a]pyridin-5 - yl]-4-(oxetan-3-yloxy) thiazole-5-carboxylate | Int 56 + CAS# 26272-85-5 | H | 440.5 | 441.7 |
| 139 | 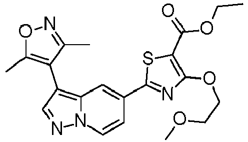 | ethyl 2-[3-(3,5-dimethylisoxazol-4-yl)pyrazolo[1,5-a]pyridin-5 - yl]-4-(2-methoxyethoxy) thiazole-5-carboxylate | Int 56 + CAS# 6482-24-2 | H | 442.5 | 443.8 |
| 140 | 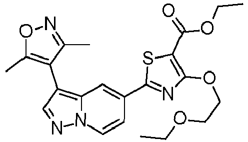 | ethyl 2-[3-(3,5-dimethylisoxazol-4-yl)pyrazolo[1,5-a]pyridin-5- yl]-4-(2-ethoxyethoxy) thiazole-5 -carboxylate | Int 56 + CAS# 592-55-2 | H | 456.5 | 457.7 |
| 141 | 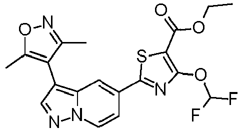 | ethyl 4-(difluoromethoxy)-2-[3-(3,5-dimethylisoxazol-4-yl)pyrazolo[1,5-a]pyridin-5-yl]thiazole-5-carboxylate | Int 56 + CAS# 115262-01-6 | H | 434.4 | 435.3 |
| 142 | 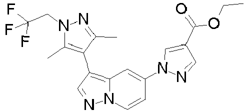 | ethyl 1-[3-[3,5-dimethyl-1-(2,2,2-trifluoroethyl)pyrazol-4-yl]pyrazolo[1,5-a]pyridin-5-yl]pyrazole-4-carboxylate | Int 54 + CAS# 5042-30-8 | C | 432.4 | 433.8 |
| 143 | 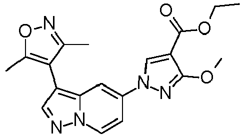 | ethyl 1-[3-(3,5-dimethylisoxazol-4-yl)pyrazolo[1,5-a]pyridin-5- yl]-3-methoxy-pyrazole-4-carboxylate | Int 59 + CAS# 832114-00-8 | E | 381.4 | 382.7 |
| 144 | 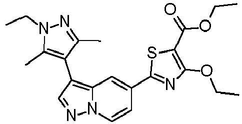 | ethyl 4-ethoxy-2-[3-(1-ethyl-3,5-dimethyl-pyrazol-4-yl)pyrazolo[1,5-a]pyridin-5 - yl]thiazole-5-carboxylate | Int 61 + CAS# 6629-60-3 | C | 439.5 | 440.5 |
| 145 | 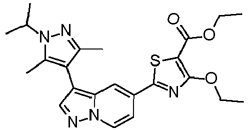 | ethyl 4-ethoxy-2-[3-(1-isopropyl-3,5-dimethyl-pyrazol-4-yl)pyrazolo[1,5-a]pyridin-5-yl]thiazole-5-carboxylate | Int 61 + CAS# 16726-41-3 | C | 453.6 | 454.5 |
| 146 | 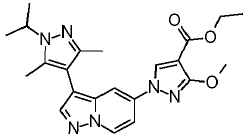 | ethyl 1-[3-(1-isopropyl-3,5-dimethyl-pyrazol-4-yl)pyrazolo[1,5-a]pyridin-5-yl]-3-methoxy-pyrazole-4-carboxylate | Int 62 + CAS# 16726-41-3 | C | 422.5 | 423.5 |
| 147 | 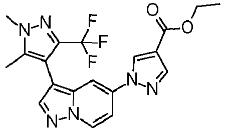 | ethyl 1-[3-[1,5-dimethyl-3-(trifluoromethyl)pyrazol-4-yl]pyrazolo[1,5-a]pyridin-5- yl]pyrazole-4-carboxylate | Int 66 | E | 418.1 | NA |
| 148 | 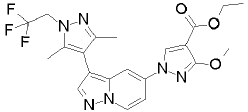 | ethyl 1-[3-[3,5-dimethyl-1-(2,2,2-trifluoroethyl)pyrazol-4-yl]pyrazolo[1,5-a]pyridin-5-yl]-3-methoxy-pyrazole-4-carboxylate | Int 62 + CAS# 5042-30-8 | C | 462.4 | 463.5 |
| 149 | 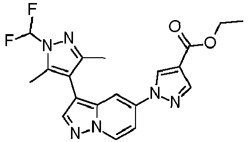 | ethyl 1-[3-[1-(difluoro methyl)-3,5-dimethyl-pyrazol-4-yl]pyrazolo[1,5-a]pyridin-5-yl]pyrazole-4- carboxylate | Int 63 + CAS# 1895-39-2 | B2 | 400.4 | 401.5 |

| **Cpd#** | **Structure** | **Name** | **SM** | **Mtd** | **MW** | **MS Mes' d** |
|---|---|---|---|---|---|---|
| 150 | 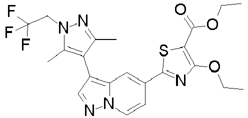 | ethyl 2-[3-[3,5-dimethyl-1-(2,2,2-trifluoroethyl)pyrazol-4-yl]pyrazolo[1,5-a]pyridin-5-yl]-4-ethoxy-thiazole-5- carboxylate | Int 61 + CAS# 5042-30-8 | C | 493.5 | 494.4 |
| 151 | 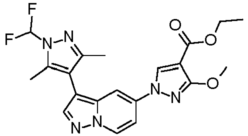 | ethyl 1-[3-[1-(difluoro methyl)-3,5-dimethyl-pyrazol-4-yl]pyrazolo[1,5-a]pyridin-5-yl]-3-methoxy-pyrazole-4-carboxylate | Int 59 + CAS# 1258401-28-3 | E | 430.4 | 431.5 |
| 152 | 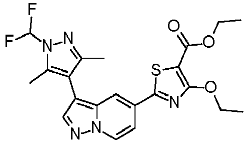 | ethyl 2-[3-[1-(difluoro methyl)-3,5-dimethyl-pyrazol-4-yl]pyrazolo[1,5-a]pyridin-5-yl]-4-ethoxy-thiazole-5-carboxylate | Int 64 + CAS# 1895-39-2 | B2 | 461.5 | 462.5 |
| 153 | 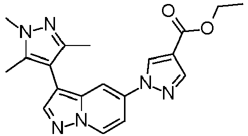 | ethyl 1-[3-(1,3,5-trimethylpyrazol-4-yl)pyrazolo[1,5-a]pyridin-5-yl]pyrazole-4-carboxylate | Int 48 + CAS# 844891-04-9 | C | 364.4 | 365.5 |
| 154 | 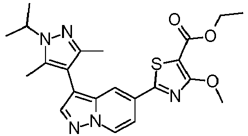 | ethyl 2-[3-(1-isopropyl-3,5-dimethyl-pyrazol-4-yl) pyrazolo[1,5-a]pyridin-5-yl]-4-methoxy-thiazole-5-carboxylate | Int 65 + CAS# 16726-41-3 | C | 439.5 | 440.5 |
| 155 | 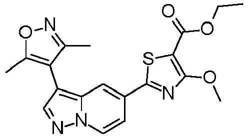 | ethyl 2-[3-(3,5-dimethyl isoxazol-4-yl)pyrazolo[1,5-a]pyridin-5-yl]-4-methoxy-thiazole-5-carboxylate | Int 56 | H | 398.4 | 399.4 |
| 156 | 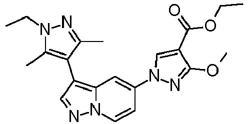 | ethyl 1-[3-(1-ethyl-3,5-dimethyl-pyrazol-4-yl) pyrazolo[1,5-a]pyridin-5-yl]-3-methoxy-pyrazole-4-carboxylate | Int 62 + CAS# 6629-60-3 | C | 408.5 | 409.6 |

| **Cpd#** | **Structure** | **Name** | **SM** | **Mtd** | **MW** | **MS Mes'd** |
|---|---|---|---|---|---|---|
| 157 | 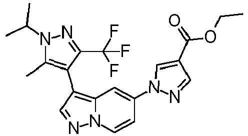 | ethyl 1-[3-[1-isopropyl-5-methyl-3-(trifluoromethyl) pyrazol-4-yl]pyrazolo[1,5-a]pyridin-5-yl]pyrazole-4-carboxylate | Int 66 + Int 67 | E | 446.4 | 447.6 |
| 158 | 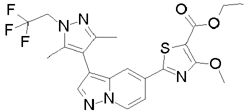 | ethyl 2-[3-[3,5-dimethyl-1-(2,2,2-trifluoroethyl)pyrazol-4-yl]pyrazolo[1,5-a]pyridin-5-yl]-4-methoxy-thiazole-5- carboxylate | Int 65 + CAS# 5042-30-8 | C | 479.5 | 480.6 |
| 159 | 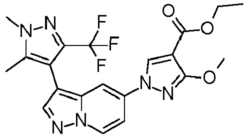 | ethyl 1-[3-[1,5-dimethyl-3-(trifluoromethyl)pyrazol-4-yl]pyrazolo[1,5-a]pyridin-5-yl]-3-methoxy-pyrazole-4-carboxylate | Int 76 + CAS# 721402-02-4 | E | 448.4 | 449.3 |
| 160 | 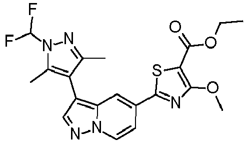 | ethyl 2-[3-[1-(difluoro methyl)-3,5-dimethyl-pyrazol-4-yl]pyrazolo[1,5-a]pyridin-5-yl]-4-methoxy-thiazole-5 -carboxylate | Int 70 + CAS# 1895-39-2 | B2 | 447.5 | 448.3 |
| 161 | 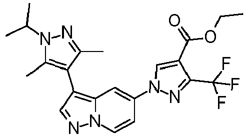 | ethyl 1-[3-(1-isopropyl-3,5-dimethyl-pyrazol-4-yl)pyrazolo[1,5-a]pyridin-5-yl]-3-(trifluoromethyl) pyrazole-4-carboxylate | Int 71 + CAS# 16726-41-3 | C | 460.5 | 461.6 |
| 162 | 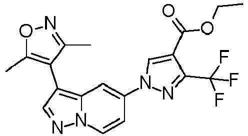 | ethyl 1-[3-(3,5-dimethyl isoxazol-4-yl)pyrazolo[1,5-a]pyridin-5-yl]-3-(trifluoro methyl)pyrazole-4-carboxylate | Int 72 + CAS# 832114-00-8 | E | 419.4 | 420.4 |
| 163 | 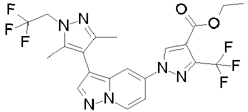 | ethyl 1-[3-[3,5-dimethyl-1-(2,2,2-trifluoroethyl)pyrazol-4-yl]pyrazolo[1,5-a]pyridin-5-yl]-3-(trifluoromethyl) pyrazole-4-carboxylate | Int 71 + CAS# 5042-30-8 | C | 500.4 | 501.5 |
| 164 | 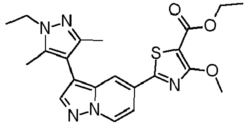 | ethyl 2-[3-(1-ethyl-3,5-dimethyl-pyrazol-4-yl)pyrazolo[1,5-a]pyridin-5-yl]-4-methoxy-thiazole-5-carboxylate | Int 65 + CAS# 6629-60-3 | C | 425.5 | 426.6 |
| 165 | 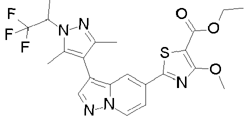 | ethyl 2-[3-[3,5-dimethyl-1-(2,2,2-trifluoro-1-methylethyl)pyrazol-4-yl] pyrazolo[1,5-a]pyridin-5-yl]- 4-methoxy-thiazole-5-carboxylate | Int 65 + CAS# 1453472-98-4 | C | 493.5 | 494.3 |
| 166 | 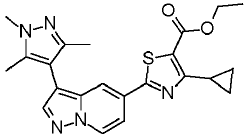 | ethyl 4-cyclopropyl-2-[3-(1,3,5-trimethylpyrazol-4-yl)pyrazolo[1,5-a]pyridin-5- yl]thiazole-5-carboxylate | Int 40 + CAS# 847818-62-6 | E | 421.5 | 422.3 |
| 167 | 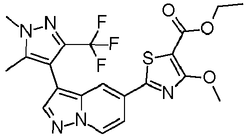 | ethyl 2-[3-[1,5-dimethyl-3-(trifluoromethyl)pyrazol-4-yl]pyrazolo[1,5-a]pyridin-5 - yl] -4-methoxy-thiazole-5- carboxylate | Int 77 + CAS# 721402-02-4 | E | 465.5 | 466.6 |
| 168 | 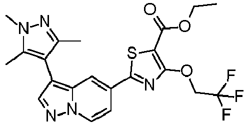 | ethyl 4-(2,2,2-trifluoroethoxy)-2-[3-(1,3,5-trimethylpyrazol-4-yl)pyrazolo[1,5-a]pyridin-5-yl]thiazole-5-carboxylate | Int 74 + CAS# 844891-04-9 | E | 479.5 | 480.5 |

| | | | | | | |
|---|---|---|---|---|---|---|
| SM = Starting Material, Mtd = Method, MS Mes'd = Mesured mass | | | | | | |

**Table IV. NMR data of illustrative compounds of the invention.**

| **Cpd#** | **NMR data** |
|---|---|
| 2 | ¹H NMR (300 MHz, DMSO-*d*₆) δ 8.75 (dd, 1H), 8.38 (d, 1H), 8.00 (s, 1H), 7.62 (dd, 1H), 7.48 (d, 1H), 7.32 (dd, 1H), 3.75 (s, 3H), 2.16 (s, 3H), 2.07 (s, 3H) |
| 4 | ¹H NMR (400 MHz, DMSO-*d*₆) δ 8.80 (dd, 1H), 8.42 (d, 1H), 8.17 (s, 1H), 7.80 (dd, 1H), 7.57 (d, 1H), 7.36 (dd, 1H), 2.36 (s, 3H), 2.18 (s, 3H) |
| 5 | ¹H NMR (400 MHz, DMSO-*d*₆) δ 8.75 (dd, 1H), 8.50 (d, 1H), 8.00 (s, 1H), 7.64 (dd, 1H), 7.57 (d, 1H), 7.32 (dd, 1H), 3.81 (s, 3H), 3.74 (s, 3H), 2.15 (s, 3H), 2.06 (s, 3H) |
| 9 | ¹H NMR (400 MHz, DMSO-*d*₆) δ 12.93 - 12.88 (br s, 1H), 8.82 (d, 1H), 8.11 (s, 1H), 7.96 (d, 1H), 7.39 (dd, 1H), 4.54 (q, 2H), 3.75 (s, 3H), 2.18 (s, 3H), 2.09 (s, 3H), 1.38 (t, 3H) |
| 18 | ¹H NMR (400 MHz, DMSO-*d*₆) δ 12.85 (br s, 1H), 8.74 (dd, 1H), 8.38 (d, 1H), 8.01 (s, 1H), 7.62 (dd, 1H), 7.49 (d, 1H), 7.31 (dd, 1H), 4.51 (m, 1H), 2.17 (s, 3H), 2.09 (s, 3H), 1.42 (d, 6H) |
| 32 | ¹H NMR (300 MHz, DMSO-*d*₆) δ 12.75 (br s, 1H), 8.75 (dd, 1H), 8.16 (s, 1H), 7.68 (dd, 1H), 7.43 (s, 1H), 7.29 (dd, 1H), 2.88 - 2.75 (m, 1H), 2.36 (s, 3H), 2.20 (s, 3H), 1.14 (m, 4H) |
| 35 | ¹H NMR (300 MHz, DMSO-*d*₆) δ 12.87 (br s, 1H), 8.80 (dd, 1H), 8.41 (d, 1H), 8.13 (s, 1H), 7.72 (dd, 1H), 7.65 - 7.53 (m, 1H), 7.36 (dd, 1H), 2.33 (s, 3H), 2.15 (s, 3H) |
| 36 | ¹H NMR (400 MHz, DMSO-*d*₆) δ 8.94 (dd, 1H), 8.32 (s, 1H), 8.08 (dd, 1H), 7.47 (dd, 1H), 2.35 (s, 3H), 2.17 (s, 3H) |
| 39 | ¹H NMR (400 MHz, DMSO-*d*₆) δ 13.51 (br s, 1H), 8.79 (dd, 1H), 8.10 (s, 1H), 7.88 (dd, 1H), 7.37 (dd, 1H), 4.53 (m, 1H), 3.09 - 2.98 (m, 1H), 2.19 (s, 3H), 2.10 (s, 3H), 1.42 (d, 6H), 1.14 - 1.07 (m, 4H) |
| 40 | ¹H NMR (400 MHz, DMSO-*d*₆) δ 12.84 (br s, 1H), 8.76 (dd, 1H), 8.40 (d, 1H), 8.07 (s, 1H), 7.60 (dd, 1H), 7.50 (d, 1H), 7.34 (dd, 1H), 5.07 (q, 2H), 2.21 (s, 3H), 2.10 (s, 3H) |
| 42 | ¹H NMR (400 MHz, DMSO-*d*₆) δ 13.51 (br s, 1H), 8.81 (dd, 1H), 8.17 (s, 1H), 7.88 (dd, 1H), 7.38 (dd, 1H), 5.09 (q, 2H), 3.03 (tt, 1H), 2.23 (s, 3H), 2.12 (s, 3H), 1.22 - 1.05 (m, 4H) |
| 43 | ¹H NMR (400 MHz, DMSO-*d*₆) δ 8.74 (d, 1H), 8.28 (s, 1H), 8.00 (s, 1H), 7.71 (s, 1H), 7.54 (s, 1H), 7.45 (s, 1H), 7.31 (s, 1H), 7.21 (d, 1H), 3.74 (s, 3H), 2.15 (s, 3H), 2.06 (s, 3H) |
| 55 | ¹H NMR (400 MHz, DMSO-*d*₆) δ 12.81 (br s, 1H), 8.73 (dd, 1H), 8.07 (s, 1H), 7.56 (t, 1H), 7.38 (s, 1H), 7.31 (dd, 1H), 5.08 (q, 2H), 3.99 - 3.90 (m, 2H), 3.76 - 3.64 (m, 1H), 3.44 (td, 2H), 2.23 (s, 3H), 2.12 (s, 3H), 1.86 (m, 2H), 1.76 (d, 2H) |
| 64 | ¹H NMR (400 MHz, CDCl₃) δ 8.47 (d, 1H), 7.90 - 7.85 (m, 2H), 7.24 (dd, 1H), 4.65 (q, 2H), 4.17 (m, 2H), 2.17 (d, 6H), 1.56 - 1.40 (m, 6H), 1.18 (s, 1H) |
| 67 | ¹H NMR (400 MHz, DMSO-*d*₆) δ 12.72 (br s, 1H), 9.24 (s, 1H), 8.90 (d, 1H), 8.13 (s, 1H), 8.02 (s, 1H), 7.93 (d, 1H), 7.64 (dd, 1H), 3.92 (s, 3H), 2.18 (s, 3H) |
| 78 | ¹H NMR (400 MHz, DMSO-*d*₆) δ 12.44 (br s, 1H), 9.05 (s, 1H), 8.85 (dd, 1H), 7.99 (s, 1H), 7.80 (d, 1H), 7.58 (dd, 1H), 3.96 (s, 3H), 3.92 (s, 3H), 2.18 (s, 3H) |
| 86 | ¹H NMR (400 MHz, DMSO-*d*₆) δ 13.46 (br s, 1H), 8.79 (d, 1H), 8.09 (s, 1H), 7.90 (s, 1H), 7.37 (d, 1H), 3.75 (s, 3H), 3.03 (m, 1H), 2.17 (s, 3H), 2.08 (s, 3H), 1.11 (d, 4H) |

### BIOLOGICAL EXAMPLES

### Example 3. In vitro assays

### 3.1. Biochemical assays

### 3.1.1. ³³P Radioactive Kinase Assay

### 3.1.1.1. Overview

The principle of the ³³P radioactive kinase assay consists in measuring the incorporated ³³P into the ZIPtide peptide substrate when phosphorylated by human PASK using [³³P]-γ-ATP and ATP, which correlates with kinase activity.

### 3.1.1.2. Protocol

The test compounds are prepared as a serial dilution of 10 point dose responses with 1/5 dilution steps in 100% DMSO starting from 0.2 or 2 mM highest concentration, diluted 1/20 in water and 5 µL is transferred to the assay plates (Greiner, Cat# 651201).

1% DMSO and 10 µM staurosporine final concentrations are used respectively as negative and positive controls.

11 µL of enzyme-substrate mixture is added on the assay plates. The reactions are started by adding 9 µL ATP mixture, consisting of non-labeled and ³³P-labeled ATP, on the assay plates. Plates are incubated at 30°C for the time intervals indicated in Table V.

**Table V. Conditions for human PASK kinase ³³P radioactive assay**

| **Kinase, [Kinase]** | **Substrate, [Substrate]** | **ATP** | **Assay buffer** |
|---|---|---|---|
| PASK (ThermoFisher Scientific, Cat# PR7013A), 200 ng/mL | ZIPtide (Merck Millipore, Cat# 12-545), 1 µM | 10 µM ATP + 0.25 µCi/25 µL [γ-³³P]ATP | 25 mM MOPS pH 7.0 |
| | | | 0.01% Triton X-100 |
| | | | 0.5 mM EGTA |
| | | | 2.5 mM DTT |
| | | | 5 mM MgCl₂ |

The reactions are stopped by adding 25 µL phosphoric acid (150 mM) to the reactions.

The completely terminated kinase reactions are transferred using a harvester on pre-wetted UniFilter-96 plates (UniFilter-96 GF/B, PerkinElmer Inc., Cat# 6005177).

After harvesting the kinase reactions, the filter plates are washed 6 times with phosphoric acid (75 mM). The back of the UniFilter-96 plates are sealed and 40 µL MicroScint-20 (PerkinElmer Inc., Cat#6013621) is added to each well. The top of each plate is sealed with TopSeal-A (PerkinElmer Inc., Cat# 6050185). Read-out is performed with a TopCount instrument (PerkinElmer Inc.).

### 3.1.1.3. Data analysis and results

Raw data are generated following the read-out performed on the TopCount, used to calculate percentage inhibition (PIN) values and plotted to generate dose response curves and derive the average half maximal inhibitory concentrations (IC₅₀) reported in Table VI.

**Table VI. ³³P radioactive PASK kinase assay IC₅₀ of illustrative compounds of the invention**

| **Cpd#** | **PASK IC₅₀** |
|---|---|
| 1 | **** |
| 2 | **** |
| 3 | *** |
| 4 | **** |
| 5 | *** |
| 6 | *** |
| 7 | *** |
| 8 | **** |
| 9 | **** |
| 11 | **** |
| 14 | **** |
| 15 | **** |
| 16 | **** |
| 17 | **** |
| 18 | **** |
| 19 | **** |
| 20 | *** |
| 21 | *** |
| 22 | *** |
| 23 | **** |
| 24 | **** |
| 25 | **** |
| 26 | **** |
| 27 | **** |
| 28 | **** |
| 29 | **** |
| 30 | **** |
| 31 | *** |
| 32 | **** |
| 33 | *** |
| 34 | *** |
| 35 | **** |
| 36 | ** |
| 37 | **** |
| 38 | **** |
| 39 | **** |
| 40 | **** |
| 41 | **** |
| 42 | **** |
| 43 | **** |
| 44 | **** |
| 45 | *** |
| 46 | **** |

| | |
|---|---|
| * > 500 nM ** > 100 - 500 nM *** > 10 - 100 nM **** 0.01 - 10 nM | |

### 3.1.2. ADP-Glo^{™} Kinase Assay

### 3.1.2.1. Overview

The ADP-Glo^{™} kinase assay is a luminescent technology assay which measures the ADP formed from a kinase reaction. In this specific study, the kinase reactions consisted of the phosphorylation of the ZIPtide peptide substrate by human recombinant PASK. In a second step the kinase reaction is terminated and all the remaining ATP is depleted. In a final step the ADP is converted into ATP and this newly synthesized ATP is measured by using a luciferase/luciferin reaction. The generated light is measured using an Envision plate reader, wherein the luminescent signal obtained positively correlates with the kinase activity.

### 3.1.2.2. Protocol

The test compounds are prepared as a serial dilution of 10 point dose responses with 1/5 dilution steps in 100% DMSO starting from 2 mM highest concentration, diluted 1/20 in water and 1 µL is transferred to the assay plates (PerkinElmer Inc., Cat# 6007290).

1% DMSO and 10 µM staurosporine final concentrations are used respectively as negative and positive controls.

2 µL enzyme-substrate mixture is added to the assay plates.

The reaction is started by adding 2 µL diluted ATP on the assay plates immediately after addition of the enzyme-substrate mixture to the compound. Plates are centrifuged for a few seconds at 1000 rpm and gently shaken for 2 min followed by an incubation at RT for 120 min.

The reactions are stopped and the unconsumed ATP is depleted by adding 5 µL ADP-Glo Reagent (Promega, Cat# V912B) to the reaction. The plates are centrifuged for a few seconds at 1000 rpm and incubated at RT for 40 min (ATP depletion).

The ADP is converted to ATP and luciferase and luciferin is introduced to detect ATP by adding 10 µL Kinase Detection Reagent (Promega, Cat# V913B + V914B) to the reaction. The plates are centrifuged for a few seconds at 1000 rpm and incubated at RT for 30 min (ADP detection).

Luminescence is measured on an Envision plate reader (PerkinElmer Inc.).

**Table VII. Conditions for human PASK kinase ADP-Glo^{™} assay**

| **Kinase, [Kinase]** | **Substrate, [Substrate]** | **ATP** | **Assay buffer** |
|---|---|---|---|
| PASK (ThermoFisher Scientific, Cat# PR7013A), 125 ng/mL | ZIPtide (Merck Millipore, Cat# 12-545), 25 µM | 25 µM ATP (Promega, Cat# V915B) | 25 mM MOPS pH 7.0 |
| | | | 0.01% Triton X-100 |
| | | | 0.5 mM EGTA |
| | | | 2.5 mM DTT |
| | | | 5 mM MgCl₂ |

### 3.1.2.3. Data analysis and results

Raw data are generated following the read-out performed on the Envision plate reader, used to calculate percentage inhibition (PIN) values and plotted to generate dose response curves and derive the average half maximal inhibitory concentrations (IC₅₀) reported in Table VIII.

**Table VIII. ADP-Glo^{™} PASK kinase assay IC₅₀ of illustrative compounds of the invention**

| **Cpd#** | **PASK IC₅₀** |
|---|---|
| 2 | **** |
| 4 | **** |
| 8 | **** |
| 9 | **** |
| 10 | **** |
| 12 | **** |
| 13 | ** |
| 14 | **** |
| 15 | **** |
| 17 | **** |
| 18 | **** |
| 20 | ** |
| 28 | **** |
| 29 | **** |
| 30 | **** |
| 32 | **** |
| 34 | *** |
| 35 | **** |
| 36 | ** |
| 37 | **** |
| 38 | **** |
| 39 | **** |
| 40 | **** |
| 42 | **** |
| 43 | **** |
| 44 | **** |
| 47 | *** |
| 48 | **** |
| 49 | **** |
| 50 | **** |
| 51 | **** |
| 52 | **** |
| 53 | **** |
| 54 | **** |
| 55 | **** |
| 56 | **** |
| 57 | **** |
| 58 | **** |
| 59 | **** |
| 60 | **** |
| 61 | **** |
| 62 | **** |
| 63 | *** |
| 64 | **** |
| 65 | **** |
| 66 | **** |
| 67 | **** |
| 68 | **** |
| 69 | **** |
| 70 | **** |
| 71 | **** |
| 72 | **** |
| 73 | **** |
| 74 | **** |
| 75 | **** |
| 76 | **** |
| 77 | **** |
| 78 | **** |
| 79 | **** |
| 80 | *** |
| 81 | **** |
| 82 | **** |
| 83 | **** |
| 84 | **** |
| 85 | **** |
| 86 | **** |
| 87 | ** |
| 88 | ** |
| 89 | **** |
| 90 | **** |

| | |
|---|---|
| * > 500 nM ** > 100 - 500 nM *** > 10 - 100 nM **** 0.01 - 10 nM | |

### 3.2. Cellular assays

### 3.2.1. PASK autophosphorylation ELISA assay

### 3.2.1.1. Overview

The compounds of the invention are profiled in a cellular assay to determine their capacity to reduce the autophosphorylation levels of PASK at position Thr307 in Hek293 cells overexpressing PASK, using an ELISA-based readout.

### 3.2.1.2. Protocol

### 3.2.1.2.1 Cell assay procedure

At day 1, a 96-well cell assay plate is coated with poly-D-lysine (50 µL/well of a 0.05 mg/mL solution in PBS) and incubated for h at 37 °C. The plate is subsequently washed once with PBS and stored dry at RT until further use. Hek293 cells are transfected with a pcDNA3.1-PASK(FL,WT)-FLAG construct (SEQ ID1) and seeded in culture medium (DMEM + 10% FBS) in a poly-D-lysine coated 96-well plate (0.3 µL JetPEI DNA transfection reagent (Polyplus-transfection SA, Cat# 101-40), 10 ng construct and 90 ng pBluescript, 60000 cells per well). As positive control (representing lack of Thr307 phosphorylation), Hek293 cells are transfected with pcDNA3.1-PASK(FL,DN)-FLAG construct (kinase inactive K1028R mutant of PASK; SEQ ID2), following the same transfection conditions. The cell plate is incubated overnight at 37 °C, 5% CO₂.

At day 2, the medium of the assay plate is removed and 100 µL of fresh medium (DMEM + 10% FBS) is added to the plate. Cell plate is further incubated overnight at 37 °C, 5% CO₂. This to allow further expression of PASK protein.

At day 3, the medium is removed and replaced with 100 µL of serum free medium (DMEM). Test compound is added to the plate (8 points concentration curve with 1/3 dilution steps starting from 30 µM final concentration in 0.3% DMSO final). In the positive and negative control wells of the plate, 0.3% DMSO final is added. For each tested condition, duplicates are made for ELISA readout. Cell plate is incubated 24 h at 37 °C, 5% CO₂.

At day 4, the medium is removed and the plate is washed once with 100 µL/well PBS. Cells are lysed by adding 50 µL/well western blot lysis buffer (20 mM Tris pH 7.5, 150 mM NaCl and 1% Triton) to the plate. The assay plate is stored at -80 °C and thawed to perform the ELISA readout.

### 3.2.1.2.2 PASK autophosphorylation ELISA readout

At day 1, the ELISA plate is coated with the mouse anti-FLAG antibody (2 µg/mL diluted in PBS, 100 µL/well) and incubated overnight at 4 °C.

At day 2, the plate is washed once with 150 µL/well PBS. 200 µL/well blocking buffer (PBS with 3% BSA) is added and the plate is incubated for 4 h at RT. The plate is washed with 150 µL/well high salt washing buffer (20 mM Na₂HPO₄, 0.5% Triton X-100, 0.1% SDS, 0.1% BSA and 1 M NaCl) followed by one wash with 150 µL/well low salt washing buffer (20 mM NazHPOa, 0.5% Triton X-100, 0.1% SDS, 0.1% BSA and 150 mM NaCl). Meanwhile, the cell lysate plate is thawed, two wells of the same condition are pooled (2 × 50 µL) and added to the ELISA plate. The ELISA plate is incubated overnight at 4 °C.

At day 3, the ELISA plate is washed twice with 150 µL/well high salt washing buffer followed by two washes with low salt washing buffer. 100 µL/well detection antibody for pPASK (phospho-Akt substrate (RXXS*/T*) (110B7E) rabbit antibody (Cell Signaling Technology, Inc., Cat# 9614) 2 µg/mL diluted in PBS with 3% BSA and 1x Halt^{™} protease and phosphatase inhibitor cocktail (Thermo Fisher Scientific, Inc., Cat# 78447)) is added and incubated for 2 h at RT. The plate is washed twice with high and low salt wash buffer (150 µL/well) and 100 µL/well of an HRP antibody (swine anti-rabbit HRP antibody (Agilent Technologies, Inc., Cat# P039901) diluted 1/2000 in PBS with 3% BSA and 1x Halt^{™} protease and phosphatase inhibitor cocktail) is added to the plate, followed by an incubation for 1 h at RT in the dark (plate sealed with aluminum seal). The plate is washed twice with high and low salt wash buffer (150 µL/well) and 100 µL/well of SuperSignal^{™} ELISA Pico Chemiluminescent Substrate (Thermo Fisher Scientific, Inc., Cat# 37070)(premix part 1 and part 2, 1/1) is added. The plate is incubated for 4 min at RT before measuring luminescence on the Luminoskan^{™} Ascent (Thermo Fisher Scientific, Inc.) (PMT default voltage; 100 ms integration time).

### 3.2.1.3. Data analysis and results

Raw data are generated following the read-out performed by the Luminoskan^{™} Ascent, used to calculate percentage inhibition (PIN) values which are then imported into Graphpad Prism^{®} software (GraphPad Software, Inc.) to generate dose response curves and derive the average half maximal inhibitory concentrations (IC₅₀) reported in Table IX.

**Table IX. PASK autophosphorylation IC₅₀ of illustrative compounds of the invention**

| **Cpd#** | **PASK IC₅₀** |
|---|---|
| 2 | *** |
| 8 | * |
| 9 | *** |
| 18 | *** |
| 23 | **** |
| 28 | *** |
| 32 | ** |
| 35 | ** |
| 38 | **** |
| 39 | **** |
| 40 | ** |
| 42 | **** |
| 43 | ** |
| 50 | * |
| 62 | * |
| 64 | **** |
| 65 | **** |
| 66 | * |
| 67 | ** |
| 68 | * |
| 69 | * |
| 70 | **** |
| 71 | * |
| 72 | ** |
| 74 | * |
| 76 | ** |
| 77 | * |
| 78 | ** |
| 79 | * |
| 82 | * |
| 84 | ** |
| 85 | ** |
| 86 | **** |
| 89 | ** |
| 90 | *** |

| | |
|---|---|
| * > 5000 nM ** > 1000 - 5000 nM *** > 500 - 1000 nM **** 0.1 - 500 nM | |

### Example 4. In vivo assays

### 4.1. Western diet murine diabetes model

The aim of this assay is to determine the efficacy of a test compound in a diet-induced mouse model where the insulin resistance disease is a consequence of a high fat, high fructose diet.

### 4.1.1. Materials

High fat diet obtained from Research Diets, Inc. (Cat# D12492i)

Chow Diet obtained from Research Diets, Inc. (Cat# D12450Ji)

### 4.1.2. Animals

Five week-old C57BL/6NRj male mice (Janvier Labs, France) are maintained at 22 °C on a 12h light/dark cycle (7 AM - 7 PM); food and water are provided *ad libitum.*

### 4.1.3. Study design

After a 7-day acclimatization period, the routine diet of mice is replaced by a chow diet (10 kcal% fat) for the control group or by a high fat diet (60 kcal% fat) for western diet (WD) group mice. Furthermore, for WD groups, drinking water is supplemented with 15% fructose and 1% dextrose and water is changed twice a week. Mice are maintained under chow or western diet for 6 weeks with a weekly body weight measurement.

After these 42 days of induction, mice are randomly assigned to a group according to their body weight and glycaemia. Mice are dosed from day 42 to day 84 with either vehicle (PEG200/methylcellulose 0.5% (25/75) + 1 mol eq. NaOH), metformin (150 mg/kg, *b.i.d., p.o.* in methylcellulose 0.5%), or test compound (5 mg/kg, *b.i.d., p.o.* in PEG200/methylcellulose 0.5% (25/75) + 1 mol eq. NaOH).

At day 72, fat and lean mass are measured using a Bruker minispec LF50 Body Composition Analyzer on non-anesthetized mice.

At day 74, an insulin tolerance test is performed. After 6 h fasting, glycaemia is measured at T0 then mice undergo intra-peritoneal insulin injection (2 U/kg), then glycaemia is measured atT15, T30, T60, and T90 min with a handheld glucose meter, by pricking the tail vein in order to obtain a drop of blood.

At day 79, an oral glucose tolerance test is performed. After 18 h fasting, mice are dosed in order to be at Tmax at T0. Glycaemia is measured at T0; the mice then undergo oral glucose administration (1 g/kg) and glycaemia is measured at T30, T60, T90 and T120 min with a handheld glucose meter, by pricking tail vein in order to obtain a drop of blood.

At day 84, mice are sacrificed. Blood is collected on EDTA, centrifuged, and plasma is frozen.

### 4.1.4. Assessment of disease

Measured parameters are:
- body weight (once per week)
- fat and lean mass repartition (D72)
- insulin tolerance test (D74)
- oral glucose tolerance test (D79)
- homeostasis model assessment of insulin resistance (HOMA-IR, D84)
- delta fasted glycaemia (D42 to D79)
- blood triglyceride levels (D84)

### 4.1.5. Histology

At sacrifice, part of the liver is collected and fixed in 4% formaldehyde for 48 h before embedding in paraffin. 4 µm thick sections are stained with hematoxylin and eosin and are scanned (NanoZoomer, Hamamatsu) before quantification by image analysis (CaloPix^{®} software, TRIBVN Healthcare SAS). Liver steatosis is measured as the percentage of lipid droplet area per liver tissue area.

### 4.2. Diet-induced obesity (DIO) mouse model

The aim of this assay is to determine the effect of a test compound on the glucose profile in a high fat, high fructose diet mouse model.

### 4.2.1. Materials

High fat diet obtained from Research Diets, Inc. (Cat# D12492)

Rat and Mouse No. 1 maintenance (RM1) diet from Dietex International, Ltd. (Cat# 801002)

### 4.2.2. Animals

Five week-old C57BL/6 male mice (Charles River, France) are maintained at 22 °C on a 12h light/dark cycle (8 AM - 8 PM); food and water are provided *ad libitum.*

For the thirteen weeks prior to study initiation, animals are fed a 60 kcal% high fat diet and 15% fructose in drinking water. Control animals are fed an RM1 diet and tap water.

### 4.2.3. Study design

At study day 0, the mice are fasted for 6 h (fructose replaced by tap water) and blood is collected to measure glucose, insulin and calculate the HOMA-IR. Responding DIO mice are then randomized in homogenous groups according to their body weight and HOMA-IR.

Dosing starts at day 1 for 6 consecutive weeks. Mice are dosed with either vehicle (PEG200/methylcellulose 0.5% (25/75)), pioglitazone (30 mg/kg, *q.d., p.o.* in PEG200/methylcellulose 0.5% (25/75)), sitagliptin (50 mg/kg, *q.d., p.o.* in PEG200/methylcellulose 0.5% (25/75)), or test compound (30 mg/kg, *b.i.d., p.o.* in PEG200/methylcellulose 0.5% (25/75) + 1 mol eq. NaOH).

At day 15, 29, and 41, mice are fasted for 6 h, and blood (+ EDTA) is collected from the tail tip to measure glycaemia, insulin, free fatty acids, triglycerides, and total cholesterol.

At day 29, mice are subjected to an oral glucose tolerance test. Mice are fasted for 6 h (+ tap water) and blood glucose is measured before (T0) and after a bolus injection of glucose solution (2 g/kg) at 15, 30, 60, 90 and 120 min. Plasma insulin level is also assessed before and 15 min post-glucose bolus to evaluate glucose-induced insulin secretion.

At day 41, mice are submitted to an insulin tolerance test. In this context, mice are fasted for 6 h (+ tap water) and blood glucose is measured before and after subcutaneous injection of insulin (1 U/kg) at 15, 30, 60, 90 and 120 min.

At day 42, a steady state pharmacokinetics sampling is done for the treatment groups. Mice from each group are sampled at time T0, 15 min, 3 h and 6 h post-dosing. In this context, blood plasma is collected in Li-heparin tubes via the retro-orbital sinus, then centrifuged at 3000 rpm, 4 °C for 20 min.

At day 43, mice are sacrificed ~2 h after the last dosing by maximal blood withdrawal performed via the retro-orbital sinus and cervical dislocation under 4% isoflurane. Fed plasma triglycerides are measured, and liver, pancreas and epididymal white adipose tissue (eWAT) are collected.

### 4.2.4. Assessment of disease

Measured parameters are:
- body weight (once per week)
- blood chemistry (blood glucose, plasma insulin, HOMA-IR, plasma free fatty acids, plasma triglycerides, plasma cholesterol) (week 0, 2, 4, 6)
- insulin tolerance test (D41)
- oral glucose tolerance test (D29)
- hepatic free fatty acids, hepatic cholesterol, hepatic triglycerides (D43)
- liver and right eWAT weight (D43)

### 4.2.5. Histology

At sacrifice, part of the liver is collected and fixed in 4% formaldehyde for 48 h before embedding in paraffin. 4 µm thick sections are stained with hematoxylin and eosin and are scanned (NanoZoomer, Hamamatsu) before quantification by image analysis (CaloPix^{®} software, TRIBVN Healthcare SAS). Liver steatosis is measured as the percentage of lipid droplet area per liver tissue area.

### 4.3. Diabetic monkey model

The aim of this assay is to determine the effect of a test compound on lipid metabolism and glucose handling in diabetic non human primates (NHPs) under a high-calorie diet (HCD) consisting of high-fat and high-fructose.

### 4.3.1. Materials

Metformin hydrochloride (CAS# 1115-70-4) was obtained from TCI Europe NV (Cat# M2009).

The composition of the high-calorie diet (HCD) is shown in **Error! Reference source not found..**

**Table X. Nutrient and energy composition of the high fat high fructose diet (HCD)**

| Nutrient composition (weight %) | | | | | | Energy composition (cal %) | | |
|---|---|---|---|---|---|---|---|---|
| protein | fat | fiber | calcium | phosphate | cholesterol | protein | fat | carbohydrate |
| ≥16.3% | ≥17.7% | ≥1.9% | 1.1% | 0.6% | ≥0.5% | 16.2% | 39.5% | 44.3% (10% of total energy from fructose) |

### 4.3.2. Animals

Male obese diabetic cynomolgus monkeys were selected based on their body weights, glucose, insulin levels, and blood lipid parameters. The inclusion criteria were the following: age ≤ 22 years; fasted plasma glucose between 120 and 300 mg/dL; triglycerides > 125 mg/dL, and insulin > 75 mIU/mL.

The animals are maintained at 20-23 °C and 40-70% humidity on a 12h light/dark cycle (7 AM - 7 PM). Water is provided *ad libitum* and the animals are fed twice daily with the high-calorie diet (HCD) enriched with seasonal fruits or vegetables.

### 4.3.3. Study design

The animals were fed with HCD for 16 weeks, divided into 3 periods:
- Baseline: induction period, 2 weeks of HCD, followed by 2 weeks for acclimatization, training, vehicle dosing (*b.i.d*), and collection of baseline data, while still on HCD.
- Treatment: animals were treated for 8 weeks with vehicle or drug treatment (metformin as positive control, test compound alone, or a combination of metformin and test compound) while still fed with HCD.
- Washout: 4 weeks of washout without treatment, while still fed with HCD.

After the baseline period, animals were randomized into 4 groups according to body weight, as well as their blood glucose and lipid parameters. The animals were then dosed with either vehicle (methylcellulose 0.5%, *b.i.d., p.o.*), metformin (25 mg/kg, *b.i.d., p.o.* in methylcellulose 0.5%), test compound alone (30 mg/kg, *q.d., p.o.* in methylcellulose 0.5% + 1 mol eq. NaOH in the morning, with vehicle dosing in the afternoon), or a combination of metformin and test compound (morning: 25 mg/kg metformin + 30 mg/kg test compound, both in methylcellulose 0.5%; afternoon: 25 mg/kg metformin in methylcellulose 0.5%).

All doses of metformin and test compound were reduced by one third after 4 weeks of treatment because of some mild clinical signs (soft stools and some vomiting) in some of the drug-treated animals. After the dose reduction, these mild clinical signs improved for the other 4 weeks of treatment.

The treatment groups of the study are summarized in Table XI.

**Table XI. Study groups overview**

| **Group #** | **Group** | **Purpose** | **Dose (weeks 1-4)** | **Dose (weeks 5-8)** | **Vehicle** | **Dose schedule** | **Route** | **N** |
|---|---|---|---|---|---|---|---|---|
| **1** | vehicle | negative control | - | - | methylcellulose 0.5% | b.i.d. | p.o. | 6 |
| **2** | metformin | positive control | 25 mg/kg | 16.7 mg/kg | methylcellulose 0.5% | b.i.d. | p.o. | 6 |
| **3** | test compound | test | 30 mg/kg | 20 mg/kg | methylcellulose 0.5% + 1 mol eq. NaOH | q.d. | p.o. | 6 |
| **4** | metformin + test compound | test | 25 mg/kg + 30 mg/kg | 16.7 mg/kg + 20 mg/kg | methylcellulose 0.5% | b.i.d. + q.d. | p.o. | 6 |

### 4.3.4. Assessment of disease

The following parameters were measured once per week during the 8 weeks of treatment and also during the 4 weeks of washout:
- body weight
- food consumption
- blood chemistry (fasted blood glucose, plasma triglycerides, plasma total cholesterol, low density lipoproteins (LDL), and high density lipoproteins (HDL))

Oral glucose tolerance was measured in fasted animals before treatment initiation (baseline), at the end of the treatment period (week 8), and after 4 weeks of washout (week 12).

Hepatic echogenicity attenuation is a marker of liver steatosis, measured by ultrasound technology. At baseline and at the end of the 8-week treatment period, hepatic echogenicity measurements were generated by calculating the echogenicity attenuation of near and far regions of liver tissue by ultrasound. The kidney cortex region was used as a reference region in which there is no change expected during the treatment period. Data are expressed as H/R: Hepatic (right lobe)/Renal cortex (right kidney) echogenicity ratios.

### 4.3.5. Results

When tested in this protocol, the following data were obtained for **Cpd 18** (data analysis performed only with animals for which a complete dataset could be collected over the treatment period):

**Table XII. Bodyweight change (% change from baseline)**

| **Week** | **1** | **2** | **3** | **4** | **5** | **6** | **7** | **8** | **9** | **10** | **11** | **12** |
|---|---|---|---|---|---|---|---|---|---|---|---|---|
| **Period** | **treatment** | | | | | | | | **washout** | | | |
| Group 1 | 2.1 | 2.5 | 3.6 | 2.5 | 3.1 | 2.2 | 1.6 | 2.9 | 2.4 | 1.9 | 2.5 | 1.4 |
| s.e.m. | 0.8 | 0.6 | 1.0 | 1.3 | 1.2 | 0.9 | 1.0 | 0.9 | 1.0 | 1.1 | 1.1 | 1.2 |
| N | 6 | 6 | 6 | 6 | 6 | 6 | 6 | 6 | 6 | 6 | 6 | 6 |
| Group 2 | 1.0 | -0.7 | -1.3 | -5.2 | -5.1 | -6.1 | -6.6 | -6.2 | -6.5 | -7.3 | -6.3 | -6.1 |
| s.e.m. | 0.4 | 1.3 | 1.8 | 1.5 | 2.3 | 3.3 | 3.8 | 4.4 | 4.8 | 4.6 | 4.4 | 4.3 |
| N | 6 | 6 | 6 | 6 | 6 | 6 | 6 | 6 | 6 | 6 | 6 | 6 |
| p-value | ns | ns | ns | ns | ns | ns | ns | ns | ns | ns | ns | ns |
| Group 3 | 0.4 | -1.9 | -3.8 | -5.6 | -7.5 | -9.8 | -11.9 | -12.6 | -13.6 | -14.5 | -14.3 | -14.8 |
| s.e.m. | 0.7 | 0.7 | 1.7 | 1.8 | 2.7 | 3.7 | 4.3 | 5.0 | 5.1 | 5.2 | 4.9 | 5.3 |
| N | 5 | 5 | 5 | 5 | 5 | 5 | 5 | 5 | 5 | 5 | 5 | 5 |
| p-value | ns | ns | ns | ns | ns | ns | ns | * | * | ** | ** | ** |
| Group 4 | -0.6 | -3.8 | -5.7 | -8.1 | -11.7 | -14.2 | -16.4 | -15.9 | -16.9 | -16.7 | -15.0 | -14.7 |
| s.e.m. | 1.6 | 2.3 | 3.1 | 3.1 | 3.8 | 4.3 | 4.8 | 4.9 | 4.8 | 4.9 | 4.8 | 4.6 |
| N | 5 | 5 | 5 | 5 | 5 | 5 | 5 | 5 | 5 | 5 | 5 | 5 |
| p-value | ns | ns | ns | ns | * | * | ** | ** | ** | ** | ** | ** |

| | | | | | | | | | | | | |
|---|---|---|---|---|---|---|---|---|---|---|---|---|
| ns: not significant \| p-values: *** (<0.001) - ** (<0.01) - * (<0.05) vs vehicle group using a repeated measurements (longitudinal mixed) model with heterogeneous Toeplitz correlations on the time points, followed by Tukey's multiple comparisons procedure | | | | | | | | | | | | |

**Table XIII. HbAlc change (% change from baseline)**

| **Week** | **1** | **2** | **3** | **4** | **5** | **6** | **7** | **8** | **9** | **10** | **11** | **12** |
|---|---|---|---|---|---|---|---|---|---|---|---|---|
| **Period** | **treatment** | | | | | | | | **washout** | | | |
| Group 1 | -2.4 | -5.4 | -6.3 | -6.5 | -6.4 | -5.5 | -2.5 | -4.8 | -8.4 | -10.1 | -6.2 | -5.4 |
| s.e.m. | 1.0 | 1.2 | 1.5 | 1.3 | 3.4 | 2.7 | 2.8 | 3.3 | 2.3 | 3.9 | 5.0 | 4.0 |
| N | 6 | 6 | 6 | 6 | 6 | 6 | 6 | 6 | 6 | 6 | 6 | 6 |
| Group 2 | -2.8 | -12.3 | -17.2 | -18.9 | -28.6 | -30.9 | -28.0 | -20.5 | -24.1 | -23.5 | -30.1 | -28.6 |
| s.e.m. | 1.4 | 2.9 | 2.7 | 3.4 | 2.3 | 2.0 | 3.5 | 5.8 | 5.8 | 7.5 | 3.8 | 5.3 |
| N | 6 | 6 | 6 | 6 | 6 | 6 | 6 | 6 | 6 | 6 | 6 | 6 |
| p-value | ns | ns | ** | ns | *** | *** | *** | ** | ns | ns | ** | * |
| Group 3 | -6.3 | -9.4 | -17.2 | -24.1 | -19.9 | -24.3 | -21.4 | -20.3 | -30.7 | -25.6 | -21.3 | -27.9 |
| s.e.m. | 1.3 | 1.0 | 4.1 | 7.4 | 4.3 | 2.5 | 4.1 | 3.5 | 9.8 | 1.6 | 1.2 | 7.0 |
| N | 5 | 5 | 5 | 5 | 5 | 5 | 5 | 5 | 5 | 5 | 5 | 5 |
| p-value | ns | ns | * | * | * | *** | * | ns | ns | ns | ns | * |
| Group 4 | -3.2 | -9.6 | -14.1 | -16.9 | -20.6 | -26.1 | -22.9 | -28.5 | -29.7 | -31.7 | -29.9 | -33.4 |
| s.e.m. | 2.2 | 1.2 | 1.6 | 3.3 | 4.5 | 2.4 | 6.5 | 5.3 | 8.1 | 6.9 | 6.8 | 7.6 |
| N | 5 | 5 | 5 | 5 | 5 | 5 | 5 | 5 | 5 | 5 | 5 | 5 |
| p-value | ns | ns | ns | ns | * | *** | ** | ** | ns | ns | ** | ** |

| | | | | | | | | | | | | |
|---|---|---|---|---|---|---|---|---|---|---|---|---|
| ns: not significant \| p-values: *** (<0.001) - ** (<0.01) - * (<0.05) vs vehicle group using a repeated measurements (longitudinal mixed) model with heterogeneous first-order autoregressive correlations on the time points, followed by Tukey's multiple comparisons procedure | | | | | | | | | | | | |

**Table XIV. Oral glucose tolerance test (glucose AUC mmol/L*min)**

| **Week** | **0** | **8** | **12** |
|---|---|---|---|
| **Period** | **baseline** | **treatment** | **washout** |
| Group 1 | 2568 | 3192 | 3249 |
| s.e.m. | 354 | 255 | 295 |
| N | 6 | 6 | 6 |
| Group 2 | 2167 | 1562 | 2376 |
| s.e.m. | 418 | 300 | 491 |
| N | 6 | 6 | 6 |
| p-value | ns | * | ns |
| Group 3 | 2371 | 2307 | 2291 |
| s.e.m. | 464 | 486 | 540 |
| N | 5 | 5 | 5 |
| p-value | ns | ns | ns |
| Group 4 | 2182 | 1136 | 1710 |
| s.e.m. | 385 | 178 | 507 |
| N | 5 | 5 | 4 |
| p-value | ns | ** | ns |

| | | | |
|---|---|---|---|
| ns: not significant \| p-values: *** (<0.001) - ** (<0.01) - * (<0.05) vs vehicle group using a repeated measurements (longitudinal mixed) model with compound symmetry correlations on the time points, followed by Tukey's multiple comparisons procedure | | | |

**Table XV. Triglyceride change (% change from baseline)**

| **Week** | **1** | **2** | **3** | **4** | **5** | **6** | **7** | **8** | **9** | **10** | **11** | **12** |
|---|---|---|---|---|---|---|---|---|---|---|---|---|
| **Period** | **treatment** | | | | | | | | **washout** | | | |
| Group 1 | 31.6 | 15.6 | 88.9 | 82.5 | 93.7 | 90.3 | 139.8 | 191.8 | 181.0 | 235.7 | 336.1 | 235.5 |
| s.e.m. | 20.4 | 23.9 | 41.6 | 34.2 | 50.9 | 32.1 | 49.7 | 80.3 | 78.6 | 106.6 | 120.5 | 82.8 |
| N | 6 | 6 | 6 | 6 | 6 | 6 | 6 | 6 | 6 | 6 | 6 | 6 |
| Group 2 | -33.3 | -31.2 | -36.7 | -44.2 | -62.6 | -59.6 | -55.9 | -39.3 | -33.4 | 9.8 | 49.7 | 32.2 |
| s.e.m. | 12.8 | 17.3 | 15.8 | 14.4 | 6.7 | 7.3 | 8.0 | 15.8 | 27.7 | 72.8 | 85.8 | 70.1 |
| N | 6 | 6 | 6 | 6 | 6 | 6 | 6 | 6 | 6 | 6 | 6 | 6 |
| p-value | ns | ns | * | ** | ** | *** | *** | ** | ** | * | * | ns |
| Group 3 | -20.2 | -56.1 | -41.6 | -44.5 | -44.4 | -46.2 | -35.0 | -37.4 | -49.3 | -45.9 | -19.9 | 18.8 |
| s.e.m. | 16.6 | 9.9 | 13.8 | 20.5 | 23.3 | 22.2 | 14.6 | 12.4 | 17.2 | 6.3 | 15.0 | 68.5 |
| N | 5 | 5 | 5 | 5 | 5 | 5 | 5 | 5 | 5 | 5 | 5 | 5 |
| p-value | ns | ns | ** | ** | * | *** | *** | ** | ** | * | ** | ns |
| Group 4 | -31.7 | -30.0 | -28.2 | -48.3 | -52.3 | -50.8 | -63.2 | -56.1 | -60.2 | -58.1 | -41.4 | -65.4 |
| s.e.m. | 7.1 | 15.2 | 23.1 | 7.2 | 9.4 | 7.8 | 8.4 | 6.9 | 11.2 | 15.4 | 12.2 | 6.5 |
| N | 5 | 5 | 5 | 5 | 5 | 5 | 5 | 5 | 5 | 5 | 5 | 5 |
| p-value | ns | ns | * | ** | * | *** | *** | ** | ** | ** | ** | ** |

| | | | | | | | | | | | | |
|---|---|---|---|---|---|---|---|---|---|---|---|---|
| ns: not significant \| p-values: *** (<0.001) - ** (<0.01) - * (<0.05) vs vehicle group using a repeated measurements (longitudinal mixed) model with heterogeneous first-order autoregressive correlations on the time points, followed by Tukey's multiple comparisons procedure | | | | | | | | | | | | |

**Table XVI. Total cholesterol change (% change from baseline)**

| **Week** | **1** | **2** | **3** | **4** | **5** | **6** | **7** | **8** | **9** | **10** | **11** | **12** |
|---|---|---|---|---|---|---|---|---|---|---|---|---|
| **Period** | **treatment** | | | | | | | | **washout** | | | |
| Group 1 | 69.5 | 88.2 | 116.7 | 144.8 | 138.1 | 153.3 | 179.3 | 196.5 | 185.9 | 190.6 | 207.7 | 230.3 |
| s.e.m. | 18.1 | 27.6 | 40.3 | 45.0 | 49.7 | 47.1 | 58.8 | 69.3 | 69.1 | 67.4 | 75.3 | 77.5 |
| N | 6 | 6 | 6 | 6 | 6 | 6 | 6 | 6 | 6 | 6 | 6 | 6 |
| Group 2 | 48.2 | 33.8 | 18.6 | 28.0 | 3.2 | -6.7 | -12.0 | -5.1 | 5.8 | 42.4 | 83.8 | 115.8 |
| s.e.m. | 11.0 | 22.4 | 24.2 | 35.2 | 21.7 | 13.0 | 12.9 | 16.7 | 21.8 | 34.3 | 43.7 | 46.5 |
| N | 6 | 6 | 6 | 6 | 6 | 6 | 6 | 6 | 6 | 6 | 6 | 6 |
| p-value | ns | ns | ns | ns | * | * | ** | ** | ** | * | ns | ns |
| Group 3 | 46.1 | 41.5 | 22.3 | -22.3 | -21.0 | -26.5 | -30.5 | -20.4 | -31.5 | -8.2 | 15.5 | 48.7 |
| s.e.m. | 23.3 | 28.9 | 24.0 | 19.6 | 12.5 | 14.9 | 15.0 | 17.0 | 12.8 | 14.8 | 19.0 | 39.8 |
| N | 5 | 5 | 5 | 5 | 5 | 5 | 5 | 5 | 5 | 5 | 5 | 5 |
| p-value | ns | ns | ns | * | * | * | ** | *** | ** | ** | ** | * |
| Group 4 | 50.9 | 30.3 | 20.9 | -22.6 | -7.8 | -2.6 | -32.9 | -26.1 | -15.5 | 10.9 | 28.5 | 58.3 |
| s.e.m. | 19.4 | 9.0 | 12.9 | 7.9 | 8.4 | 8.0 | 15.0 | 14.5 | 19.2 | 20.3 | 18.3 | 21.6 |
| N | 5 | 5 | 5 | 5 | 5 | 5 | 5 | 5 | 5 | 5 | 5 | 5 |
| p-value | ns | ns | ns | * | * | ** | ** | *** | *** | ** | ** | ** |

| | | | | | | | | | | | | |
|---|---|---|---|---|---|---|---|---|---|---|---|---|
| ns: not significant \| p-values: *** (<0.001) - ** (<0.01) - * (<0.05) vs vehicle group using a repeated measurements (longitudinal mixed) model with Toeplitz correlations on the time points, followed by Tukey's multiple comparisons procedure | | | | | | | | | | | | |

**Table XVII. HDL change (% change from baseline)**

| **Week** | **1** | **2** | **3** | **4** | **5** | **6** | **7** | **8** | **9** | **10** | **11** | **12** |
|---|---|---|---|---|---|---|---|---|---|---|---|---|
| **Period** | **treatment** | | | | | | | | **washout** | | | |
| Group 1 | 1.6 | 0.7 | -11.2 | -15.2 | -17.0 | -13.9 | -10.6 | -8.8 | -6.4 | -1.6 | -11.1 | -4.4 |
| s.e.m. | 5.0 | 4.2 | 5.8 | 5.5 | 6.2 | 3.3 | 6.8 | 8.0 | 4.2 | 5.5 | 5.2 | 5.4 |
| N | 6 | 6 | 6 | 6 | 6 | 6 | 6 | 6 | 6 | 6 | 6 | 6 |
| Group 2 | 14.1 | 8.4 | 6.2 | -4.3 | 4.2 | 11.7 | 13.0 | 12.4 | 13.5 | 22.7 | 21.2 | 24.9 |
| s.e.m. | 4.8 | 4.0 | 6.1 | 12.7 | 11.4 | 8.1 | 7.1 | 5.9 | 8.0 | 11.8 | 13.1 | 9.8 |
| N | 6 | 6 | 6 | 6 | 6 | 6 | 6 | 6 | 6 | 6 | 6 | 6 |
| p-value | ns | ns | ns | ns | ns | * | ns | ns | ns | ns | ns | ns |
| Group 3 | 18.6 | 3.7 | 7.7 | -12.0 | 1.0 | 9.5 | 8.3 | -0.9 | 1.5 | 8.0 | 21.9 | 23.9 |
| s.e.m. | 6.0 | 8.7 | 11.0 | 13.1 | 11.3 | 11.8 | 14.8 | 8.0 | 6.0 | 7.3 | 6.0 | 12.8 |
| N | 5 | 5 | 5 | 5 | 5 | 5 | 5 | 5 | 5 | 5 | 5 | 5 |
| p-value | ns | ns | ns | ns | ns | ns | ns | ns | ns | ns | ns | ns |
| Group 4 | 2.1 | -13.1 | -14.9 | -16.0 | -6.9 | 0.3 | 0.9 | 8.7 | 18.9 | 6.0 | 11.0 | 12.0 |
| s.e.m. | 5.7 | 4.5 | 4.5 | 5.6 | 3.0 | 4.6 | 7.4 | 7.9 | 10.5 | 13.2 | 12.4 | 12.4 |
| N | 5 | 5 | 5 | 5 | 5 | 5 | 5 | 5 | 5 | 5 | 5 | 5 |
| p-value | ns | ns | ns | ns | ns | ns | ns | ns | ns | ns | ns | ns |

| | | | | | | | | | | | | |
|---|---|---|---|---|---|---|---|---|---|---|---|---|
| ns: not significant \| p-values: *** (<0.001) - ** (<0.01) - * (<0.05) vs vehicle group using a repeated measurements (longitudinal mixed) model with heterogeneous first-order autoregressive correlations on the time points, followed by Tukey's multiple comparisons procedure | | | | | | | | | | | | |

**Table XVIII. LDL change (% change from baseline)**

| **Week** | **1** | **2** | **3** | **4** | **5** | **6** | **7** | **8** | **9** | **10** | **11** | **12** |
|---|---|---|---|---|---|---|---|---|---|---|---|---|
| **Period** | **treatment** | | | | | | | | **washout** | | | |
| Group 1 | 84.9 | 106.7 | 134.5 | 161.7 | 164.4 | 182.0 | 207.8 | 221.4 | 210.4 | 213.5 | 233.0 | 272.1 |
| s.e.m. | 20.7 | 35.5 | 47.7 | 52.4 | 63.8 | 60.7 | 73.0 | 84.9 | 81.8 | 79.9 | 89.2 | 102.1 |
| N | 6 | 6 | 6 | 6 | 6 | 6 | 6 | 6 | 6 | 6 | 6 | 6 |
| Group 2 | 52.8 | 38.3 | 16.6 | 31.0 | 5.1 | -10.5 | -17.2 | -10.5 | -1.6 | 38.6 | 81.5 | 118.6 |
| s.e.m. | 13.2 | 27.0 | 29.4 | 43.0 | 27.8 | 17.0 | 15.7 | 19.7 | 24.3 | 36.4 | 40.6 | 44.6 |
| N | 6 | 6 | 6 | 6 | 6 | 6 | 6 | 6 | 6 | 6 | 6 | 6 |
| p-value | ns | ns | ns | ns | ns | ** | ** | ** | ** | * | ns | ns |
| Group 3 | 60.5 | 58.3 | 35.1 | -28.7 | -27.5 | -33.2 | -39.4 | -27.9 | -41.8 | -13.5 | 10.2 | 46.2 |
| s.e.m. | 31.3 | 37.6 | 33.5 | 24.0 | 18.2 | 19.9 | 19.2 | 21.4 | 15.8 | 18.2 | 21.3 | 38.0 |
| N | 5 | 5 | 5 | 5 | 5 | 5 | 5 | 5 | 5 | 5 | 5 | 5 |
| p-value | ns | ns | ns | ns | * | ** | ** | ** | ** | ** | * | * |
| Group 4 | 67.1 | 46.7 | 33.6 | -21.9 | 0.0 | 2.1 | -38.5 | -32.5 | -20.0 | 29.3 | 45.1 | 87.5 |
| s.e.m. | 17.8 | 10.0 | 15.5 | 9.4 | 13.8 | 10.0 | 17.2 | 16.9 | 24.1 | 38.9 | 31.8 | 42.2 |
| N | 5 | 5 | 5 | 5 | 5 | 5 | 5 | 5 | 5 | 5 | 5 | 5 |
| p-value | ns | ns | ns | ns | ns | * | ** | ** | ** | * | * | ns |

| | | | | | | | | | | | | |
|---|---|---|---|---|---|---|---|---|---|---|---|---|
| ns: not significant \| p-values: *** (<0.001) - ** (<0.01) - * (<0.05) vs vehicle group using a repeated measurements (longitudinal mixed) model with heterogeneous first-order autoregressive correlations on the time points, followed by Tukey's multiple comparisons procedure | | | | | | | | | | | | |

**Table XIX. Hepatic echogenicity (H/R ratio)**

| **Week** | **-3** | **9** |
|---|---|---|
| **Period** | **baseline** | **washout** |
| Group 1 | 1.10 | 1.29 |
| s.e.m. | 0.12 | 0.11 |
| N | 6 | 6 |
| Group 2 | 1.21 | 1.37 |
| s.e.m. | 0.13 | 0.13 |
| N | 6 | 6 |
| Group 3 | 1.13 | 1.03 |
| s.e.m. | 0.17 | 0.09 |
| N | 5 | 5 |
| Group 4 | 1.24 | 1.14 |
| s.e.m. | 0.24 | 0.10 |
| N | 5 | 5 |

### FINAL REMARKS

It will be appreciated by those skilled in the art that the foregoing descriptions are exemplary and explanatory in nature, and intended to illustrate the invention and its preferred embodiments.

It should be understood that factors such as the differential cell penetration capacity of the various compounds can contribute to discrepancies between the activity of the compounds in the *in vitro* biochemical and cellular assays.

At least some of the chemical names of compound of the invention as given and set forth in this application, may have been generated on an automated basis by use of a commercially available chemical naming software program, and have not been independently verified. Representative programs performing this function include the Lexichem^{®} naming tool sold by OpenEye Scientific Software, Inc. and the Autonom Software tool sold by MDL, Inc. In the instance where the indicated chemical name and the depicted structure differ, the depicted structure will control.

### REFERENCES

Bundgaard H. 1985. Design of prodrugs, Elsevier.
Hao H-X et al. 2007. PAS kinase is required for normal cellular energy balance. Proc. Natl. Acad. Sci. U. S. A. 104, 15466-15471.
Hao H-X, Rutter J. 2008. The role of PAS kinase in regulating energy metabolism. IUBMB Life 60, 204-209
Katschinski DM et al. 2003. Targeted disruption of the mouse PAS domain serine/threonine kinase PASKIN. Mol. Cell. Biol. 23, 6780-6789.
Moller DE, Kaufman KD. 2005. Metabolic syndrome: a clinical and molecular perspective. Annu. Rev. Med. 56, 45-62.
Pérez-García A et al. 2018. High-fat diet alters PAS kinase regulation by fasting and feeding in liver. J. Nutr. Biochem. 57, 14-25.
Wu X et al. 2014. PAS kinase drives lipogenesis through SREBP-1 maturation. Cell Rep. 8, 242-255.
Wuts PGM, Greene TW. 2006. Greene's Protective Groups in Organic Synthesis 4th ed., Wiley-Interscience.
Zhang D et al. 2015. Per-Arnt-Sim Kinase (PASK): An Emerging Regulator of Mammalian Glucose and Lipid Metabolism. Nutrients 7, 7437-7450.

## Claims

1. A compound according to Formula I: 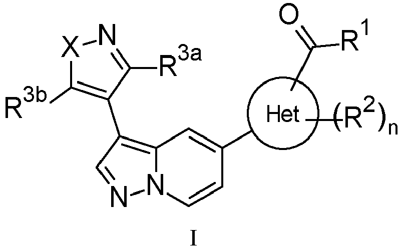 wherein,
X is O or NR⁴;
n is 0, 1, or 2;
Het is 5 membered monocyclic heteroaryl comprising one, two or three heteroatoms independently selected from N, O, and S;
R¹ is -OR⁵ or -NR^{6a}R^{6b};
each R² is independently selected from
- -O-R⁷,
- C₁₋₆ alkyl optionally substituted with one or more independently selected halo,
- C₃₋₆ cycloalkyl,
- -C(=O)-NR^{8a}R^{8b},
- 4-6 membered monocyclic heterocycloalkyl comprising one, two or three heteroatoms independently selected from N, O, and S, and
- 4-6 membered monocyclic heterocycloalkenyl comprising one double bond and further comprising one, or two heteroatoms independently selected from N, O, and S;
R^{3a} and R^{3b} are independently H or C₁₋₃ alkyl optionally substituted with one or more independently selected halo;
R⁴ is C₁₋₃ alkyl optionally substituted with one or more F;
R⁵ is H or C₁₋₄ alkyl optionally substituted with one or more independently selected -C(=O)-NR^{9a}R^{9b} or -O-C(=O)-C₁₋₆ alkyl;
R^{6a} and R^{6b} are independently H, -S(=O)₂-C₁₋₄ alkyl, or -S(=O)₂-C₃₋₆ cycloalkyl;
each R⁷ is independently selected from
- C₁₋₆ alkyl optionally substituted with one or more independently selected halo or C₁₋₄ alkoxy, and
- 4-6 membered monocyclic heterocycloalkyl comprising one, two or three heteroatoms independently selected from N, O, and S;
R^{8a} and R^{8b} are independently H, C₁₋₄ alkyl, or phenyl; and
R^{9a} and R^{9b} are independently H or C₁₋₄ alkyl;
or a pharmaceutically acceptable salt, solvate, or salt of a solvate thereof.

2. A compound or pharmaceutically acceptable salt thereof, according to claim 1, wherein Het is furanyl, pyrazolyl, oxazolyl, or thiazolyl.

3. A compound or pharmaceutically acceptable salt thereof, according to claim 1, wherein the compound is according to Formula IIa, IIb, IIc, or IId:

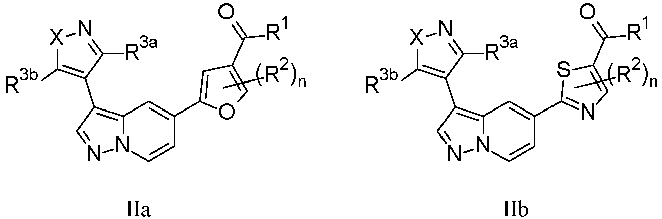

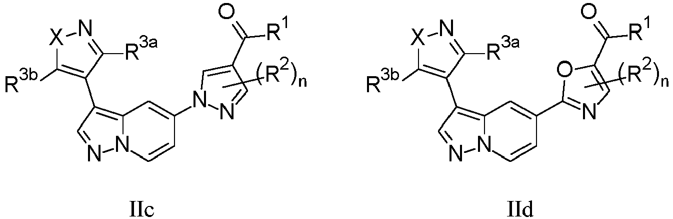

4. A compound or pharmaceutically acceptable salt thereof, according to any one of claims 1-3, wherein R^{3a} and R^{3b} are independently H, -CH₃, -CH₂CH₃,-CH(CH₃)₂, or -CF₃.

5. A compound or pharmaceutically acceptable salt thereof, according to any one of claims 1-3, wherein R^{3a} and R^{3b} are both -CH₃.

6. A compound or pharmaceutically acceptable salt thereof, according to claim 1, wherein the compound is according to Formula IIIa, IIIb, IIIc, or IIId:

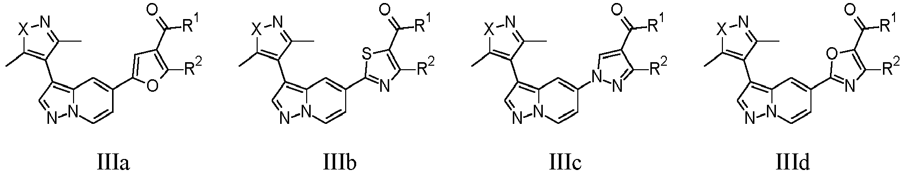

7. A compound or pharmaceutically acceptable salt thereof, according to any one of claims 1-6, wherein R² is -O-R⁷, or C₃₋₆ cycloalkyl.

8. A compound or pharmaceutically acceptable salt thereof, according to any one of claims 1-6, wherein R² is -O-CH₂CH₃, or cyclopropyl.

9. A compound or pharmaceutically acceptable salt thereof, according to claim 1, wherein the compound is according to Formula IVa, IVb, IVc, or IVd:

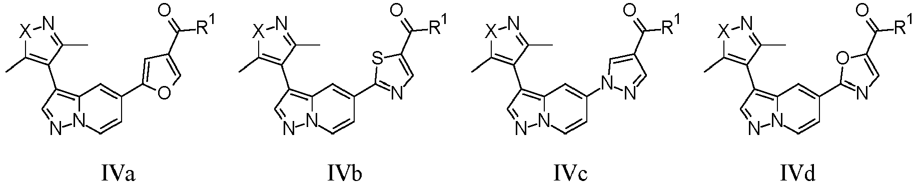

10. A compound or pharmaceutically acceptable salt thereof, according to any one of claims 1-9, wherein X is O.

11. A compound or pharmaceutically acceptable salt thereof, according to claim 1, wherein the compound is according to Formula Va, Vb, or Vc:

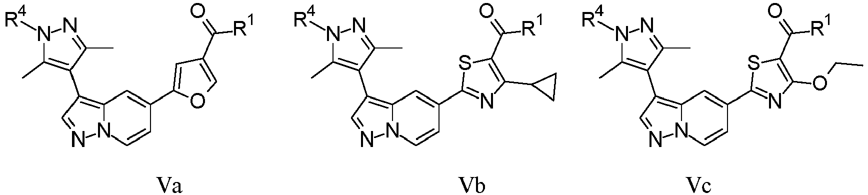

12. A compound or pharmaceutically acceptable salt thereof, according to any one of claims 1-9 and 11, wherein R⁴ is -CH₃, -CH₂CH₃, -CH(CH₃)₂, -CHF₂ or -CH₂-CF₃.

13. A pharmaceutical composition comprising a pharmaceutically acceptable carrier and a pharmaceutically effective amount of a compound or pharmaceutically acceptable salt thereof according to any one of claims 1-12.

14. A compound or pharmaceutically acceptable salt thereof, according to any one of claims 1-12, or a pharmaceutical composition according to claim 13 for use in medicine.

15. A compound or pharmaceutically acceptable salt thereof, according to any one of claims 1-12, or a pharmaceutical composition according to claim 13 for use in the prophylaxis and/or treatment of endocrine, nutritional, metabolic, and/or cardiovascular diseases.

## Patentansprüche

1. Verbindung gemäß Formel I: 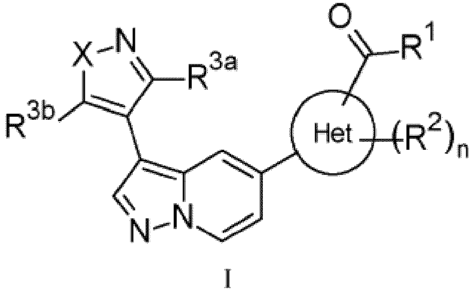 wobei
X O oder NR⁴ ist;
n 0, 1 oder 2 ist;
Het ein 5-gliedriges monocyclisches Heteroaryl mit ein, zwei oder drei Heteroatomen, unabhängig ausgewählt aus N, O und S, ist;
R¹-OR⁵ oder -NR^{6a}R^{6b} ist;
jedes R² unabhängig aus
- -O-R⁷,
- C₁₋₆ ^{-Alkyl}, gegebenenfalls substituiert durch ein oder mehrere unabhängig ausgewählte Halogene,
- ein C₃₋₆-Cycloalkyl,
- C(=O)-NR^{8a}R^{8b},
- ein monocyclisches Heterocycloalkyl mit 4 bis 6 Gliedern und umfassend ein, zwei oder drei Heteroatome, unabhängig ausgewählt aus N, O und S, und
- ein 4-6-gliedriges monocyclisches Heterocycloalkenyl mit einer Doppelbindung und ferner mit ein oder zwei Heteroatomen, unabhängig ausgewählt aus N, O und S, ausgewählt ist;
R^{3a} und R^{3b} unabhängig voneinander H oder C₁₋₃-Alkyl, gegebenenfalls substituiert durch ein oder mehrere unabhängig gewählten Halogene, sind;
R⁴ C₁₋₃-Alkyl, gegebenenfalls substituiert durch ein oder mehreren F, ist;
R⁵ H oder ein C₁₋₄-Alkyl, gegebenenfalls substituiert durch ein oder mehrere unabhängig ausgewählten -C(=O)-NR^{9a}R^{9b} oder -OC(=O)-C₁₋₆-Alkyl ist;
R^{6a} und R^{6b} unabhängig H, -S(=O)₂-C₁₋₄-Alkyl oder -S(=O)₂-C₃₋₆-Cycloalkyl sind;
jedes R⁷ unabhängig aus
- einem C₁₋₆-Alkyl, gegebenenfalls substituiert durch ein oder mehrere Halogene oder C₁₋₄-Alkoxy, und
- einem monocyclischen 4-6-gliederigen Heterocycloalkyl mit ein, zwei oder drei Heteroatomen, unabhängig ausgewählt aus N, O und S, ausgewählt ist;
R^{8a} und R^{8b} unabhängig voneinander H, C₁₋₄-Alkyl oder Phenyl sind; und
R^{9a} und R^{9b} unabhängig voneinander H oder C₁₋₄-Alkyl sind ;
oder ein pharmazeutisch annehmbares Salz, Solvat oder Solvatsalz davon.

2. Verbindung oder pharmazeutisch annehmbares Salz davon nach Anspruch 1, wobei Het Furanyl, Pyrazolyl, Oxazolyl oder Thiazolyl ist.

3. Verbindung oder pharmazeutisch annehmbares Salz davon nach Anspruch 1, wobei die Verbindung der Formel Ila, IIb, IIc oder IId entspricht:

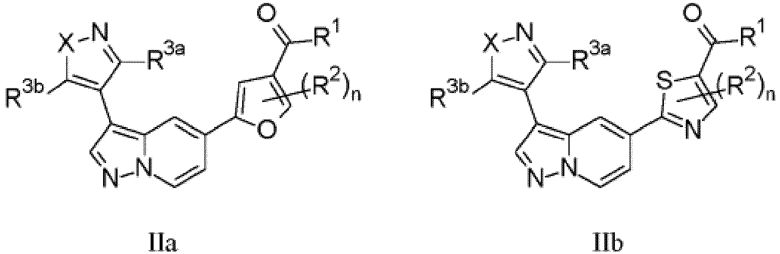

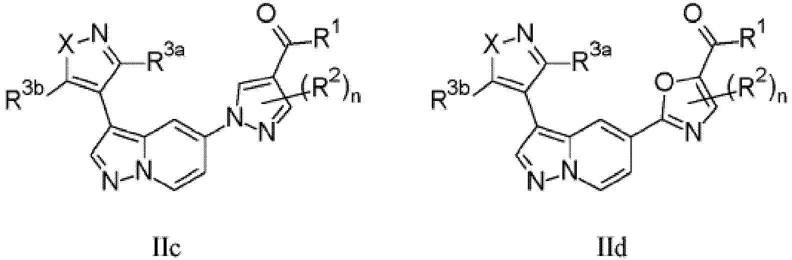

4. Verbindung oder pharmazeutisch annehmbares Salz davon nach einem der Ansprüche 1 bis 3, wobei R^{3a} und R^{3b} unabhängig voneinander H, -CH₃, -CH₂CH₃, -CH(CH₃)₂ oder -CF₃ sind .

5. Verbindung oder pharmazeutisch annehmbares Salz davon nach einem der Ansprüche 1 bis 3, wobei R^{3a} und R^{3b} beide -CH³ sind.

6. Verbindung oder pharmazeutisch annehmbares Salz davon nach Anspruch 1, wobei die Verbindung der Formel IIIa, IIIb, IIIe oder IIId entspricht:

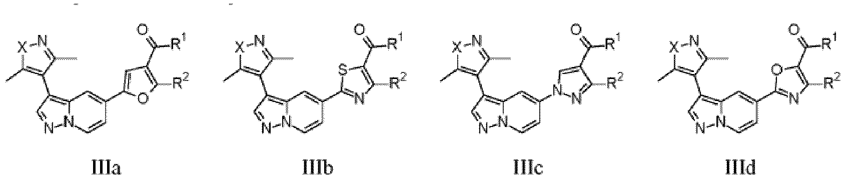

7. Verbindung oder pharmazeutisch annehmbares Salz davon nach einem der Ansprüche 1 bis 6, wobei R²-OR⁷ oder ein C₃₋₆-Cycloalkyl ist.

8. Verbindung oder pharmazeutisch annehmbares Salz davon nach einem der Ansprüche 1 bis 6, wobei R² -O-CH₂ CH₃ oder Cyclopropyl ist.

9. Verbindung oder pharmazeutisch annehmbares Salz davon nach Anspruch 1, wobei die Verbindung der Formel Iva, IVb, IVc oder IVd entspricht:

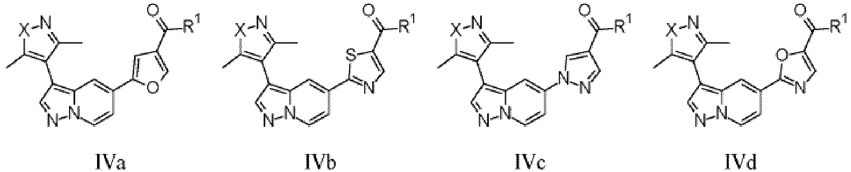

10. Verbindung oder pharmazeutisch annehmbares Salz davon nach einem der Ansprüche 1 bis 9, wobei X O ist.

11. Verbindung oder pharmazeutisch annehmbares Salz davon nach Anspruch 1, wobei die Verbindung der Formel Va, Vb oder Vc entspricht:

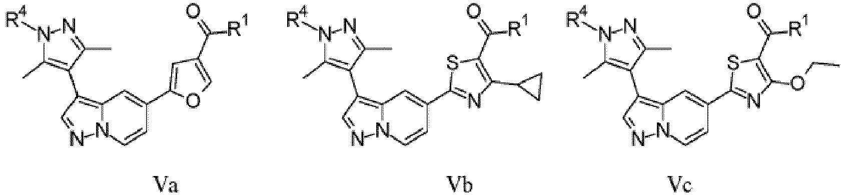

12. Verbindung oder pharmazeutisch annehmbares Salz davon nach einem der Ansprüche 1 bis 9 und 11, wobei R⁴ -CH₃, -CH₂CH₃, -CH(CH₃)₂, -CHF₂ oder -CH₂-CF3 ist.

13. Pharmazeutische Zusammensetzung, umfassend einen pharmazeutisch annehmbaren Träger und eine pharmazeutisch wirksame Menge einer Verbindung oder eines pharmazeutisch annehmbaren Salzes davon nach einem der Ansprüche 1 bis 12.

14. Verbindung oder pharmazeutisch annehmbares Salz davon nach einem der Ansprüche 1 bis 12 oder pharmazeutische Zusammensetzung nach Anspruch 13 zur medizinischen Verwendung.

15. Verbindung oder pharmazeutisch annehmbares Salz davon nach einem der Ansprüche 1 bis 12 oder pharmazeutische Zusammensetzung nach Anspruch 13, für die Verwendung bei der Prophylaxe und/oder Behandlung von endokrinen, ernährungsbedingten, metabolischen und/oder kardiovaskulären Erkrankungen.

## Revendications

1. Composé selon la Formule I : 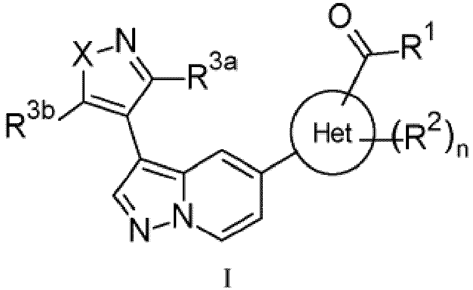 dans laquelle
X est O ou NR⁴ ;
N est 0, 1, ou 2 ;
Het est un hétéroaryle monocyclique à 5 chaînons comprenant un, deux ou trois hétéroatomes indépendamment choisi parmi N, O et S ;
R¹ est -OR⁵ ou -NR^{6a}R^{6b} ;
chaque R² est choisi indépendamment parmi
- -O-R⁷,
- alkyle en C₁₋₆ éventuellement substitué par un ou plusieurs halogéno choisis indépendamment,
- un cycloalkyle en C₃₋₆,
- C(=O)-NR^{8a}R^{8b},
- un hétérocycloalkyle monocyclique ayant 4 à 6 chaînons et comprenant un, deux ou trois hétéroatomes choisis indépendamment parmi N, O et S, et
- un hétérocycloalcényle monocyclique ayant 4 à 6 chaînons, comprenant une double liaison et comprenant en outre un ou deux hétéroatomes choisis indépendamment parmi N, O et S ;
R^{3a} et R^{3b} sont indépendamment H ou un alkyle en C₁₋₃ éventuellement substitué par un ou plusieurs halogéno choiis indépendamment ;
R⁴ est un alkyle en C₁₋₃ éventuellement substitué par un ou plusieurs F ;
R⁵ est H ou un alkyle en C₁₋₄ éventuellement substitué par un ou plusieurs -C(=O)-NR^{9a}R^{9b} choisis indépendamment ou -O-C(=O)-alkyle en C₁₋₆ :
R^{6a} et R^{6b} sont indépendamment H, -S(=O)₂-alkyle en Cₗ₋₄, ou -S(=O)₂-cycloalkyle en C₃₋₆ ;
chaque R⁷ est choisi indépendamment parmi
- alkyle en C₁₋₆ éventuellement substitué par un ou plusieurs halogéno ou alcoxy en C₁₋₄ et
- un hétérocycloalkyle monocyclique ayant 4 à 6 chaînons et comprenant un, deux ou trois hétéroatomes choisis indépendamment parmi N, O, et S ;
R^{8a} et R^{8b} sont indépendamment H, un alkyle en C₁₋₄ ou un phényle : et
R^{9a} et R^{9b} sont indépendamment H ou un alkyle en C₁₋₄ ;
ou un sel, un solvate ou un sel d'un solvate pharmaceutiquement acceptable de celui-ci.

2. Composé ou sel pharmaceutiquement acceptable de celui-ci, selon la revendication 1, Het étant un furanyle, un pyrazolyle, un oxazolyle, ou un thiazolyle.

3. Composé ou sel pharmaceutiquement acceptable de celui-ci selon la revendication 1, le composé étant conforme à la formule IIa, IIb, IIc, ou IId :

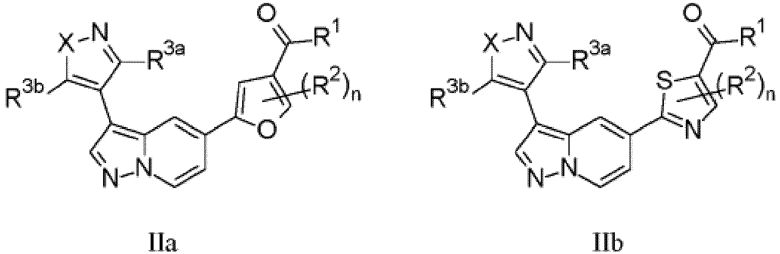

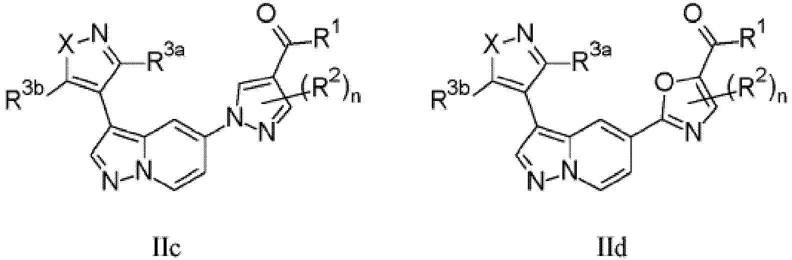

4. Composé ou sel pharmaceutiquement acceptable de celui-ci, selon l'une quelconque des revendications 1 à 3, R^{3a} et R^{3b} étant indépendamment H, -CH₃, -CH₂CH₃, -CH(CH₃)₂ ou -CF₃.

5. Composé ou sel pharmaceutiquement acceptable de celui-ci, selon l'une quelconque des revendications 1 à 3, R^{3a} et R^{3b} étant tous deux -CH³.

6. Composé ou sel pharmaceutiquement acceptable de celui-ci selon la revendication 1, le composé étant conforme à la Formule IIIa, IIIb, IIIc, ou IIId :

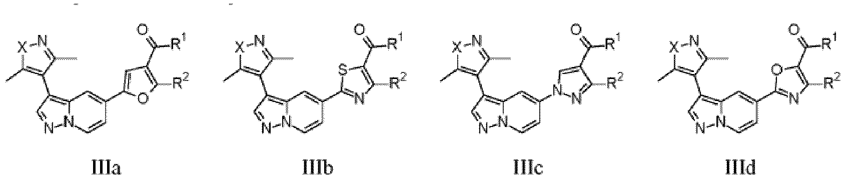

7. Composé ou sel pharmaceutiquement acceptable de celui-ci, selon l'une quelconque des revendications 1 à 6, R² étant -O-R⁷, ou un cycloalkyle en C₃₋₆.

8. Composé ou sel pharmaceutiquement acceptable de celui-ci, selon l'une quelconque des revendications 1 à 6, R² étant -O-CH₂CH₃, ou un cyclopropyle.

9. Composé ou sel pharmaceutiquement acceptable de celui-ci selon la revendication 1, le composé étant conforme à la Formule Iva, IVb, IVc, ou IVd :

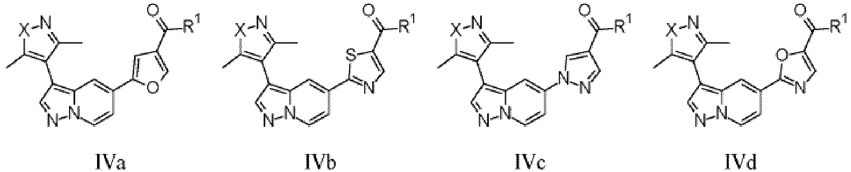

10. Composé ou sel pharmaceutiquement acceptable de celui-ci, selon l'une quelconque des revendications 1 à 9, X étant O.

11. Composé ou sel pharmaceutiquement acceptable de celui-ci, selon la revendication 1, le composé étant conforme à la Formule Va, Vb, ou Vc :

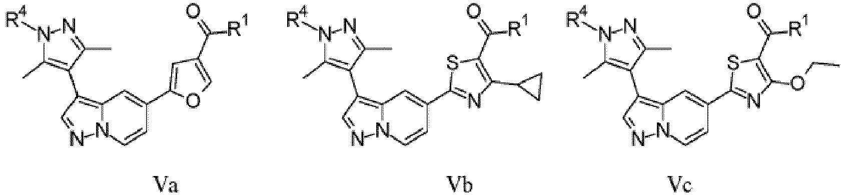

12. Composé ou sel pharmaceutiquement acceptable de celui-ci, selon l'une quelconque des revendications 1 à 9 et 11, R⁴ étant -CH₃, -CH₂CH₃, -CH(CH₃)₂, -CHF₂ ou -CH₂-CF₃.

13. Composition pharmaceutique comprenant un véhicule pharmaceutiquement acceptable et une quantité pharmaceutiquement efficace d'un composé ou d'un sel pharmaceutiquement acceptable de celui-ci selon l'une quelconque des revendications 1 à 12.

14. Composé ou sel pharmaceutiquement acceptable de celui-ci, selon l'une quelconque des revendications 1 à 12, ou composition pharmaceutique selon la revendication 13 destinés à une utilisation médicale.

15. Composé ou sel pharmaceutiquement acceptable de celui-ci selon l'une quelconque des revendications 1 à 12, ou composition pharmaceutique selon la revendication 13 destinés à une utilisation dans la prophylaxie et/ou le traitement de maladies endocriniennes, nutritionnelles, métaboliques et/ou cardiovasculaires.
